# EUROPEAN PATENT APPLICATION

(11) **EP 4 009 051 A1**
(43) Date of publication of application: **08.06.2022**
(21) Application number: 20834295.6
(22) Date of filing: 24.04.2020
(51) Int. Cl.: G01N 33/68

(54) **USE OF DKK1 INHIBITOR IN PREVENTION AND/OR TREATMENT OF TUMOR CACHEXIA AND DISEASES ASSOCIATED WITH DIABETES**

(30) Priority: 04.07.2019 CN 201910600862
(71) Applicant: Shanghi Dongtsu Biosciences Co., Ltd., Shanghai 200438 (CN)
(72) Inventor: ZHU, Weidong, Shanghai 200438 (CN)
(74) Representative: Mewburn Ellis LLP
(86) International application number: PCT/CN2020/086807
(87) International publication number: WO 2021/000640

(57) **Abstract**

Disclosed is the use of a DKK1 gene or the coded protein inhibitor thereof for the preparation of a composition or formulation used to prevent and/or treat tumor cachexia and diseases associated with diabetes. By inhibiting the expression or activity of the DKK1 gene or the protein encoded thereby in tumor cells, which can effectively prevent and/or treat tumor cachexia and diseases associated with diabetes, also provided is a method for detecting the protein expression level of DKK1 in blood, which level is taken as an indicator for precise treatment and judging prognosis.

## Description

### Technical field

The present invention relates to the field of biomedicine. More specifically, the present invention relates to the use of DKK1 inhibitors in the prevention and/or treatment of tumor cachexia and diabetes-associated diseases.

### Background

Tumor cachexia is accompanied by weight loss, which severely impairs the quality of life, limits the treatment of cancer, and shortens the lifetime. However, there is currently no effective treatment method. Similarly, diabetes is accompanied by damage to various organs in the body, which seriously endangers the life and health of patients, and at the same time restricts the treatment of many accompanying diseases or inherent diseases. Although various hypoglycemic therapies can effectively prevent the occurrence and development of diseases, insulin itself has strong toxic side effects on a large group of patients (such as patients with diabetes and heart failure), and how to judge whether the organs of some patients whose hypoglycemic effect is unstable are still suffering from damage, etc., there are still a large number of clinical problems that need to be resolved.

Therefore, there is an urgent need in the art to develop a therapeutic regimen that can effectively prevent and/or treat these clinically difficult diseases.

### Summary of the invention

The purpose of the present invention is to provide a therapeutic regimen that can effectively prevent and/or treat tumor cachexia and diabetes-associated diseases.

In the first aspect of the present invention, it provides a use of an inhibitor of DKK1 gene or an encoded protein thereof for preparing a composition or preparation for the prevention and/or treatment of tumor cachexia and diseases accompanied with diabetes.

In another preferred embodiment, the inhibitor includes an inhibitor that inhibits the binding of DKK1 to LRP5/LRP6.

In another preferred embodiment, the inhibitor inhibits the activity and/or expression level of DKK1.

In another preferred embodiment, the inhibitor inhibits the formation of a complex of DKK1 and LRP5/LRP6.

In another preferred embodiment, the inhibitor inhibits the activity and/or expression level of LRP5/LRP6.

In another preferred embodiment, the inhibitor inhibits the activity and/or expression level of Kremen1/Kremen2.

In another preferred embodiment, the inhibitor is selected from the group consisting of: antibody, free DKK1 receptor binding domain or a mutant thereof, other free ligands of DKK1 receptor or mutants thereof, a small molecule compound, microRNA, siRNA, shRNA, CRISPR reagent, and a combination thereof.

In another preferred embodiment, the inhibitor is selected from the group consisting of MDC, CDHC/STHC, IGFBP4 protein, a vector expressing IGFBP4, IGFBP4 mutant, a vector expressing IGFBP4 mutant, LRP5/LRP6 neutralizing antibody, a binding domain of free LRP5/LRP6 to DKK1 or a mutant thereof, Kremen1/Kremen2 inhibitor (including neutralizing antibody), a binding domain of free Kremen1/Kremen2 to DKK1 or a mutant thereof, and a combination thereof.

In another preferred embodiment, the vector expressing IGFBP4 or the vector expressing IGFBP4 mutant includes a viral vector.

In another preferred embodiment, the viral vector is selected from the group consisting of: adeno-associated virus vector, lentiviral vector, and a combination thereof.

In another preferred embodiment, the IGFBP4 protein includes a full-length protein or protein fragment.

In another preferred embodiment, the IGFBP4 protein further includes a derivative of IGFBP4 protein.

In another preferred embodiment, the derivative of the IGFBP4 protein includes a modified IGFBP4 protein, a protein molecule whose amino acid sequence is homologous to the natural IGFBP4 protein and has the activity of the natural IGFBP4 protein, a dimer or polymer of the IGFBP4 protein, a fusion protein containing the amino acid sequence of the IGFBP4 protein.

In another preferred embodiment, the "protein molecule whose amino acid sequence is homologous to the natural IGFBP4 protein and has natural IGFBP4 protein activity" means that a protein molecule with native IGFBP4 protein activity whose amino acid sequence has ≥85% homology, preferably ≥90% homology, more preferably ≥95% homology, most preferably ≥98% homology with IGFBP4 protein.

In another preferred embodiment, the IGFBP4 mutant is selected from the group consisting of IGFBP4/H95P, IGFBPDF/H95A, IGFBPDF/H95E, IGFBPDF/H95D, and a combination thereof.

In another preferred embodiment, the Kremen1/2 inhibitor refers to a substance capable of antagonizing the activity and/or content of the *Kremen2* gene or a protein thereof in vivo or in vitro; the substance can be a synthetic or natural compound, protein (such as antibody), nucleotide, etc.

In another preferred embodiment, the Kremen1/2 inhibitor includes a substance that antagonizes the expression of Kremen1/2.

In another preferred embodiment, the Kremen1/2 inhibitor includes a Kremen1/2 protein antagonist and/or a Kremen1/2 gene antagonist.

In another preferred embodiment, the inhibition of Kremen1/2 expression or activity refers to reducing the expression or activity of Kremen1/2 gene or protein by ≥20%, preferably ≥50%, more preferably ≥70%.

In another preferred embodiment, the tumor cachexia includes tumor cachexia with normal or low expression of Wnt.

In another preferred embodiment, the tumor cachexia includes tumor cachexia not related to the Wnt pathway.

In another preferred embodiment, the composition or preparation is further used for one or more purposes selected from the group consisting of:
(a) reducing the content of β-arrestin2 in a tumor cell;
(b) inhibiting the activation of IκB and NFκB in a mammal;
(c) Inhibiting the up-regulation of p53 and Bax/Bcl-2;
(d) inhibiting down-regulation of a muscle protein marker;
(e) inhibiting cytokine upregulation in a mammalian muscle tissue.

In another preferred embodiment, the tumor cell is from one or more tumors selected from the group consisting of intestinal cancer, lung cancer, gastric cancer, pancreatic cancer, head and neck cancer, and a combination thereof.

In another preferred embodiment, the muscle protein marker includes myoglobin.

In another preferred embodiment, the cytokine in the muscle tissue is selected from the group consisting of TNFα, IL1β, IL6, and a combination thereof.

In another preferred embodiment, the mammal includes a mammal suffering from tumor cachexia and diseases accompanied with diabetes.

In another preferred embodiment, the mammal includes a human or a non-human mammal.

In another preferred embodiment, the non-human mammal includes a rodent (e.g., a mouse, rat, or rabbit), primate (e.g., monkey).

In another preferred embodiment, the DKK1 is derived from a mammal; preferably, from a human, mouse, rat, or rabbit; more preferably, from a human.

In another preferred embodiment, the DKK1 gene includes a wild-type DKK1 gene and a mutant DKK1 gene.

In another preferred embodiment, the mutant includes a mutant form in which the function of the encoded protein is not changed after mutation (i.e" the function is the same or substantially the same as that of the wild-type encoded protein).

In another preferred embodiment, the polypeptide encoded by the mutant DKK1 gene is the same or substantially the same as the polypeptide encoded by the wild-type DKK1 gene.

In another preferred embodiment, the mutant DKK1 gene comprises a polynucleotide having a homology of ≥80% (preferably ≥90%, more preferably ≥95%, more preferably ≥98% or 99%) compared to the wild DKK1 gene.

In another preferred embodiment, the mutant DKK1 gene comprises a polynucleotide of 1-60 (preferably 1-30, more preferably 1-10) nucleotides is truncated or added at the 5' and/or 3' end of the wild-type DKK1 gene.

In another preferred embodiment, the DKK1 gene includes a cDNA sequence, a genomic sequence, and a combination thereof.

In another preferred embodiment, the DKK1 protein includes an active fragment of DKK1 or a derivative thereof.

In another preferred embodiment, the active fragment or derivative thereof has at least 90%, preferably 95%, more preferably 98% and 99% homology with DKK1.

In another preferred embodiment, the active fragment or derivative thereof has at least 80%, 85%, 90%, 95%, 100% of DKK1 activity.

In another preferred embodiment, the amino acid sequence of the DKK1 protein is selected from the group consisting of:
(i) a polypeptide having an amino acid sequence as shown in SEQ ID NO.: 1 or 3;
(ii) a polypeptide formed by substitution, deletion or addition of one or several (e.g., 1-10) amino acid residues to the amino acid sequence as shown in SEQ ID NO.: 1 or 3 derived from (i) with the protein function; or
(iii) a polypeptide whose amino acid sequence has ≥90% (preferably ≥95%, more preferably ≥98% or 99%) homology with the amino acid sequence as shown in SEQ ID NO.: 1 or 3 having the protein function.

In another preferred embodiment, the nucleotide sequence of the DKK1 gene is selected from the group consisting of:
(a) a polynucleotide encoding a polypeptide as shown in SEQ ID NO.: 1 or 3;
(b) a polynucleotide whose sequence is shown in SEQ ID NO.: 2 or 4;
(c) a polynucleotide whose nucleotide sequence has a homology of ≥95% (preferably ≥98%, more preferably ≥99%) with the sequence as shown in SEQ ID NO.: 2 or 4;
(d) a polynucleotide having 1-60 (preferably 1-30, more preferably 1-10) nucleotides truncated or added at the 5' end and/or 3' end of the polynucleotide as shown in SEQ ID NO.: 2 or 4;
(e) a polynucleotide complementary to any of the polynucleotides of (a)-(d).

In another preferred embodiment, the amino acid sequence of the DKK1 protein is shown in SEQ ID NO.: 1 or 3.

In another preferred embodiment, the nucleotide sequence encoding the DKK1 protein is shown in SEQ ID NO.: 2 or 4.

In another preferred embodiment, the content of the inhibitor of the DKK1 gene or an encoded protein thereof is 0.1 mg/kg-100 mg/kg, preferably 1 mg/kg-50 mg/kg, more preferably 2 mg/kg- 20mg/kg.

In another preferred embodiment, the composition includes a pharmaceutical composition.

In another preferred embodiment, the pharmaceutical composition contains (a) an inhibitor of *DKK1* gene or an encoded protein thereof; and (b) a pharmaceutically acceptable carrier.

In another preferred embodiment, the pharmaceutical composition is liquid, solid, or semi-solid.

In another preferred embodiment, the dosage form of the pharmaceutical composition includes tablets, granules, capsules, oral liquids, or injections.

In another preferred embodiment, in the pharmaceutical composition, the component (a) accounts for 1-99wt%, preferably 10-90wt%, more preferably 30-70wt% of the total weight of the pharmaceutical composition.

In another preferred embodiment, the composition further includes other drugs for preventing and/or treating tumor cachexia and diseases accompanied with diabetes.

In another preferred embodiment, the other drugs for preventing and/or treating tumor cachexia and diseases accompanied with diabetes are selected from the group consisting of tumor drugs, diabetes drugs, and combinations thereof.

In another preferred embodiment, the diabetes drugs are selected from the group consisting of insulin-like drugs, metformin-like hypoglycemic drugs, GLP-like drugs, ghrelin, and a combination thereof.

In another preferred embodiment, the tumor drugs are selected from the group consisting of chemotherapeutic drugs, targeting therapeutic agents, immunotherapy drugs, cell therapy drugs, and combinations thereof.

In another preferred embodiment, the chemotherapeutic drugs are selected from the group consisting of gemcitabine, cisplatin, vincristine, paclitaxel, doxorubicin, 5-FU, and a combination thereof.

In another preferred embodiment, the targeting therapeutic agents are selected from the group consisting of lapatinib, erlotinib, apatinib, rituximab, bevacizumab, and trastuzumab, and a combination thereof.

In another preferred embodiment, the immunotherapy drugs are selected from the group consisting of PD-1 mAb, PDL-1 mAb, CD47 mAb, and a combination thereof.

In another preferred embodiment, the cell therapy drugs are selected from the group consisting of NK cell, CART cell, TIL cell, and a combination thereof.

In another preferred embodiment, the composition or preparation can be used alone or in combination in the prevention and/or treatment of tumor cachexia and diseases accompanied with diabetes.

In another preferred embodiment, the use in combination includes: use in combination with other drugs for the prevention and/or treatment of tumor cachexia and diseases accompanied with diabetes.

In a second aspect of the present invention, it provides a pharmaceutical composition, comprising:
(a1) a first active ingredient for preventing and/or treating tumor cachexia and diseases accompanied with diabetes, the first active ingredient comprises: an inhibitor *of DKK1* gene or an encoded protein thereof;
(a2) a second active ingredient for preventing and/or treating tumor cachexia and diseases accompanied with diabetes, the second active ingredient comprises: other drugs for preventing and/or treating tumor cachexia and diseases accompanied with diabetes; and
(b) a pharmaceutically acceptable carrier.

In another preferred embodiment, in the pharmaceutical composition, the component (a1) accounts for 1-99wt%, preferably 10-90wt%, more preferably 30-70wt% of the total weight of the pharmaceutical composition.

In another preferred embodiment, in the pharmaceutical composition, the component (a2) accounts for 1-99wt%, preferably 10-90wt%, more preferably 30-70wt% of the total weight of the pharmaceutical composition.

In another preferred embodiment, the weight ratio of the first active ingredient to the second active ingredient is 1:100 to 100:1, preferably 1:10 to 10:1.

In another preferred embodiment, other drugs for preventing and/or treating tumor cachexia and diseases accompanied with diabetes are selected from the group consisting of tumor drugs, diabetes drugs, and combinations thereof.

In another preferred embodiment, the diabetes drugs are selected from the group consisting of insulin-like drugs, metformin-like hypoglycemic drugs, GLP-like drugs, ghrelin, and a combination thereof.

In another preferred embodiment, the tumor drugs are selected from the group consisting of chemotherapeutic drugs, targeting therapeutic agents, immunotherapy drugs, cell therapy drugs, and combinations thereof.

In another preferred embodiment, the chemotherapeutic drugs are selected from the group consisting of gemcitabine, cisplatin, vincristine, paclitaxel, doxorubicin, 5-FU, and a combination thereof.

In another preferred embodiment, the targeting therapeutic agents are selected from the group consisting of lapatinib, erlotinib, apatinib, rituximab, bevacizumab, and trastuzumab, and a combination thereof.

In another preferred embodiment, the immunotherapy drugs are selected from the group consisting of PD-1 mAb, PDL-1 mAb, CD47 mAb, and a combination thereof.

In another preferred embodiment, the cell therapy drugs are selected from the group consisting of NK cell, CART cell, TIL cell, and a combination thereof.

In another preferred embodiment, the pharmaceutical composition may be a single compound or a mixture of multiple compounds.

In another preferred embodiment, the pharmaceutical composition is used to prepare a medicament or preparation for treating or preventing tumor cachexia and diseases accompanied with diabetes.

In another preferred embodiment, the pharmaceutical dosage form is an oral administration or a non-oral administration dosage form.

In another preferred embodiment, the oral dosage form is a tablet, powder, granule or capsule, or emulsion or syrup.

In another preferred embodiment, the non-oral dosage form is an injection.

In another preferred embodiment, the total content of the active ingredient (a1) and the active ingredient (a2) is 1-99 wt %, more preferably 5-90 wt % of the total weight of the composition.

In a third aspect of the present invention, it provides a kit, comprising:
(i) a first container, and an active ingredient (a1) an inhibitor of *DKK1* gene or an encoded protein thereof, or a drug containing the active ingredient (a), located in the first container; and
(ii) a second container, and an active ingredient (a2) other drugs for preventing and/or treating tumor cachexia and diseases accompanied with diabetes, or a medicament containing the active ingredient (a2), located in the second container.

In another preferred embodiment, the first container and the second container are the same or different containers.

In another preferred embodiment, the drug in the first container is a single preparation containing an inhibitor *of DKK1* gene or an encoded protein thereof.

In another preferred embodiment, the drug in the second container is a single preparation containing other drugs for preventing and/or treating tumor cachexia and diseases accompanied with diabetes.

In another preferred embodiment, the dosage form of the drug is an oral dosage form or an injection dosage form.

In another preferred embodiment, the kit further contains instructions, which describe the instructions for co-administering the active ingredient (a1) and the active ingredient (a2) to prevent and/or treat tumor cachexia and diseases accompanied with diabetes.

In another preferred embodiment, the dosage form of the preparation containing the active ingredient (a1) an inhibitor of *DKK1* gene or an encoded protein thereof or the preparation containing other drugs for the prevention and/or treatment of tumor cachexia and diseases accompanied with diabetes respectively includes capsules, tablets, suppositories, or intravenous injections.

In another preferred embodiment, in the preparation containing the active ingredient (a1) an inhibitor of *DKK1* gene or an encoded protein thereof, the concentration of the inhibitor *of DKK1* gene or an encoded protein thereof is 0.1 mg/kg-100 mg/kg, preferably, 1 mg/kg-50 mg/kg, more preferably, 2 mg/kg-20 mg/kg.

In a fourth aspect of the present invention, it provides a method for reducing the content of β-arrestin2 in a tumor cell in vitro, comprising the steps of:
In the presence of the inhibitor of *DKK1* gene or an encoded protein thereof, culturing a tumor cell, thereby reducing the content of β-arrestin2 in a tumor cell.

In another preferred embodiment, the tumor cell is from one or more tumors selected from the group consisting of intestinal cancer, lung cancer, gastric cancer, pancreatic cancer, head and neck cancer, and a combination thereof.

In another preferred embodiment, the tumor cell is a cell cultured in vitro.

In another preferred embodiment, the method is non-diagnostic and non-therapeutic.

In another preferred embodiment, the action concentration of the inhibitor of *DKK1* gene or an encoded protein thereof is 0.1 mg/kg-100 mg/kg, preferably 1 mg/kg-50 mg/kg, more preferably 2 mg/kg- 20mg/kg.

In a fifth aspect of the present invention, it provides use of the pharmaceutical composition according to the second aspect of the present invention or the kit according to the third aspect of the present invention for the preparation of a medicament for preventing and/or treating tumor cachexia and diseases accompanied with diabetes.

In another preferred embodiment, in the pharmaceutical composition, the action concentration of the inhibitor *of DKK1* gene or an encoded protein thereof is 0.1 mg/kg-100 mg/kg, preferably 1 mg/kg-50 mg/kg, more preferably, 2mg/kg-20mg/kg.

In a sixth aspect of the present invention, it provides a method for screening a potential therapeutic agent for tumor cachexia and diseases accompanied with diabetes, comprising:
(a) in a test group, in a culture system, in the presence of a test compound, culturing a cell expressing *DKK1* gene or a protein thereof for a period of time T1, and detecting the expression level E1 and/or activity A1 *of DKK1* gene or a protein thereof in the culture system of the test group;
   and in the control group without the test compound and other conditions are the same, detecting the expression level E2 and/or activity A2 of the *DKK1* gene or a protein thereof in the culture system of the control group; and
(b) comparing E1 and E2, if E1 is significantly lower than E2, indicating that the test compound is a potential therapeutic agent for tumor cachexia and diseases accompanied with diabetes; or
   comparing A1 and A2, and if A1 is significantly lower than A2, indicating that the test compound is a potential therapeutic agent for tumor cachexia and diseases accompanied with diabetes.

In another preferred embodiment, the "significantly higher than" refers to E1/E2≤ 1/2, preferably, ≤ 1/3, more preferably,≤ 1/4.

In another preferred embodiment, the cell includes a tumor cell.

In another preferred embodiment, the tumor cell is from one or more tumors selected from the group consisting of intestinal cancer, lung cancer, gastric cancer, pancreatic cancer, head and neck cancer, and a combination thereof.

In another preferred embodiment, the method is non-diagnostic and non-therapeutic.

In another preferred embodiment, the method includes step (c): administering the potential therapeutic agent identified in step (a) to a mammal, thereby determining its effects on tumor cachexia and diseases accompanied with diabetes in the mammal.

In another preferred embodiment, the mammal includes a human or a non-human mammal.

In another preferred embodiment, the non-human mammal includes a rodent and primate, preferably a mouse, rat, rabbit, and monkey.

In a seventh aspect of the present invention, it provides a method for screening a potential therapeutic agent for tumor cachexia and diseases accompanied with diabetes, comprising:
(a) in a test group, in a culture system, in the presence of a test compound, culturing a tumor cell for a period of time T1, detecting the formation of the complex of DKK1 and LRP6 in the culture system of the test group;
   and in the control group without the test compound and other conditions being the same, detecting the formation of the complex of DKK1 and LRP6 in the culture system of the control group;
(b) if the formation quantity Q1 of the complex of DKK1 and LRP6 in the test group is significantly lower than the formation quantity Q2 of the complex of DKK1 and LRP6 in the control group, indicating that the test compound is a candidate compound.

In another preferred embodiment, the method includes step (b): administering the candidate compound determined in step (a) to a mammal, thereby determining its effects on tumor cachexia and diseases accompanied with diabetes in a mammal.

In another preferred embodiment, the mammal includes a human or a non-human mammal.

In another preferred embodiment, the non-human mammal includes a rodent and primate, preferably a mouse, rat, rabbit, and monkey.

In another preferred embodiment, the "significantly lower" means that Q1/Q2 ≤ 1/2, preferably, ≤ 1/3, more preferably ≤ 1/4.

In another preferred embodiment, the method is non-diagnostic and non-therapeutic.

In another preferred embodiment, the tumor cell is derived from one or more tumors selected from the group consisting of intestinal cancer, lung cancer, gastric cancer, pancreatic cancer, head and neck cancer, and a combination thereof.

In an eighth aspect of the present invention, it provides a method for preventing and/or treating tumor cachexia and diseases accompanied with diabetes, comprising the steps of:
administering an inhibitor of *DKK1* gene or an encoded protein thereof, the pharmaceutical composition of claim 2, or the kit of claim 3 to a subject in need.

In another preferred embodiment, the administration comprises oral administration.

In another preferred embodiment, the subject includes a human or a non-human mammal.

In another preferred embodiment, the non-human mammal includes a rodent and primate, preferably a mouse, rat, rabbit, and monkey.

In another preferred embodiment, the administration dosage of the inhibitor of *DKK1* gene or an encoded protein thereof is 0.1 mg/kg-100 mg/kg, preferably 1 mg/kg-50 mg/kg, more preferably 2 mg/kg- 20mg/kg.

In another preferred embodiment, the administration frequency of the inhibitor of *DKK1* gene or an encoded protein thereof is 1-7 times a week, preferably, 2-5 times a week, more preferably, 2-3 times a week.

In another preferred embodiment, the administration time of the inhibitor of *DKK1* gene or an encoded protein thereof is 1-20 weeks, preferably, 2-12 weeks, more preferably, 4-8 weeks.

In a ninth aspect of the present invention, it provides a method for determining a therapeutic regimen for tumor cachexia and diseases accompanied with diabetes, comprising:
a) providing a test sample from a subject;
b) detecting the level of DKK1 protein in the test sample; and
c) determining a therapeutic regimen for tumor cachexia and diseases accompanied with diabetes based on the DKK1 protein level in the sample.

In another preferred embodiment, the subject is a human or a non-human mammal.

In another preferred embodiment, the sample is derived from blood, tumor tissue, ascites or urine.

In another preferred embodiment, the sample is derived from peripheral blood.

In another preferred embodiment, the following methods are used for detection: immunological detection method, methods in enzymology or mass spectroscope method.

In another preferred embodiment, the immunoassay method is selected from the group consisting of ELISA, Western blotting, immunofluorescence, chemiluminescence, and a combination thereof.

In another preferred embodiment, the therapeutic regimen further includes other therapies for preventing and/or treating tumor cachexia and diseases accompanied with diabetes.

In another preferred embodiment, the other therapies for preventing and/or treating tumor cachexia and diseases accompanied with diabetes are selected from the group consisting of:
(a) oncology drug therapy; and/or
(b) drug therapy for diabetes treatment.

In another preferred embodiment, the diabetes drug is selected from the group consisting of a insulin-like drug, metformin-like hypoglycemic drug, GLP-like drug, ghrelin, and a combination thereof.

In another preferred embodiment, the tumor drug is selected from the group consisting of chemotherapeutic drug, targeting therapeutic agent, immunotherapy drug, cell therapy drug, and a combination thereof.

In another preferred embodiment, the chemotherapeutic drug is selected from the group consisting of gemcitabine, cisplatin, vincristine, paclitaxel, doxorubicin, 5-FU, and a combination thereof.

In another preferred embodiment, the targeting therapeutic agent is selected from the group consisting of lapatinib, erlotinib, apatinib, rituximab, bevacizumab, and trastuzumab, and a combination thereof.

In another preferred embodiment, the immunotherapy drug is selected from the group consisting of PD-1 mAb, PDL-1 mAb, CD47 mAb, and a combination thereof.

In another preferred embodiment, the cell therapy drug is selected from the group consisting of NK cell, CART cell, TIL cell, and a combination thereof.

It should be understood that, within the scope of the present invention, each technical feature of the present invention described above and in the following (as examples) may be combined with each other to form a new or preferred technical solution, which is not listed here due to space limitations.

### Description of Figure

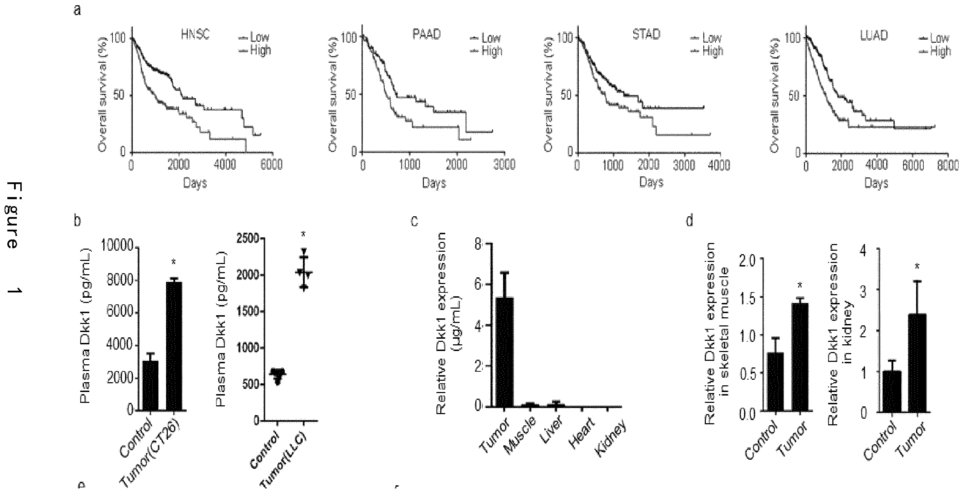

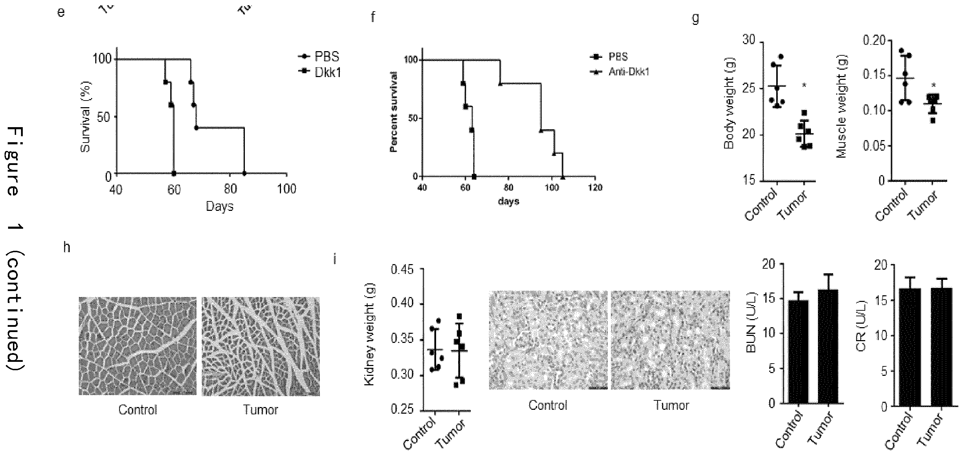

Figure 1 shows that Dkkl upregulation may be associated with cachexia-related tumor death.

Among them, Figure 1a shows the relationship between the expression level of Dkkl in blood and the survival time of patients with different clinical tumor types (including head and neck cancer, pancreatic cancer, gastric adenocarcinoma and lung adenocarcinoma) analyzed by the TCGA database.

Figure 1b shows the detection of Dkkl expression in the blood of tumor-bearing mice (implantation of colorectal carcinoma cells and lung adenocarcinoma cells) by ELISA. Figure 1c shows the expression level of Dkkl in different organs detected by RT-qPCR.

Figure 1d shows RT-qPCR method is used to detect the effect of implanted tumor on the expression level of Dkkl in muscle and kidney.

Figure 1e shows the Kaplan-Meier analysis of the effect of Dkkl on survival in tumor-bearing mice.

Figure If shows the Kaplan-Meier analysis of the effect of Dkkl general antibody on prolonging the survival of tumor-bearing mice.

Figure 1g shows advanced tumor stage causes changes in tumor-free body weight and muscle weight in mice.

Figure 1h shows the pathological state of muscle in cachexia mice observed by HE staining.

Figure 1i shows the weight of the kidney observed in the late stage of tumor implantation, the pathological state of the kidney observed by HE staining, and the expression of renal function detected by automatic biochemical analyzer.

Figure 2 shows that downregulation of membrane proteins LRP6 and Kremen2 can cause tumor cachexia.

Figure 2a shows the expression of membrane/total proteins LRP6, LRP5, Kremen1 and Kremen2 in the muscle of the advanced cachexia detected by Western blot.

Figure 2b shows the expression of membrane/total proteins LRP6, LRP5, Kremen1 and Kremen2 in the kidney of advanced cachexia detected by Western blot.

Figure 2c shows the expression of total/nucleoprotein β-catenin in kidney of advanced cachexia is detected by Western blot.

Figure 3 shows that reversal of membrane downregulation of LRP6 and Kremen2 prevents tumor cachexia.

Figure 3a shows Western blot is used to detect the changes of membrane/plasma proteins LRP6 and Kremen2 induced by intramuscular injection of Dkkl, and the reverse effect of MDC on them.

Figure 3b shows the Kaplan-Meier analysis of the combined use of MDC and Dkkl to prolong the shortening of survival in tumor-bearing mice caused by Dkkl.

Figure 3c shows that intraperitoneal injection of MDC and IGFBP4 can reverse tumor-induced loss of tumor-free body weight and muscle atrophy.

Figures 3d and 3e show Kaplan-Meier analysis of the effect of Clathin-TG2 inhibitors (MDC, Cystamine and Spermidine) on survival in tumor-bearing mice.

Figure 3f shows Western blot analysis of the expression of membrane proteins LRP6 and Kremen2 and the expression of total protein Myoglobin caused by MDC reversal of tumor.

Figure 3g shows Kaplan-Meier analysis of the effect of IGFBP4 on survival in tumor-bearing mice.

Figure 3h shows Western blot analysis of the effects of IGFBP4 on the expression of membrane/plasma proteins LRP6 and Kremen2 induced by Dkkl.

Figure 4 shows RNA-Seq analysis: Knockdown of the LRP5/6 gene broadly has altered GPCR expression.

(A) Following knockdown of LRP5/6 (red) or CTNNB1 (β-catenin, blue) by siRNA in HepG2 cells, RNA-Seq analysis has revealed the number distribution of GPCRs with expression fold changes >0.2 using Venn diagrams. (B and C) After LRP5/6 or CTNNB1 (β-catenin) is knocked down by siRNA in HepG2 cells, RNA-Seq analysis has showed the fold change of GPCR expression with histogram (B); the fold change (log₂ value) of GPCRs whose expression is up-regulated (red) or down-regulated (green) is shown as a heat map (C).

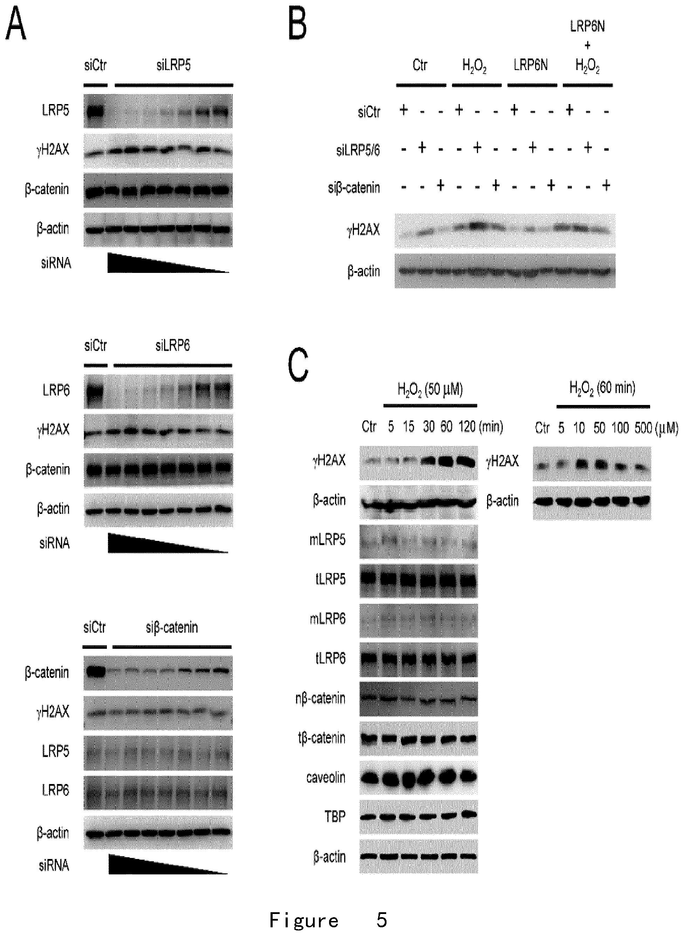

Figure 5 shows that exogenous LRP6N reverses cellular DNA damage induced by knockdown of LRP5/6.

(A) Expression of LRP5/6, β-catenin and yH2AX in HUVEC cells after LRP5, LRP6 or β-catenin is knocked down by gradient diluted siRNA for 48 h. n = 3. (B) 48 h after LRP5/6 or β-catenin knockdown, LRP6N protein pretreatment and H₂O₂ stimulation induced yH2AX response in HUVEC cells. n = 3. (C) expression of yH2AX, cell membrane or total LRP5/6 (m/tLRP5/6), nucleus or total β-catenin (n/tβ-catenin) after H₂O₂ stimulation of HUVEC cells for the indicated time (left panel) and concentration (right panel). n = 3.

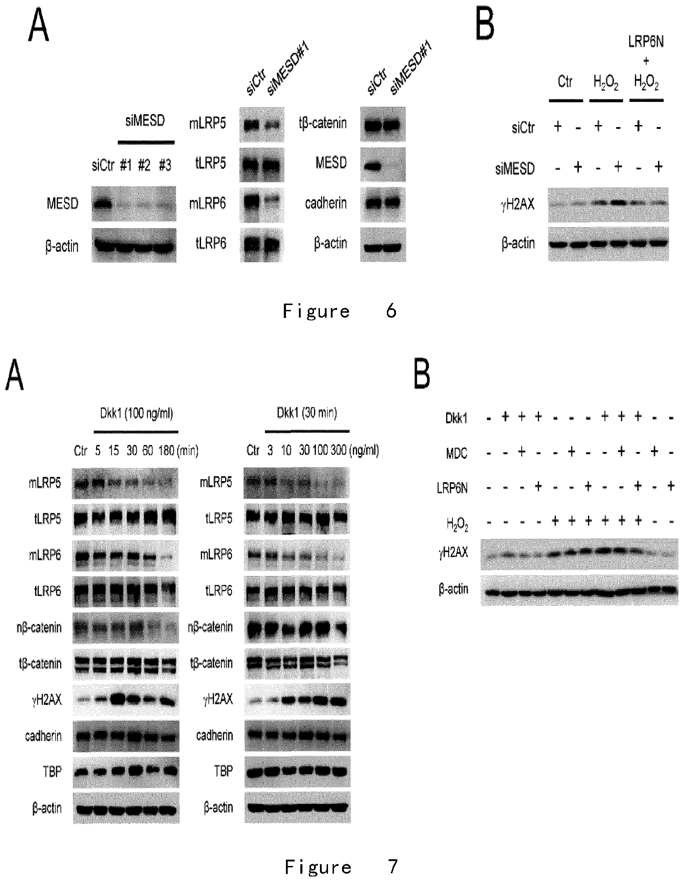

Figure 6 shows that knockdown of MESD results in downregulation of LRP5/6 on the membrane and attenuated resistance to oxidative stress.

(A) Expression of MESD, cell membrane or total LRP5/6 (m/tLRP5/6) and total β-catenin (tβ-catenin) in HUVEC cells after MESD knockdown for 48 h. n = 3. (B) After MESD is knocked down by siRNA#1 for 48 h, LRP6N protein pretreatment and H₂O₂ stimulation has induced γ H2AX response in HUVEC cells. n = 3.

Figure 7 shows that Dkkl induces LRP5/6 endocytosis and DNA damage responses at the cell membrane.

(A) expression of cell membrane or total LRP5/6 (m/tLRP5/6), cell nucleus or total β-catenin (n/tβ-catenin) and yH2AX after stimulation of HUVEC cells by Dkkl protein for the indicated time (on the left) and concentration (on the right). n = 3. (B) MDC or LRP6N protein pretreatment combined with Dkkl protein and H₂O₂ stimulated the yH2AX response induced by HUVEC cells alone or successively. n = 3.

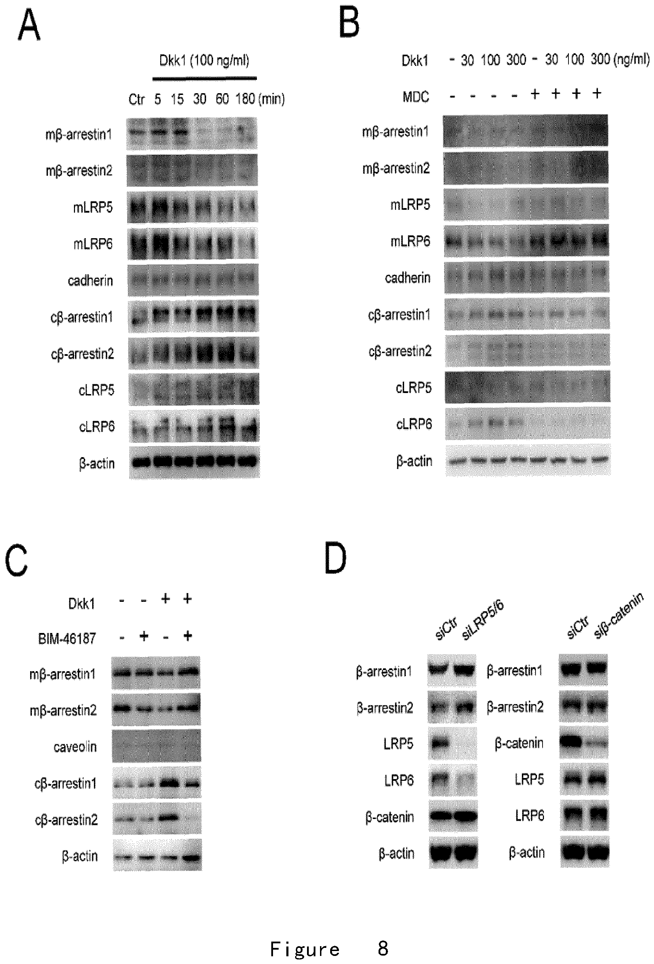

Figure 8 shows that Dkkl activates β-arrestin1/2 signaling by downregulating LRP5/6 on the membrane.

(A and B) Expression of cell membrane or cytoplasm β-arrestin1/2 (m/cβ-arrestin1/2) and LRP5/6 (m/cLRP5/6), after MDC pretreatment combined with Dkkl protein at indicated times (A) and concentrations (B) to stimulate HUVEC cells, n = 3. (C) Expression of cell membrane or cytoplasm β-arrestin1/2 (m/cβ-arrestin1/2) after BIM-46187 pretreatment combined with Dkkl protein to stimulate HUVEC cells. n = 3. (D) Expression of β-arrestin1/2, LRP5/6 and β -catenin in HUVEC cells 48 h after LRP5/6 or β-catenin knockdown. n = 3.

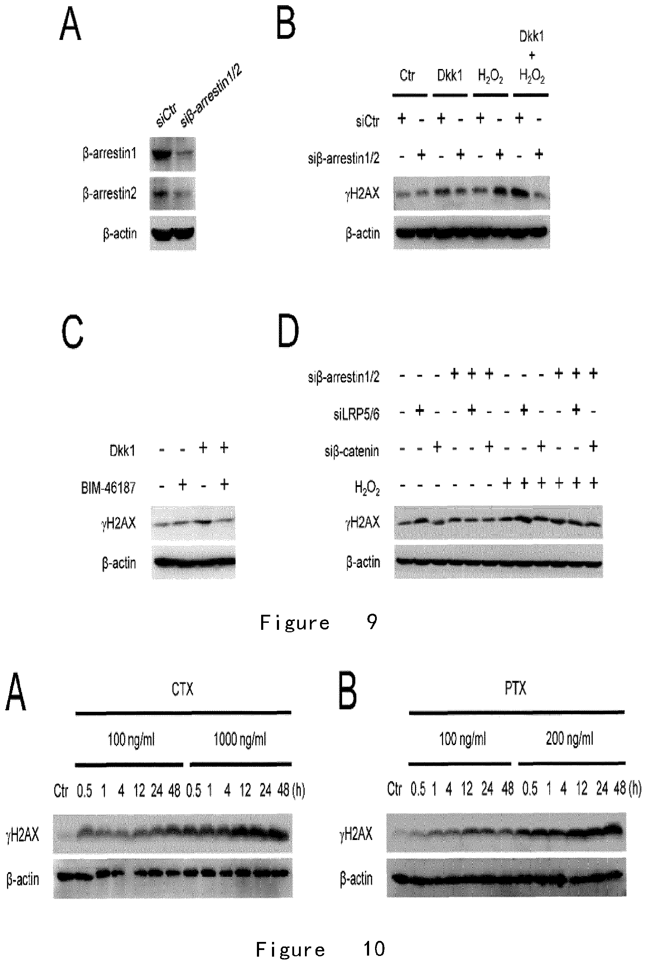

Figure 9 shows that β-arrestin1/2 mediates cellular DNA damage caused by downregulation of LRP5/6 on the membrane.

(A) Expression of β-arrestin1/2 in HUVEC cells 48 h after β-arrestin1/2 knockdown. n = 3. (B) 48 h after β-arrestin1/2 knockdown, Dkkl protein and H₂O₂ stimulate the yH2AX response induced by HUVEC cells alone or successively. n = 3. (C) Expression of yH2AX after BIM-46187 pretreatment combined with Dkkl protein to stimulate HUVEC cells. n = 3. (D) 48 h after β-arrestin1/2 combined with LRP5/6 or β-catenin knockdown, H₂O₂ stimulation has induced yH2AX response in HUVEC cells. n = 3.

Figure 10 shows that G protein agonists can induce DNA damage responses.

(A and B) CTX (A) or PTX (B) has stimulated and induced yH2AX responses in HUVEC cells at the indicated times and concentrations. n = 3.

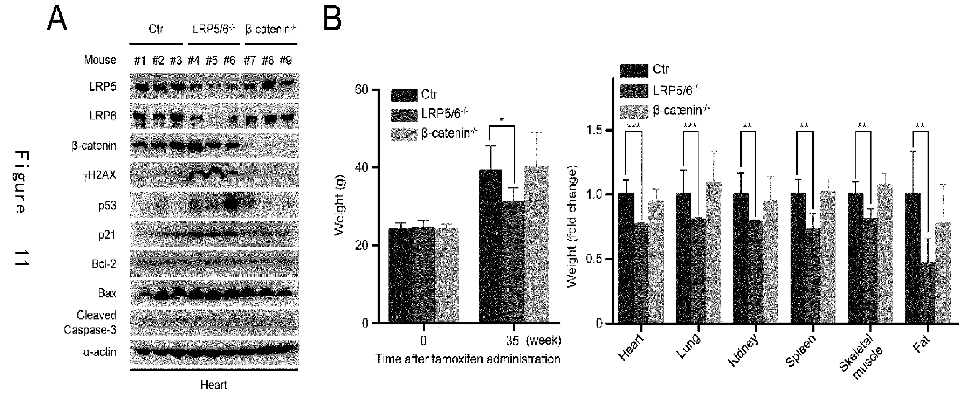

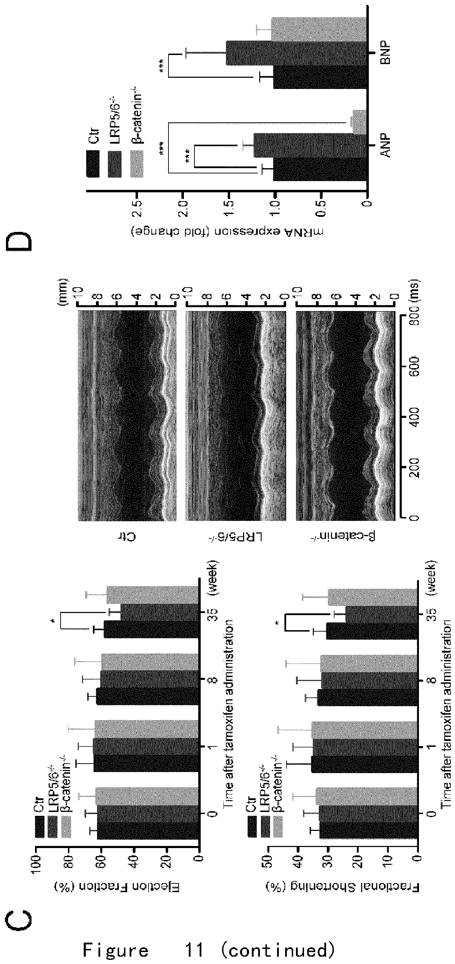

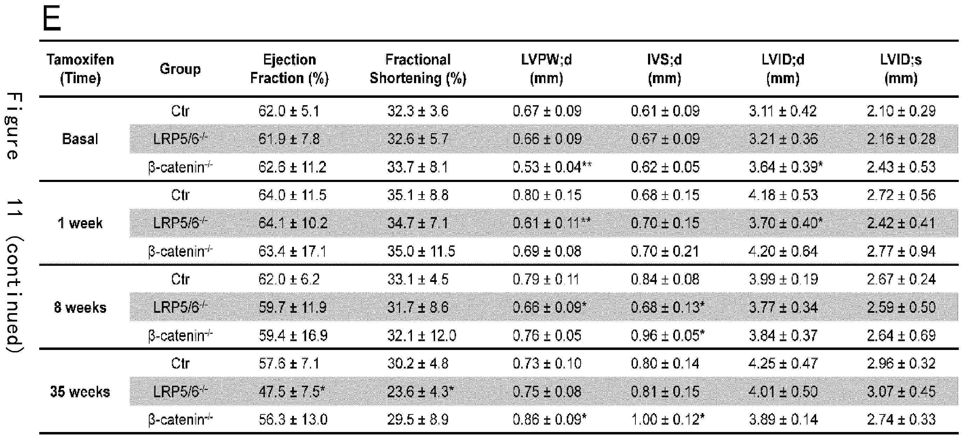

Figure 11 shows that LRP5/6 gene knockout induces cardiac injury in mice.

(A) Tamoxifen-induced control for 1 week, LRP5/6^{-/-} and β-catenin^{-/-} mice, expression of LRP5/6, β-catenin, yH2AX, p53, p21, Bcl-2, Bax and Cleaved Caspase-3 in the heart. n = 9. (B) changes in body weight (on the left) and relative weight ratios of multiple organs (heart, lung, kidney, spleen, skeletal muscle and fat) in Tamoxifen-induced controls for 35 weeks, RP5/6^{-/-} and β-catenin^{-/-} mice (on the right). n = 6. (C) Changes in left ventricular ejection fraction (EF%) and left ventricular fractional shortening (FS%) at indicated times after tamoxifen induction in control, LRP5/6^{-/-} and β**-**catenin^{-/-} mice (on the left); representative two-dimensional echocardiograms of tamoxifen-induced controls for 35 weeks, LRP5/6^{-/-} and β-catenin^{-/-} mice show left ventricular end-systolic and end-diastolic wave patterns. Flat-wave patterns in LRP5/6^{-/-} mice indicate impaired left ventricular wall muscle movement (on the right). n = 6. (D) Real-time quantitative PCR analysis. Expression of ANP and BNP in the heart of tamoxifen-induced control for 35 weeks, LRP5/6^{-/-} and β-catenin^{-/-} mice. Data are normalized by GAPDH internal reference. n = 6. (E) Values of two-dimensional echocardiographic parameters measured at indicated times after tamoxifen induction in control, LRP5/6^{-/-} and β-catenin^{-/-} mice. n = 6.

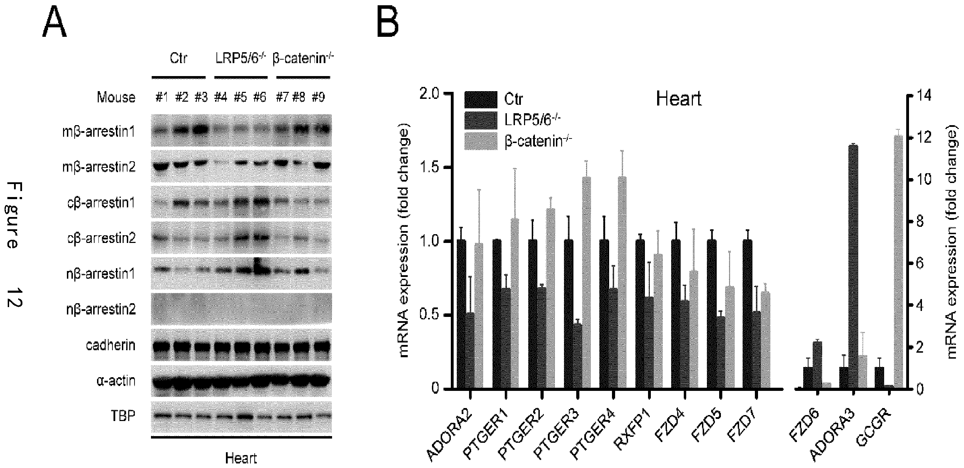

Figure 12 shows that LRP5/6 gene knockout activates cardiac β-arrestin1/2 signaling in mice.

(A) expression of cell membrane, cytoplasm and cell nuclear β-arrestin1/2 (m/c/nβ-arrestin1/2) in the heart of tamoxifen-induced control for 1 week, LRP5/6^{-/-} and β-catenin^{-/-} mice. n = 6. (B) Real-time quantitative PCR analysis. Expression of some GPCRs in the heart of Tamoxifen-induced control for 8 weeks, LRP5/6^{-/-} and β -catenin^{-/-} mice. Data are normalized by GAPDH internal reference. n = 6.

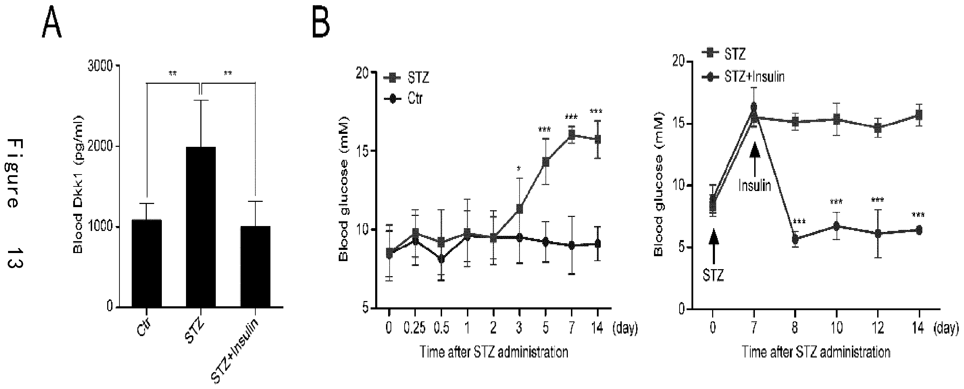

Figure 13 shows the up-regulation of Dkkl in the blood of hyperglycemia-induced diabetic mice.

(A) ELISA analysis of blood Dkkl in normal mice, diabetic mice modeled by STZ or combined with insulin treatment for 7 days. n = 8. (B) Blood glucose concentrations at indicated times in diabetic mice modeled with STZ (on the left) or in combination with insulin treatment (on the right). n = 10.

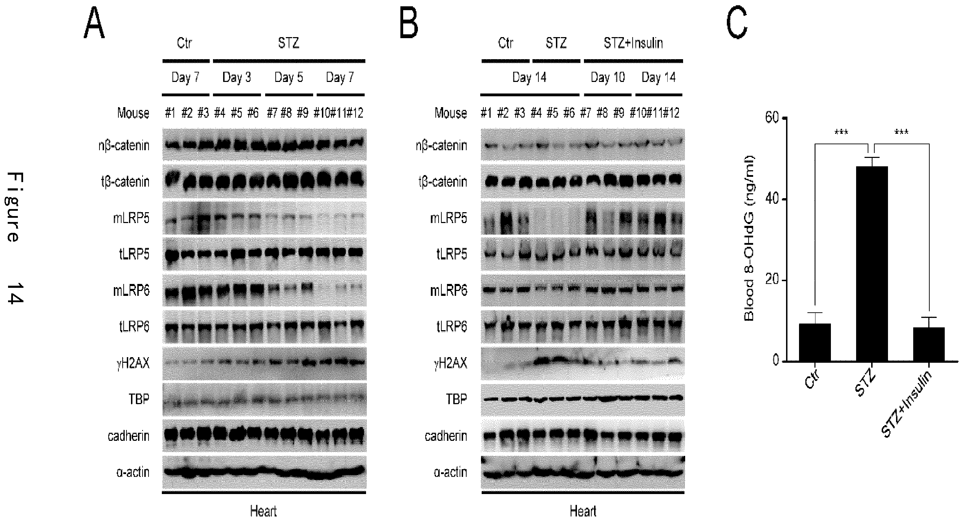

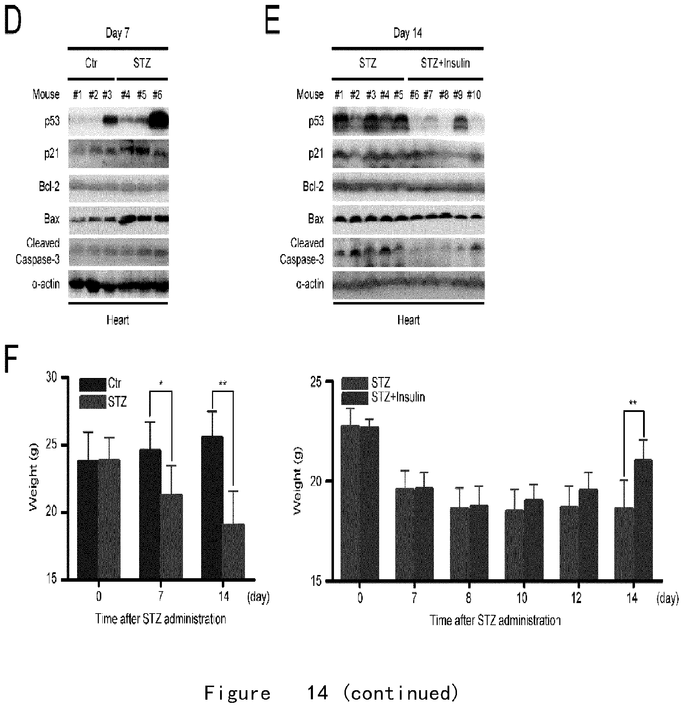

Figure 14 shows the close relationship between the downregulation of LRP5/6 on the membrane of diabetic mice and cardiac injury.

(A and B) expression of cell nucleus or total β-catenin (n/tβ-catenin), cell membrane or total LRP5/6 (m/tLRP5/6) and yH2AX in hearts of diabetic mice modeled by STZ (A) or combined with insulin treatment (B) at indicated times. n = 9. (C) ELISA analysis of blood 8-OHdG in normal mice, diabetic mice treated with STZ for 7 days or combined with insulin treatment for 7 days. n = 5. (D and E) Expression of p53, p21, Bcl-2, Bax and Cleaved Caspase-3 in the hearts of diabetic mice treated with STZ for 7 days (D) or combined with insulin treatment for 7 days (E). n = 9. (F) Changes in body weight at specified time of diabetic mice modeled by STZ (on the left) or combined with insulin treatment (on the right). n = 10.

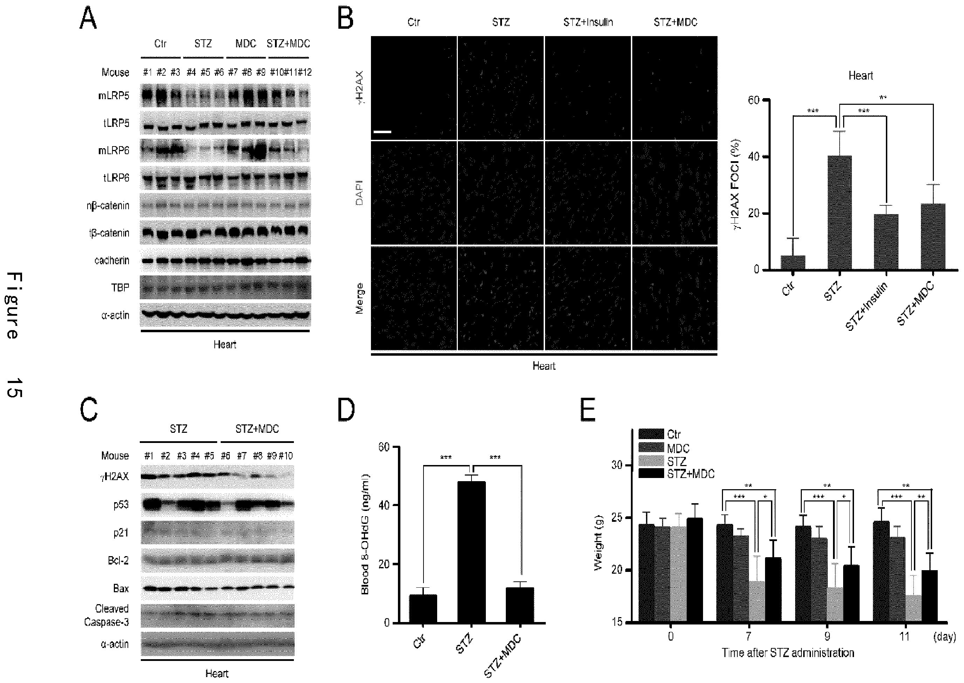

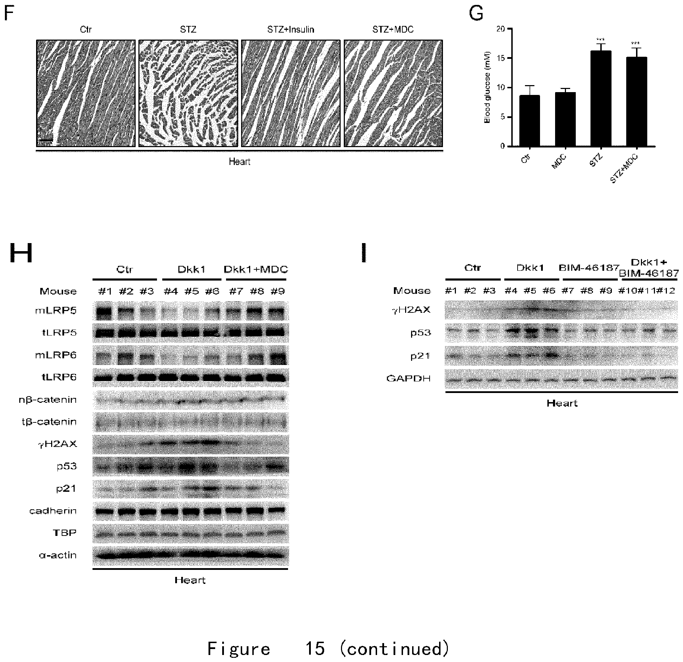

Figure 15 shows that Dkkl causes heart damage from diabetes by inducing LRP5/6 membrane endocytosis.

(A) Expressions of m/tLRP5/6 and n/tβ-catenin in the hearts of diabetic mice with STZ modeling for 7 days or combined with MDC intervention for 4 days. n = 9. (B) Immunofluorescence staining of yH2AX (red) and DAPI (blue) in the hearts of normal mice, STZ model and diabetic mice combined with insulin and MDC, respectively, scale bar is 50 µm. n = 5. (C) Expressions of yH2AX, p53, p21, Bcl-2, Bax and Cleaved Caspase-3 in the hearts of diabetic mice treated with STZ for 7 days or combined with MDC intervention for 4 days,. n = 9. (D) ELISA analysis of blood 8-OHdG in normal mice, STZ modeled or diabetic mice combined with MDC intervention. n = 5. (E) Body weight changes at indicated times in diabetic mice modeled by STZ or combined with MDC intervention. n = 10. (F) HE staining of the hearts of normal mice, STZ model and diabetic mice combined with insulin and MDC, respectively, the scale bar is 70 µm. n = 5. (G) Blood glucose concentration of diabetic mice after 7 days of STZ modeling or combined with MDC intervention for 4 days. n = 10. (H) Expression of m/tLRP5/6, n/tβ-catenin, yH2AX, p53 and p21 in hearts of wild-type mice injected with Dkkl protein intracardially or combined with MDC intervention for 2 days. n = 6. (I) Expression of yH2AX, p53 and p21 in hearts of wild-type mice injected intracardially with Dkkl protein combined with BIM-46187 for 2 days. n = 6.

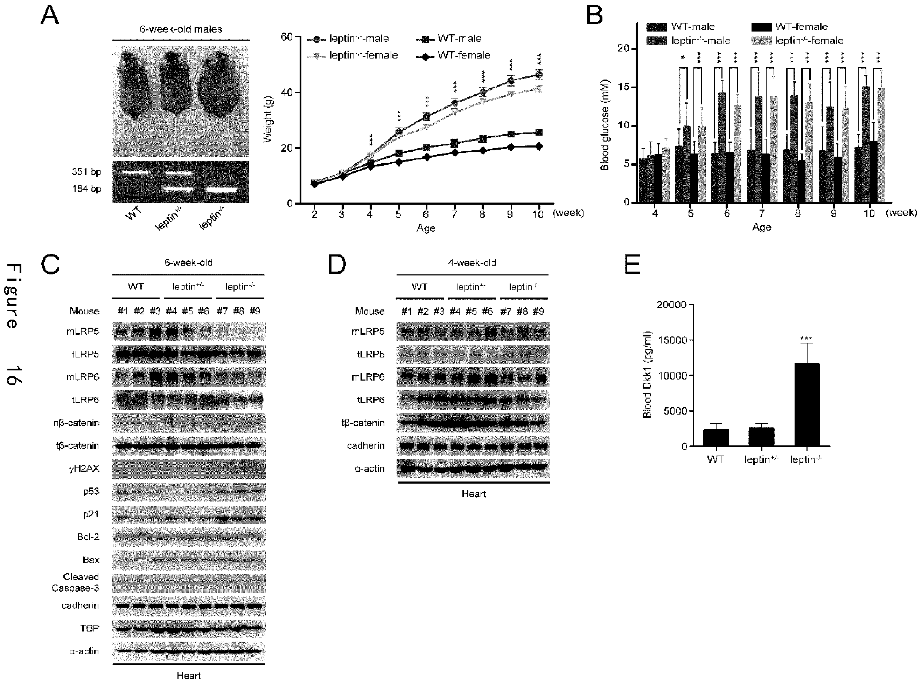

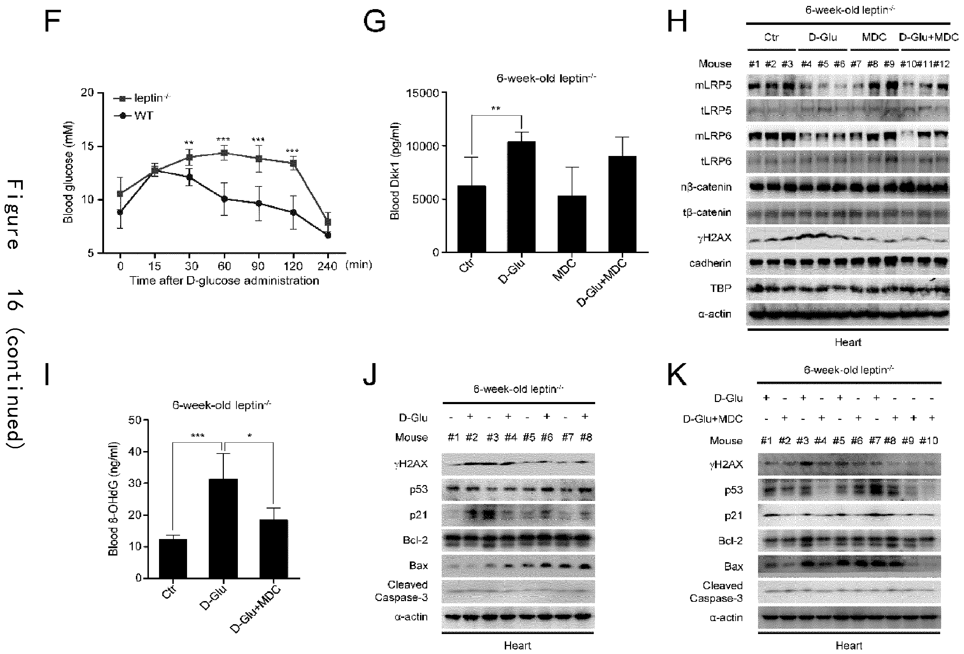

Figure 16 shows that downregulation of LRP5/6 on the membrane of Leptin^{-/-} mice leads to heart damage from diabetes.

(A and B) 6-week-old profile, genotype identification and body weight and blood glucose concentrations at different weeks of age in WT (male n = 17; female n = 9) and Leptin^{-/-} (male n = 12; female n = 9) mice. (C) Expression of m/tLRP5/6, n/tβ-catenin, yH2AX, p53, p21, Bcl-2, Bax and Cleaved Caspase-3 in hearts of 6-week-old WT, Leptin^{+/-} and Leptin^{-/-} mice. n = 6. (D) Expression of m/tLRP5/6 and tβ-catenin in the hearts of 4-week-old WT, Leptin^{+/-} and Leptin^{-/-} mice. n = 6. (E) ELISA analysis of Dkkl in blood of 6-week-old WT, Leptin^{+/-} and Leptin^{-/-} mice. n = 10. (F) Glucose tolerance test (GTT) of 6-week-old WT and Leptin^{-/-} mice. n = 6. (G and I) D-glucose (intraperitoneal injection every 2 h for 24 h) or 6-week-old Leptin^{-/-} mice combined with MDC intervention, ELISA analysis of blood Dkkl (G, n = 7) and 8-OHdG (I, n = 5). (H) Expression of m/tLRP5/6, n/tβ-catenin and yH2AX in the heart of 6-week-old Leptin^{-/-} mice of D-glucose (intraperitoneal injection every 2h for 24h) or combined with MDC intervention. n = 10. (J and K) Expression of yH2AX, p53, p21, Bcl-2, Bax and Cleaved Caspase-3 in the heart of 6-week-old Leptin^{-/-} mice of D-glucose (intraperitoneal injection every 2h for 24h) or combined with MDC intervention. n=10.

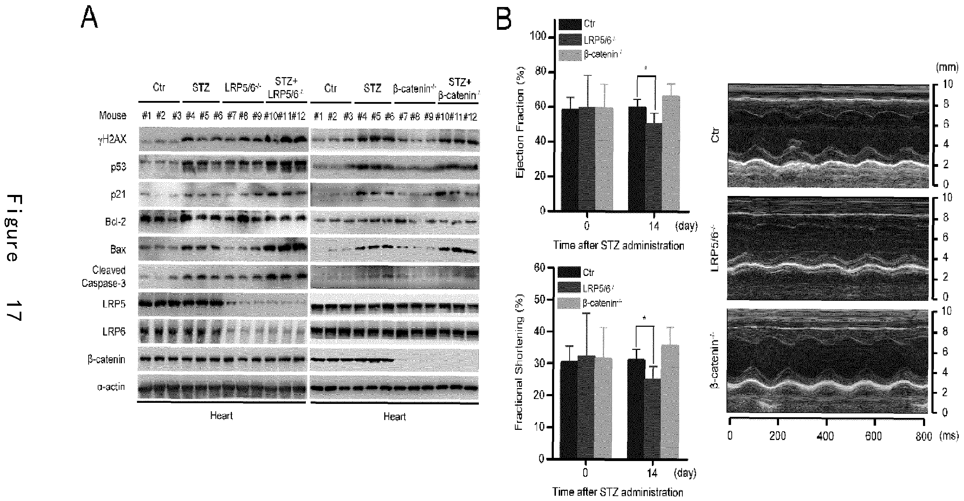

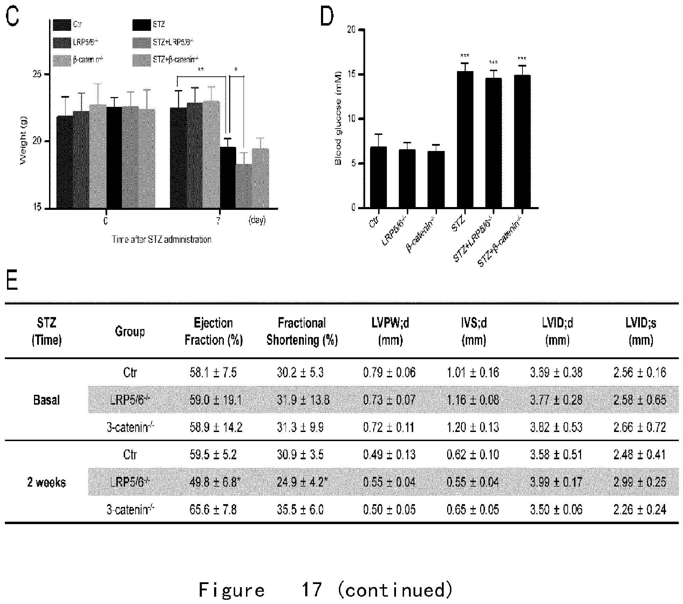

Figure 17 shows that LRP5/6 gene knockout aggravates heart damage from diabetes.

(A) Expression of yH2AX, p53, p21, Bcl-2, Bax, Cleaved Caspase-3, LRP5/6 and β-catenin in the heart of the control induced by tamoxifen for 4 weeks or combined with STZ modeling for 7 days, LRP6^{-/-} and β-catenin^{-/-} mice. n = 9. (B) Changes of left ventricular ejection fraction (EF%) and left ventricular fractional shortening (FS%) before and after STZ modeling (on the left) in the control induced by tamoxifen for 4 weeks or combined with STZ modeling for 7 days, LRP5/6^{-/-} and β-catenin^{-/-} mice (on the left); representative two-dimensional echocardiograms of control with STZ modeling for 14 days, LRP5/6^{-/-} and β -catenin^{-/-} mice, show left ventricular end-systolic and end-diastolic wave patterns. The flat wave pattern in LRP5/6^{-/-} mice indicates impaired left ventricular wall muscle movement (on the right) n = 6. (C and D) Changes in body weight (C) and blood glucose concentrations (D) of the control induced by tamoxifen for 4 weeks or combined with STZ modeling for 7 days, LRP6^{-/-} and β-catenin^{-/-} mice. n = 6. (E) The numerical value of each index of two-dimensional echocardiography measured before and after STZ modeling of the control induced by tamoxifen for 4 weeks or combined with STZ modeling for 14 days, LRP6^{-/-} and β-catenin^{-/-} mice. n = 6.

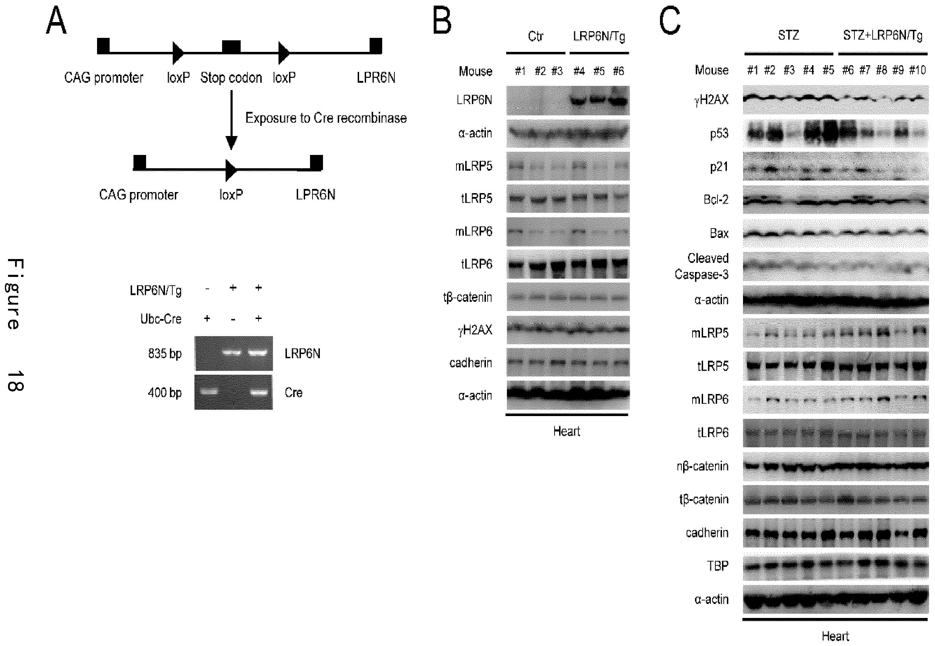

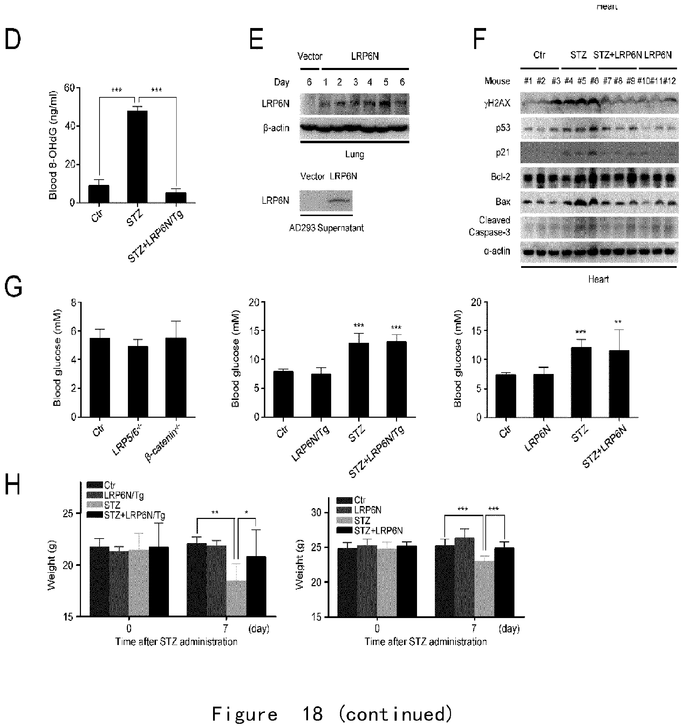

Figure 18 shows that exogenous LRP6N prevents heart damage from diabetes.

(A) Schematic diagram of the construction of the LRP6N/Tg mice strain based on the Cre-loxP recombinase system (top panel) and genotype identification of UBC-Cre-positive LRP6N/Tg mice (bottom panel). Tamoxifen-induced deletion of the loxP sequence by Cre recombinase, and the CAG promoter initiates the transcription of the myc-tagged LRP6N gene. (B) Expression of myc-tagged LRP6N, m/tLRP5/6, tβ-catenin and yH2AX in the heart of of the control induced by tamoxifen for 4 weeks and LRP6N/Tg mice. n = 6. (C) Expression of yH2AX, p53, p21, Bcl-2, Bax, Cleaved Caspase-3, m/tLRP5/6 and n/tβ-catenin in the hearts of the control of STZ modeling for 7 days and LRP6N/Tg mice. n = 9. (D) ELISA analysis of blood 8-OHdG in normal mice, STZ modeled or diabetic mice combined with LRP6N/Tg overexpression. n = 5. (E) Expression of myc-tagged LRP6N in the lungs of wild-type mice with Vector and LRP6N plasmids injected into the tail vein at the indicated times. n = 3. (F) Expression of yH2AX, p53, p21, Bcl-2, Bax and Cleaved Caspase-3 in the heart of diabetic mice after 7 days of STZ modeling and tail vein injection of Vector and LRP6N plasmids. n = 8. (G) blood glucose concentrations of Control, LRP5/6^{-/-} and β-catenin^{-/-} mice (left panel, n=12) after 35 weeks of tamoxifen induction, and that of control and LRP6N/Tg mice (middle panel, n=16), that of Vector and LRP6N plasmid-injected mice (right panel, n=16) 7 days after STZ modeling. (H) Body weight changes in control and LRP6N/Tg mice (left panel, n = 15), Vector and LRP6N plasmid-injected mice (right panel, n = 15) 7 days after STZ modeling.

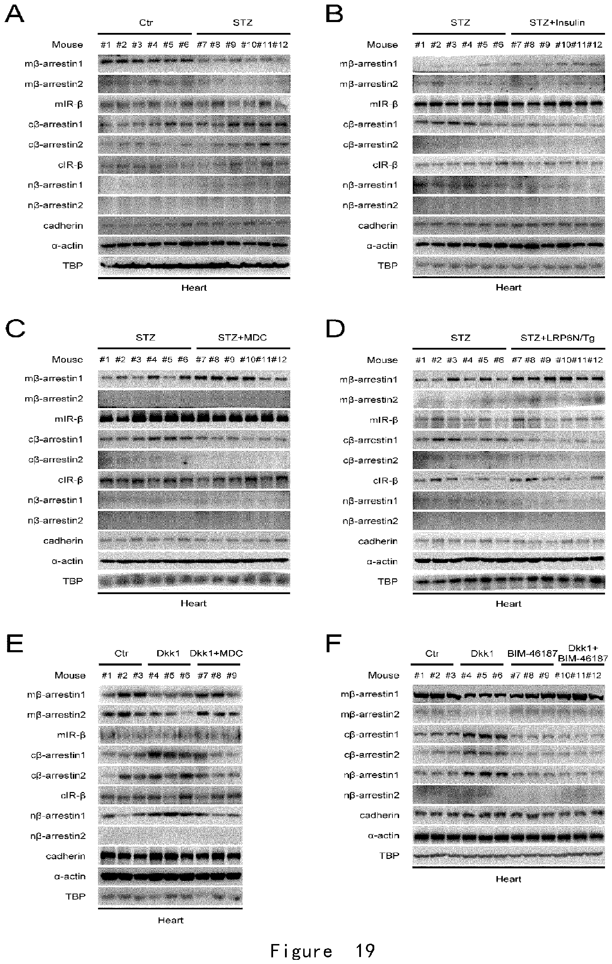

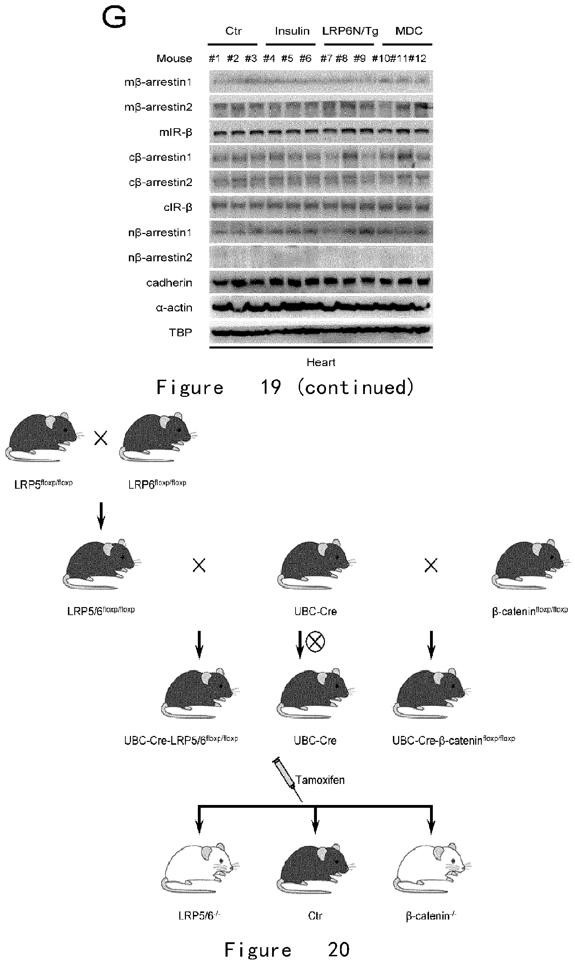

Figure 19 shows that downregulation of LRP5/6 on the membrane of diabetic mice specifically activates cardiac β-arrestin1/2 signaling.

(A) expression of cell membrane, cytoplasm and nuclear β-arrestin1/2 and cell membrane and cytoplasm insulin receptor-β (m/c/nβ-arrestin1/2 and m/cIR-β) in heart of the STZ modeled diabetic mice for 7 days. n = 6. (B) Expression of m/c/nβ-arrestin1/2 and m/cIR-β in the hearts of diabetic mice after 14 days of STZ modeling and insulin administration for 7 days. n = 6. (C) Expressions of m/c/nβ-arrestin1/2 and m/cIR-β in the hearts of diabetic mice modeled on STZ for 7 days and MDC administration for 4 days. n = 6. (D) Expression of m/c/nβ-arrestin1/2 and m/cIR-β in the heart of control after 7 days of STZ modeling or LRP6N/Tg mice. n = 6. (E) The expression of m/c/nβ-arrestin1/2 and m/cIR-β in the heart of wild-type mice with Dkkl protein intracardiac injection combined with MDC administration for 2 days. n = 6. (F) Expression of m/c/nβ-arrestin1/2 in hearts of wild-type mice with Dkkl protein combined with BIM-46187 intracardiac injection for 2 days. n = 6. (G) Expression of m/c/nβ-arrestin1/2 and m/cIR-β in the hearts of LRP6N/Tg mice in control, insulin alone for 7 days and MDC alone for 4 days. n = 6.

Figure 20 shows a schematic diagram of breeding of transgenic mice that can induce systemic LRP5 and LRP6 co-knockout or β-catenin single-knockout. Mating LRP5/6floxp/floxp or β-cateninfloxp/floxp homozygous mice with UBC-Cre-positive mice can breed inducible systemic LRP5/6 co-knockout or β-catenin single-knockout transgenic mice (UBC-Cre-LRP5/6floxp/floxp or UBC-Cre-β-cateninfloxp/floxp). The mice induced by intraperitoneal injection of tamoxifen will achieve the co-knockout of systemic LRP5/6 or the single-knockout of β-catenin (Ctr is the negative control). Such mice can be expressed as LRP5/6- /- and β-catenin-/-.

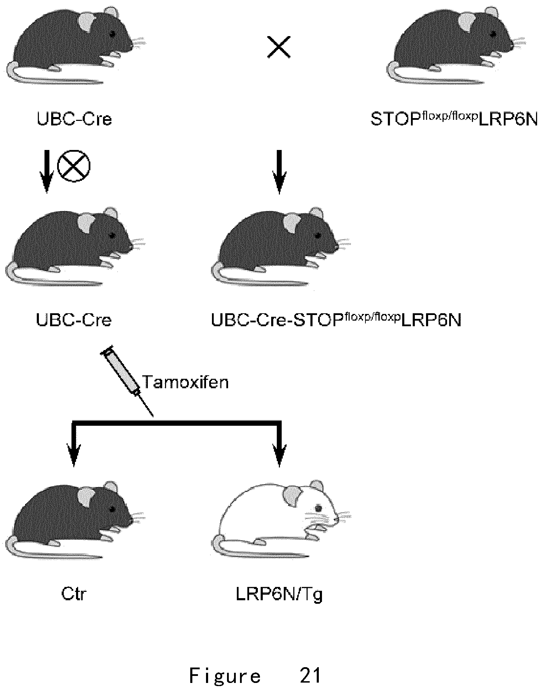

Figure 21 shows a schematic diagram of the breeding of transgenic mice that can induce systemic overexpression of LRP6N. Mating mice carrying the LRP6N gene (STOPfloxp/floxpLRP6N) with UBC-Cre-positive mice produces transgenic mice (UBC-Cre-STOPfloxp/floxpLRP6N) that induce systemic overexpression of LRP6N. The mice induced by intraperitoneal injection of tamoxifen will overexpress LRP6N (Ctr is the negative control), and such mice can be expressed as LRP6N/Tg.

### Detailed Description

After extensive and in-depth research, the inventors have unexpectedly discovered for the first time that inhibiting the expression or activity of DKK1 protein in the blood of tumor cachexia and diabetes model animals can effectively prevent and/or treat tumor cachexia and diseases accompanied with diabetes and have found that the activity of DKK1 is not related to the traditional Wnt signaling pathway inhibitor, but induces organ damage by inducing the endocytosis of its receptor LRP5/6. Moreover, the applicant has also unexpectedly found that inhibiting the binding of DKK1 and LRP5/6 (i.e., inhibiting the formation of complexes of DKK1 with LRP5 and LRP6) can also effectively prevent and/or treat tumor cachexia and diseases accompanied with diabetes. On this basis, the present inventors have completed the present invention.

Specifically, the experiments show that DKK1 protein has caused organ damage by inducing the endocytosis of LRP5 and LRP6, accelerated the death of animals caused by tumor cachexia and the death of animals with diabetes associated with acute myocardial infarction, without affecting the Wnt signaling pathway effector protein β -catenin. Small molecule drugs or protein drugs that prevent the down-regulation of membrane LRP5 and LRP6 caused by Dkkl can completely prevent tumor cachexia and diseases accompanied with diabetes, and prolong the survival of mice with these diseases. Genome-wide transcriptional analysis has shown that in skeletal muscle, blocking Dkkl-induced downregulation of membrane LRP5 and LRP6 by small-molecule drugs has prevented alterations in multiple GPCR signaling pathways as well as all major cachexia-related pathways. Moreover, direct intramuscular injection of Dkkl protein into the lower limbs of mice has immediately led to the activation of GPCR signaling pathways and cachexia-related pathways. These findings have established an important pathway that the development of tumor cachexia and diseases accompanied with diabetesis is not dependent on the Wnt signaling pathway, but affects the GPCR signaling pathway with Dkk1-LRP5/LRP6 as the main axis (Figure 1) and a very promising treatment plan is proposed for the prevention and/or treatment of tumor cachexia and diseases accompanied with diabetes. Thus, although tumor cachexia and diabetes have their own specific causes, alterations in the DKK1-LRP5/6 pathway play the same organ-damaging role in both diseases.

### tumor cachexia

Cachexia is also known as cachexy, which is manifested as extreme weight loss, skinny, skeleton-like, anemia, weakness, complete bedridden, unable to take care of themselves, extreme pain, systemic failure and other syndromes. Most are caused by cancer and other serious chronic diseases. It can be regarded as a poisoning state caused by the disorder of many organs in the body. Tumor cachexia, accompanied by weight loss, severely impairs quality of life, limits cancer treatment, and shortens survival, however, there is currently no effective treatment. Muscle wasting, a key feature of tumor cachexia, is a multifactorial disease that negatively impacts patient outcomes and quality of life. Skeletal muscle wasting is considered a meaningful prognostic factor during cancer development, regardless of body mass index (BMI), and is associated with increased incidence of chemotherapy toxicity, shorter time to tumor progression, poorer surgical outcomes, physical impairment, and shorter survival.

### diabetes

Diabetes (Diabetes mellitus (DM)) is a group of systemic metabolic diseases characterized by chronic hyperglycemia caused by defects in insulin secretion or action caused by multiple etiologies. Long-term sugar, fat and protein metabolism disorders can cause multiple organ damage and homeostasis imbalance, which can lead to chronic diseases, functional decline, and even failure and other complications of the heart, kidneys, eyes, nerves, blood vessels and other tissues and organs. Type 1 diabetes (diabetes mellitus type 1, T1DM) and type 2 diabetes (diabetes mellitus type 2, T2DM) are two important types of diabetes with different pathogenesis. The continuous increase of blood sugar is a key factor in the DNA damage of multiple organs caused by diabetes. Therefore, how to reduce the cell damage caused by blood sugar while using insulin to lower blood sugar for treatment is an important scientific issue.

### DKK1 protein and polynucleotides

In the present invention, the terms "protein of the present invention", "DKK1 protein" and "DKK1 polypeptide" are used interchangeably, and all refer to a protein or polypeptide having the amino acid sequence of DKK1. They include DKK1 proteins with or without the starting methionine. In addition, the term also includes full-length DKK1 and fragments thereof. The DKK1 protein of the present invention includes its complete amino acid sequence, its secreted protein, its mutants and its functionally active fragments.

DKK1 secreted protein is a classic Wnt/β-catenin inhibitor that inhibits the activation state of classic Wnt/β-catenin by directly binding to LRP5/6 receptors and Kremen receptors to mediate LRP5/6 endocytosis. Mouse Dkkl (NP_034181.2), composed of 272 amino acids, and the sequence as shown in SEQ ID NO.: 3; human Dkkl (NP_036374.1), composed of 266 amino acids, the sequence as shown in SEQ ID NO.: 1.

In the present invention, the terms "DKK1 gene" and "DKK1 polynucleotide" are used interchangeably, and both refer to a nucleic acid sequence having a DKK1 nucleotide sequence.

The full-length genome of the human DKK1 gene:
Human DKK1 DNA sequence (801 nt), NCBI accession number: NM_012242.4

ATGATGGCTCTGGGCGCAGCGGGAGCTACCCGGGTCTTTGTCGCGAT
GGTAGCGGCGGCTCTCGGCGGCCACCCTCTGCTGGGAGTGAGCGCCACCT
TGAACTCGGTTCTCAATTCCAACGCTATCAAGAACCTGCCCCCACCGCTG
GGCGGCGCTGCGGGGCACCCAGGCTCTGCAGTCAGCGCCGCGCCGGGAA
TCCTGTACCCGGGCGGGAATAAGTACCAGACCATTGACAACTACCAGCCG
TACCCGTGCGCAGAGGACGAGGAGTGCGGCACTGATGAGTACTGCGCTA
GTCCCACCCGCGGAGGGGACGCAGGCGTGCAAATCTGTCTCGCCTGCAGG
AAGCGCCGAAAACGCTGCATGCGTCACGCTATGTGCTGCCCCGGGAATTA
CTGCAAAAATGGAATATGTGTGTCTTCTGATCAAAATCATTTCCGAGGAG
AAATTGAGGAAACCATCACTGAAAGCTTTGGTAATGATCATAGCACCTTG
GATGGGTATTCCAGAAGAACCACCTTGTCTTCAAAAATGTATCACACCAA
AGGACAAGAAGGTTCTGTTTGTCTCCGGTCATCAGACTGTGCCTCAGGAT
TGTGTTGTGCTAGACACTTCTGGTCCAAGATCTGTAAACCTGTCCTGAAA
GAAGGTCAAGTGTGTACCAAGCATAGGAGAAAAGGCTCTCATGGACTAG
AAATATTCCAGCGTTGTTACTGTGGAGAAGGTCTGTCTTGCCGGATACAG
AAAGATCACCATCAAGCCAGTAATTCTTCTAGGCTTCACACTTGTCAGAG
ACACTAA （SEQ ID NO.:2）

The full-length genome of the murine DKK1 gene:
Mouse Dkkl DNA sequence (819 nt), NCBI accession number: NM_010051.3

ATGATGGTTGTGTGTGCAGCGGCAGCTGTCCGGTTCTTGGCCGTGTT
TACAATGATGGCTCTCTGCAGCCTCCCTCTGCTAGGAGCCAGTGCCACCT
TGAACTCAGTTCTCATCAATTCCAACGCGATCAAGAACCTGCCCCCACCG
CTGGGTGGTGCTGGGGGGCAGCCGGGCTCTGCTGTCAGTGTGGCGCCGGG
AGTTCTCTATGAGGGCGGGAACAAGTACCAGACTCTTGACAACTACCAGC
CCTACCCTTGCGCTGAAGATGAGGAGTGCGGCTCTGACGAGTACTGCTCC

AGCCCCAGCCGCGGGGCAGCCGGCGTCGGAGGTGTACAGATCTGTCTGG
CTTGCCGAAAGCGCAGGAAGCGCTGCATGAGGCACGCTATGTGCTGCCCC
GGGAACTACTGCAAAAATGGAATATGCATGCCCTCTGACCACAGCCATTT
TCCTCGAGGGGAAATTGAGGAAAGCATCATTGAAAACCTTGGTAATGACC
ACAACGCCGCCGCGGGGGATGGATATCCCAGAAGAACCACACTGACTTC
AAAAATATATCACACCAAAGGACAAGAAGGCTCCGTCTGCCTCCGATCAT
CAGACTGTGCCGCAGGGCTGTGTTGTGCAAGACACTTCTGGTCCAAGATC
TGTAAACCTGTCCTTAAAGAAGGTCAGGTGTGCACCAAGCACAAACGGA
AAGGCTCCCACGGGCTGGAGATATTCCAGCGCTGTTACTGCGGGAAGGC
CTGGCTTGCAGGATACAGAAAGATCACCATCAAGCCAGCAATTCTTCTAG
GCTCCACACCTGCCAGAGACACTAA （SEQ ID NO.:4）

Human and murine DKK1 share 83% similarity at the DNA level and 81% protein sequence similarity. It is understood that substitution of nucleotides in codons is acceptable when encoding the same amino acid. It is also to be understood that nucleotide changes are also acceptable when conservative amino acid substitutions arise from nucleotide substitutions.

When the amino acid fragment of DKK1 is obtained, a nucleic acid sequence encoding it can be constructed based on it, and a specific probe can be designed based on the nucleotide sequence. The full-length nucleotide sequence or its fragment can usually be obtained by PCR amplification method, recombinant method or artificial synthesis method. For the PCR amplification method, primers can be designed according to the DKK1 nucleotide sequence disclosed in the present invention, especially the open reading frame sequence, the relevant sequences can be obtained by amplification by using a commercially available cDNA library or a cDNA library prepared by conventional methods known to those skilled in the art as a template. When the sequence is long, it is often necessary to perform two or more PCR amplifications, and then splicing the amplified fragments together in the correct order.

Once the relevant sequences have been obtained, recombinant methods can be used to obtain the relevant sequences in large quantities. This is usually done by cloning it into a vector, transferring it into a cell, and isolating the relevant sequence from the propagated host cell by conventional methods.

In addition, synthetic methods can also be used to synthesize the relevant sequences, especially when the fragment length is short. Often, fragments of very long sequences are obtained by synthesizing multiple small fragments followed by ligation.

At present, the DNA sequences encoding the proteins of the present invention (or fragments or derivatives thereof) can be obtained completely by chemical synthesis. This DNA sequence can then be introduced into various existing DNA molecules (e.g., vectors) and cells known in the art.

The polynucleotide sequences of the present invention can be used to express or produce recombinant DKK1 polypeptides by conventional recombinant DNA techniques. Generally there are the following steps:
(1). Using the polynucleotide (or variant) encoding human DKK1 polypeptide of the present invention, or transform or transduce a suitable host cell with a recombinant expression vector containing the polynucleotide;
(2). Host cells cultured in a suitable medium;
(3). Separation and purification of proteins from culture medium or cells.

In the present invention, the DKK1 polynucleotide sequence can be inserted into a recombinant expression vector. In short, any plasmid and vector can be used as long as it is replicable and stable in the host. An important feature of expression vectors is that they typically contain an origin of replication, a promoter, marker genes and translational control elements.

Methods well known to those skilled in the art can be used to construct expression vectors containing the DKK1-encoding DNA sequence and appropriate transcriptional/translational control signals. These methods include in vitro recombinant DNA technology, DNA synthesis technology, in vivo recombinant technology, and the like. The DNA sequence can be operably linked to an appropriate promoter in an expression vector to direct mRNA synthesis. Expression vectors also include a ribosome binding site for translation initiation and a transcription terminator.

In addition, the expression vector preferably contains one or more selectable marker genes to provide phenotypic traits for selection of transformed host cells, such as dihydrofolate reductase, neomycin resistance, and green fluorescent protein (GFP) for eukaryotic cell culture, or tetracycline or ampicillin resistance in E. coli.

Vectors comprising the appropriate DNA sequences as described above, together with appropriate promoter or control sequences, can be used to transform appropriate host cells so that they can express the protein.

Host cells can be prokaryotic cells, such as bacterial cells; or lower eukaryotic cells, such as yeast cells; or higher eukaryotic cells, such as mammalian cells. Representative examples are: Escherichia coli, bacterial cells of the Streptomyces; fungal cells such as yeast; plant cells; insect cells; animal cells and the like.

Transformation of host cells with recombinant DNA can be performed using conventional techniques well known to those skilled in the art. When the host is a prokaryotic organism such as E. coli, competent cells capable of uptake of DNA can be harvested after exponential growth phase and treated with the CaCl₂ method. The steps used are well known in the art. Another method is to use MgCl₂. If desired, transformation can also be performed by electroporation. When the host is a eukaryotic organism, the following DNA transfection methods can be used: calcium phosphate co-precipitation method, conventional mechanical methods such as microinjection, electroporation, liposome packaging and the like.

The obtained transformants can be cultured by conventional methods to express the polypeptides encoded by the genes of the present invention. The medium used in the culture can be selected from various conventional media depending on the host cells used. Cultivation is carried out under conditions suitable for growth of the host cells. After the host cells have grown to an appropriate cell density, the promoter of choice is induced by a suitable method (eg, temperature switching or chemical induction), and the cells are cultured for an additional period of time.

The recombinant polypeptide in the above method can be expressed intracellularly, or on the cell membrane, or secreted outside the cell. If desired, the recombinant protein can be isolated and purified by various isolation methods utilizing its physical, chemical and other properties. These methods are well known to those skilled in the art. Examples of such methods include, but are not limited to: conventional renaturation treatment, treatment with protein precipitants (salting-out method), centrifugation, osmotic disruption, ultratreatment, ultracentrifugation, molecular sieve chromatography (gel filtration), adsorption chromatography, ion exchange chromatography, high performance liquid chromatography (HPLC) and various other liquid chromatography techniques and combinations of these methods.

### adeno-associated virus

Because adeno-associated virus (AAV) is smaller than other viral vectors, has no pathogenicity, and can transfect both dividing and non-dividing cells, AAV vector-based gene therapy approaches for inherited diseases have received extensive attention.

Adeno-associated virus (AAV), also known as adeno-associated virus, belongs to the genus Dependovirus of the family Parvoviridae, and is a class of single-stranded DNA-deficient viruses with the simplest structure found so far, which requires a helper virus (usually adenovirus) for replication. It encodes the cap and rep genes in two terminal inverted repeats (ITRs). ITRs play a decisive role in viral replication and packaging. The cap gene encodes the viral capsid protein, and the rep gene is involved in the replication and integration of the virus. AAV can infect a variety of cells.

Recombinant adeno-associated virus vector (rAAV) is derived from non-pathogenic wild-type adeno-associated virus, due to the characteristics of its good safety, wide range of host cells (dividing and non-dividing cells), low immunogenicity, and expressing foreign genes in vivo for a long time, it is regarded as one of the most promising gene transfer vectors and has been widely used in gene therapy and vaccine research worldwide. After more than 10 years of research, the biological characteristics of recombinant adeno-associated virus have been deeply understood, especially its application effect in various cells, tissues and in vivo experiments has accumulated a lot of information. In medical research, rAAV is used in gene therapy research for various diseases (including in vivo and in vitro experiments); at the same time, as a characteristic gene transfer carrier, it is also widely used in gene function research, disease model construction, and preparation of knockout mice, etc.

In a preferred embodiment of the present invention, the vector is a recombinant AAV vector. AAVs are relatively small DNA viruses that can integrate into the genomes of the cells they infect in a stable and site-specific manner. They are capable of infecting a wide range of cells without any effect on cell growth, morphology or differentiation, and they do not appear to be involved in human pathology. The AAV genome has been cloned, sequenced and characterized. AAV contains an inverted terminal repeat (ITR) region of approximately 145 bases at each end, which serves as the viral origin of replication. The rest of the genome is divided into two important regions with encapsidation functions: the left part of the genome containing the rep gene involved in viral replication and viral gene expression; and the right part of the genome containing the cap gene encoding the viral capsid protein.

AAV vectors can be prepared using standard methods in the art. Any serotype of adeno-associated virus is suitable. Methods for purifying vectors can be found, for example, in US Patent Nos. 6,566,118, 6,989,264, and 6,995,006, the disclosures of which are incorporated herein by reference in their entirety. The preparation of hybrid vectors is described, for example, in PCT Application No. PCT/US2005/027091, the disclosure of which is incorporated herein by reference in its entirety. The use of AAV-derived vectors for gene transfer in vitro and in vivo has been described (see, e.g., International Patent Application Publication Nos. WO 91/18088 and WO 93/09239; US Patent Nos. 4,797,368, 6,596,535 and 5,139,941, and European Patent No. 0488528, all of which are incorporated herein by reference in their entirety). These patent publications describe the use of various AAV-derived constructs in which the rep and/or cap genes are deleted and replaced by the genes of interest, as well as these constructs in vitro (entering into cultured cells) or the use of in vivo (directly into the organism) transport of the gene of interest. Replication-deficient recombinant AAVs can be prepared by co-transfection of a plasmid containing a nucleic acid sequence of interest flanked by two AAV inverted terminal repeats (ITRs) and a plasmid carrying the AAV encapsidation genes (rep and cap genes) into a cell line infected with a human helper virus (e.g., adenovirus). The resulting AAV recombinants are then purified by standard techniques.

In some embodiments, the recombinant vector is encapsidated into a virion (e.g., including, but not limited to, AAV virions of AAV1, AAV2, AAV3, AAV4, AAV5, AAV6, AAV7, AAV8, AAV9, AAV10, AAV11, AAV12, AAV13, AAV14, AAV15 and AAV16). Accordingly, the present disclosure includes recombinant virions (recombinant because it contains a recombinant polynucleotide) containing any of the vectors as described herein. Methods of producing such particles are known in the art and described in US Patent No. 6,596,535.

### DKK1 inhibitors and pharmaceutical compositions

Using the protein of the present invention, through various conventional screening methods, substances that interact with the DKK1 gene or protein, especially inhibitors and the like, can be screened.

DKK1 inhibitors (or antagonists) useful in the present invention include any substance that can inhibit the expression and/or activity of DKK1 gene or its encoded protein.

In the present invention, DKK1 inhibitors also include inhibitors that inhibit the binding of DKK1 to LRP5 and LRP6 (including inhibitors that inhibit the formation of complexes of DKK1, LRP5 and LRP6).

In the present invention, DKK1 inhibitors also include substances that inhibit the activities and/or expression levels of LRP5 and LRP6.

For example, the inhibitors of DKK1 include small molecule compounds, antibodies to DKK1, antisense RNAs of DKK1 nucleic acids, siRNAs, shRNAs, miRNAs, or activity inhibitors of DKK1.

In a preferred embodiment, the methods and steps for inhibiting DKK1 include neutralizing DKK1 protein with an antibody of DKK1, and silencing DKK1 gene with shRNA or siRNA or CRISPR reagent carried by a virus (such as adeno-associated virus).

The inhibition rate of DKK1 is generally at least 50% inhibition, preferably 60%, 70%, 80%, 90%, 95% inhibition, the inhibition rate of DKK1 can be controlled and detected based on conventional techniques, such as flow cytometry, fluorescence quantitative PCR or Western blot.

The inhibitors of the DKK1 protein of the present invention (including antibodies, small molecule compounds, antisense nucleic acids, CRISPR reagents and other inhibitors), when administered therapeutically, can inhibit the expression and/or activity of the DKK1 protein, or inhibit the expression and/or activity of LRP5 and LRP6, or inhibit the formation of the complex between DKK1 and LRP5 and LRP6, thereby preventing and/or treating tumor cachexia. In general, these materials can be formulated in a nontoxic, inert and pharmaceutically acceptable aqueous carrier medium, usually at a pH of about 5-8, preferably about pH 6-8, although pH can vary depending on the nature of the substance being formulated and the condition being treated. The formulated pharmaceutical compositions can be administered by conventional routes, including (but not limited to): topical, intramuscular, intraperitoneal, intravenous, subcutaneous, intradermal, topical administration, and autologous cells are extracted and cultured and then reinfused and the like.

The present invention also provides a pharmaceutical composition comprising a safe and effective amount of the inhibitor of the present invention (such as an antibody, a compound, a CRISPR reagent, an antisense sequence (such as siRNA), or an inhibitor) and a pharmaceutically acceptable carrier or excipient. Such carriers include, but are not limited to, saline, buffers, dextrose, water, glycerol, ethanol, and combinations thereof. The drug formulation should match the mode of administration. The pharmaceutical composition of the present invention can be prepared in the form of injection, for example, prepared by conventional methods with physiological saline or an aqueous solution containing glucose and other adjuvants. Pharmaceutical compositions, such as tablets and capsules, can be prepared by conventional methods. Pharmaceutical compositions such as injections, solutions, tablets and capsules are preferably manufactured under sterile conditions. The active ingredient is administered in a therapeutically effective amount, e.g., about 1 microgram to 10 mg/kg body weight per day.

The main advantages of the present invention include:
(1) The present invention has discovered for the first time that either inhibiting the expression or activity of LRP5 and LRP6, or inhibiting the formation of a complex between DKK1 and LRP5 and LRP6, can prevent and/or treat tumor cachexia and diseases accompanied with diabetes.
(2) The present invention has discovered for the first time that the tumor cachexia and diseases accompanied with diabetes prevented and/or treated by the present invention are tumor cachexia and diseases accompanied with diabetes unrelated to the Wnt pathway, or tumor cachexia and diseases accompanied with diabetes with normal or low expression of Wnt.
(3) The present invention has discovered for the first time that Dkkl protein accelerates the death of animals caused by tumor cachexia and diseases accompanied with diabetes by inducing the endocytosis of LRP5 and LRP6, without affecting β-catenin. Small-molecule drugs that prevent the down-regulation of membrane LRP5 and LRP6 caused by Dkkl can completely prevent tumor cachexia and diseases accompanied with diabetes, and prolong the survival of mice.
(4) The present inventors have discovered for the first time that molecular drugs that block Dkkl-induced downregulation of membrane LRP5 and LRP6 have prevented alterations in multiple GPCR signaling pathways as well as all major cachexia and diseases accompanied with diabetes related pathways.
(5) The present invention has discovered for the first time that the development of tumor cachexia and diseases accompanied with diabetesis mainly through Dkk1-LRP5 and LRP6 as the main axis, which affects the GPCR signaling pathway and is accompanied by the occurrence of inflammatory response.

The present invention will be further described below in conjunction with specific embodiments. It should be understood that these examples are only used to illustrate the present invention and not to limit the scope of the present invention. The experimental method of unreceipted specific conditions in the following examples, usually according to conventional conditions, such as people such as Sambrook, molecular cloning: conditions described in laboratory manual (New York: Cold Spring Harbor Laboratory Press, 1989), or according to manufacture conditions recommended by the manufacturer. Percentages and parts are weight percentages and parts unless otherwise specified.

Unless otherwise specified, the materials and reagents used in the examples of the present invention are all commercially available products.

### Experimental method

### Relevant experimental methods for cachexia research:

### 1. Cell culture and reagents

CT26 colon cancer cell lines were cultured in RPMI-1640 medium supplemented with L-glutamine (Biosera, USA) and 1% penicillin/streptomycin (Corning, USA) and 10% fetal bovine serum (Biosera, USA). Cultured in a 37°C incubator, recombinant mouse Dkkl and IGFBP-4/H95P proteins were purchased from Genscript. Mouse monoclonal antibody (mAb) to human Dkkl was produced by Shanghai Sanyou Biopharmaceutical Co., Ltd.. MDC was purchased from Sigma, dissolved in DMSO, diluted with PBS before use.

### 2. Survival analysis of cancer patients

Patients with a certain tumor type were divided into groups with high and low Dkkl expression based on the median expression of the indicated genes. The overall survival curve was generated online based on TCGA database and Oncolinc software analysis, and Kaplan-Meier survival analysis combined with Log-rank significance test was used for survival analysis.

### 3. Tumor implantation and modeling

Male BALB/c six-week-old mice were purchased from Shanghai Slack Laboratory Animal Co., Ltd. and raised in a special sterile environment. All animal studies were approved by the Animal Ethics Committee of Fujian University of Traditional Chinese Medicine and conducted in accordance with the Ethics Guidelines of the National Institutes of Health. Mice were randomly divided into two groups. The tumor-bearing mice were injected subcutaneously with 1^{∗}10⁶ CT26 tumor cells on the right side, and the control group was injected with an equal volume of PBS at the same site. Mice body weight, food intake and tumor volume were recorded every 3 days. Eight mice in each group were sacrificed on the 15th, 20th, 25th, 30th, 40th and 50th days after tumor implantation, respectively, and the early, middle and advanced stages of cachexia were detected. From day 20 after CT26 cell implantation, MDC (10 mg/kg) containing 12% DMSO or PBS was subcutaneously injected every three days as a control. Survival experiments were performed from the 40th day after CT26 cell implantation, intraperitoneal injection of PBS, Dkkl (0.25mg/kg), Dkkl comprehensive antibody (0.2gm/kg), MDC (10mg/kg) or IGFBP-4/H95P (1.7mg/kg) every three days, the survival period of tumor-bearing mice was observed. Before drug treatment, mice were randomly analyzed to ensure equal mean tumor volumes in each group. The tumor size was measured with vernier calipers every 4 days, and the calculation formula of tumor volume was V=(L^{∗}W2)/2. Tumor weight was estimated by multiplying tumor density by tumor volume, tumor density was determined by comparing the tumor reagent weight and tumor volume at the time of mouse sacrifice. Then subtracting the tumor weight from the mouse body weight to obtain the tumor-free body weight. At the end of the experiment, the mice were sacrificed, and the sampling sites: quadriceps femoris, kidneys and other organs were weighed at the same time. Blood samples were placed in centrifuge tubes containing edta after cardiac puncture, plasma samples were obtained at 2000rpm at 4°C, and stored at -80° C until testing. Plasma Dkkl levels were determined using an ELISA kit specifically for mouse Dkkl (R&D, USA) according to the manufacturer's instructions.

### 4. Intramuscular injection of Dkkl

Wild-type mice and β-arrestin2+/-KO mice were injected into the left quadriceps (0.25 mg/kg) and an equal volume of PBS was injected into the right leg as controls. MDC (10 mg/kg), IGFBP-4/H95P (1.7 mg/kg) or PBS were injected in combination with Dkkl as a control to observe the effects of drugs on Dkkl-induced signal transduction. After 48 hours, the mice were sacrificed, and the left and right quadriceps were taken to measure the plasma cytokine levels.

### 5. Western blot analysis

Membrane proteins were extracted according to the instructions of Membrane Protein Extraction Kit (Sangon Biotech, China). Western blot analysis omitted. Antibodies for WB analysis were purchased from Cell Signaling Technology and Proteintech.

### 6. Real-time PCR analysis

Total RNA was extracted and isolated according to the instructions (Takara, Japan). The relative mRNA levels were calculated by the comparative CT method and normalized to the control group.

The detection primers were as follows:
gene sequence
GAPDH (Forward) 5'- TGGCCTTCCGTGTTCCTAC -3' (SEQ ID NO.:5)
Reverse 5'- GAGTTGCTGTTGAAGTCGCA -3' (SEQ ID NO.:6)
Dkkl Forward 5'- TTGCCGAAAGCGCAGGAAC -3' (SEQ ID NO.:7)
Reverse 5'- CCTCGAGGTAAATGGCTGTG -3' (SEQ ID NO.:8)
Kremen2 Forward 5'-ACGACTAGGCATCTATGAAGTGT-3'(SEQ ID NO.:9)
Reverse 5'- CCGGTCTGGTCCATACTCATC -3'(SEQ ID NO.:10)
IL1β Forward 5'- GACCCCAAAAGATGAAGGGCT -3'(SEQ ID NO.:11)
Reverse 5'- TGATGTGCTGCTGCGAGATT -3'(SEQ ID NO.:12)
IL6Forward 5'- TGATGGATGCTACCAAACTGGA -3'(SEQ ID NO.:13)
Reverse 5'- CTGAAGGACTCTGGCTTTGTCT -3'(SEQ ID NO.:14)
TNFα Forward 5'- GCACAGAAAGCATGATCCGC -3'(SEQ ID NO.:15)
Reverse 5'- TCATCCCTTTGGGGACCGAT -3'(SEQ ID NO.:16)
Gpr153 Forward 5'- CACACAGTTATGGGCATCTGG -3'(SEQ ID NO.: 17)
Reverse 5'- GGGTGTAGAAACGCTCGCTC -3'(SEQ ID NO.:18)
CalcrlForward 5'- ATCTCAGCAGAGTCGGAAGAA -3' (SEQ ID NO.: 19)
Reverse 5'- CAGGTCCTATTGCAGTAAAGGC -3' (SEQ ID NO.:20)
Htr7 Forward 5'- CCTTACCTCCTCTCTTCGGATG -3' (SEQ ID NO.:21)
Reverse 5'- TGGAGTAGATCGTGTAGCCAAA -3' (SEQ ID NO.:22)
S1pr3 Forward 5'- ACTCTCCGGGAACATTACGAT -3' (SEQ ID NO.:23)
Reverse 5'- CAAGACGATGAAGCTACAGGTG -3' (SEQ ID NO.:24)
Adrb3 Forward 5'- GGCCCTCTCTAGTTCCCAG -3' (SEQ ID NO.:25)
Reverse 5'- TAGCCATCAAACCTGTTGAGC -3' (SEQ ID NO.:26)
Gpr35 Forward 5'- ACAACCTGTAACAGCACCCTC -3' (SEQ ID NO.:27)
Reverse 5'- GCGATAGCAGAATACCCAGAGT -3' (SEQ ID NO.:28)
Hcar1 Forward 5'- TCGTGCTGTCTCATCGAGG -3' (SEQ ID NO.:29)
Reverse 5'- TCTTCATGTGAAAGCAGAAGCC -3' (SEQ ID NO.:30)
Casr Forward 5'- AGCAGGTGACCTTCGATGAGT -3' (SEQ ID NO.:31)
Reverse 5'- ACTTCCTTGAACACAATGGAGC -3' (SEQ ID NO.:32)
Adgre1 Forward 5'- TTGTACGTGCAACTCAGGACT -3' (SEQ ID NO.:33)
Reverse 5'- GATCCCAGAGTGTTGATGCAA -3' (SEQ ID NO.:34)
C3ar1 Forward 5'- TCGATGCTGACACCAATTCAA -3' (SEQ ID NO.:35)
Reverse 5'- TCCCAATAGACAAGTGAGACCAA -3' (SEQ ID NO.:36)
Ccr2 Forward 5'- ATCCACGGCATACTATCAACATC -3' (SEQ ID NO.:37)
Reverse 5'- TCGTAGTCATACGGTGTGGTG -3' (SEQ ID NO.:38)

### 7. RNA-seq analysis

Total RNA was extracted using Trizol kit (Lige technology), RNA sequencing library was constructed from the extracted RNA using Illlumina library preparation protocol, RNA-seq was performed on Illlumina HiSeq4000 platform, using two-terminal protocol, the length of both regions was 150bp. Sequencing reads were aligned to annotated mouse transcripts (mm10) using hisat2 (v2.1.0). Using GFOLD (v1.1.4) to calculate the gene expression level and has found differentially expressed genes. The parameters are as follows: GFOLD value ≠ 0, and the changing value of the two samples is ≥ 1.35. The RNA-seq data collector GSE121873 was uploaded to the gene expression comprehensive database. KEGG pathway analysis was performed on the defined genes. The up-regulated DEGs in the quadriceps femoral muscle of CT26 tumor mice for 50 days or DEGs were up-regulated and down-regulated in quadriceps femoris of mice injected with Dkkl protein for 24 hours and control mice, respectively and the muscles of MDC-treated mice were improved.

### 8. Statistical analysis

Statistical analysis was performed using Graphpad Prism software. The results are expressed as ± standard deviation, and the Kolmogorov-Smirnov test was used to analyze the normality of the transformed data, and the T test was used to compare the differences between the two groups; one-way ANOVA was used to compare differences in three or more components, and then LSD-t post hoc analysis was performed. P<0.05 was considered statistically significant.

### Diabetes research related experimental methods:

### 1. Cryopreservation, recovery, passage and plate division of cells

When the confluence of adherent cultured cells reaches 80%, cryopreservation, passage or plate division can be performed. One 10 cm dish of cells can be cryopreserved in 3 tubes or passaged into 3 of 10 cm dishes. The cryopreservation and recovery of cells follow the principle of "slow freezing and quick thawing". Gradient cooling should be used during cryopreservation, and rapid thawing should be performed during recovery.

### A. Cell cryopreservation:

(1) removing the culture medium in the 10 cm petri dish, adding 3 ml of PBS, and gently shaking the petri dish.
(2) removing PBS, adding 1 ml trypsin, gently shaking the culture dish to make the trypsin completely cover the adherent cells, and placing the culture dish in a cell incubator for 5 min.
(3) Taking out the culture dish and observing whether the cells are completely spherical or detached from the wall. If they have become round, adding 1 ml of complete medium to stop the trypsin digestion, gently pipetting the cells to suspend them, and collecting the cell suspension to 15 ml centrifuge tube.
(4) Centrifuged at room temperature, 1000 rpm for 5 min.
(5) Preparing a freezing solution (90% complete medium + 10% DMSO), mixed well, and letting it stand to cool to room temperature.
(6) removing the cell supernatant after centrifugation, first mixing the cell pellet with 1 ml of freezing solution, then adding the remaining freezing solution, and continuing to mix by pipetting.
(7) Dispensing the cell suspension into cryopreservation tubes, 1 ml/tube, quickly putting it into a cryopreservation box, and then putting it in a -80°C refrigerator. After 48 hours, the cryopreservation tube is transferred to a liquid nitrogen tank for permanent save.

### B. Cell recovery:

(1) Removing the cells from the liquid nitrogen tank and thawing them quickly in a 37°C water bath.
(2) Centrifuged at room temperature, 1000 rpm for 5 min.
(3) Removing the supernatant, adding 1 ml of complete medium, shaking the cell pellet evenly, and then transferring it to a 10 cm petri dish (containing 7 ml of complete medium), shaking it back and forth in the incubator to mix evenly, and letting it stand for culture.

### C. Cell passage:

(1) Following the first 4 steps of cryopreservation.
(2) Removing the cell supernatant after centrifugation, first mixing the cell pellet with 1 ml of complete medium, then adding an appropriate amount of medium, and continuing to mix by pipetting.
(3) Adding 1 ml of cell suspension to each 10 cm petri dish (containing 7 ml of complete medium), shaking it up and down in the incubator, and letting it stand for culture.

### D. Cell division:

(1) Following the first 4 steps of cryopreservation.
(2) Removing the supernatant, adding 5 ml of complete medium, and mixed by pipetting.
(3) Diluting 100 µl of cell suspension by 10 times, and then taking 20 µl of the diluted solution to a hemocytometer for counting, counting the number of cells in the four large squares, and calculating the mean multiplied by 10⁵ to obtain the number of cells per milliliter in the primary cell suspension.
(4) Adding a cell suspension containing a certain number of cells to each 3.5 cm petri dish (containing 2 ml of complete medium), mixed well in the incubator, and then letting it stand for culture.

### 2. Gene knockdown of cells

Gene knockdown is an RNA interference (RNAi) technology, which is different from gene knockout, unlike gene knockout, which permanently silences the expression of target genes, it efficiently and specifically degrades mRNAs with homologous sequences in cells through double-stranded small interfering RNA (siRNA), thereby blocking the expression of the target gene, making the cell appear the phenotype of target gene deletion.
(1) First, changing the medium of HUVEC cells that have been cultured for 24 h in a 3.5 cm dish, and replacing the complete medium with a medium without double antibody and with 10% FBS.
(2) The transfection system of a 3.5 cm petri dish was prepared as follows: 1 µl of siRNA Oligo/Red (concentration of the original solution was 20 µM) was added to an EP tube containing 250 µl of pure Opti-MEM and mixed, and 1.5 µl of RNAiMAX (transfection Reagent) was added to the flow tube containing 250 µl of pure Opti-MEM and mixed, and then all the liquid in the EP tube was added to the flow tube and mixed, and allowed to stand at room temperature for 15 min.
(3) The prepared transfection system was added to a 3.5 cm culture dish, and the cells were cultured for 24-48 h, then the samples were collected and the total cell protein was extracted, and the knockdown effect of the gene was identified by Western Blotting.

### 3. RNA-Seq analysis

Transcriptome sequencing libraries were constructed from extracted RNA samples using standard Illumina library preparation protocols. Libraries were subjected to RNA-Seq analysis on the Illumina HiSeq 2000 sequencing platform using the Pair-End protocol with a read length of 100 bp. The reads were aligned to existing human transcriptome data (hg19) using two mismatched Tophat-2.0.9. Unique read mappings were preserved in gene expression profiles. Differentially expressed genes were determined by the tool Cuffdiff. For each transcript, the expression level of a gene was assessed by counting the Reads Per Kilo bases per Million reads (RPKM) from a gene.

### 4. Plasmid transformation, amplification and cell transfection

### A. Transformation of plasmids:

(1) Taking out the competent bacteria from the -80°C refrigerator and thawed on ice.
(2) Turning on the UV lamp of the bacteria-specific ultra-clean workbench for sterilization for 10 min, and turning on the water bath to heat to 42°C.
(3) Labeling several sterilized EP tubes, adding 50 µl of competent bacterial solution and 2.5 µl of plasmid to each EP tube in turn, and mixed gently.
(4) Incubating the EP tube on ice for 25 min, then placing it in a 42°C water bath for heat shock for 60 s, and inserting it into ice cubes for 90 s.
(5) Adding 1 ml of bacterial culture medium to each EP tube and putting it on a shaker at 37°C, 225 rpm, and shaked for 45 minutes.
(6) Putting the prepared bacterial culture plate (sterilized, containing Amp 1:1000) into a 37°C incubator to preheat.
(7) Bending the sterilized pipette tip, inhaling the bacterial solution and spreading it evenly on the bacterial culture plate (100 µl per plate).
(8) After the liquid was slightly dried, inverting the bacterial culture plate and placing it in a 37°C incubator for 12-16 hours.

### B. Amplification and large extraction of plasmids:

(1) Picking a medium-sized, full, single clone that was not in contact with other clones from the bacterial culture plate, and inoculating it into a 15 ml centrifuge tube containing 3 ml of 2.5% LB bacterial medium (sterilized, containing Amp 1:1000). The lid was spun gently, and placed on a shaker at 37°C, 225 rpm, for 14 h.
(2) Taking 1 ml from the small shake LB culture medium for plasmid mini-extraction, sequencing and identifying the mini-extracted products, selecting the culture solution of positive clones, inoculating 1:1000 into 100-200 ml 2.5% LB culture medium (sterilized, containing Amp 1:1000) in a large shaker flask, placed in a shaker at 37°C, 225 rpm, and shaken for 14 hours.
(3) Mixing 500 µl of bacterial solution with 500 µl of 50% sterilized glycerol from the shaken LB medium, adding it to a cryopreservation tube, recording the bacterial species information, and storing it in a -20°C refrigerator for long-term storage.
(4) Shaking the rest of the LB medium (OD600 of the bacterial liquid is generally between 2.0 and 3.0) at room temperature of 5000 g, centrifuged for 10 min, collecting the bacterial pellet into a sterilized 50 ml centrifuge tube, and aspirating the supernatant as much as possible.
(5) Plasmid extraction was performed at room temperature according to the instructions of the endotoxin-free plasmid extraction kit (TIANGEN, Beijing).
(6) Using NanoDrop to measure the concentration (µg/ml) and purity (OD260/OD280) of plasmid DNA, and the purity is usually 1.7-1.9.

### C. Cell transfection of plasmids:

(1) First, changeing the medium of AD293 cells that have been cultured for 24 h in a 3.5 cm dish, and replacing the complete medium with a medium without double antibodies and with 10% FBS.
(2) The transfection system of a 3.5 cm petri dish was prepared as follows: 3 µg of plasmid and 9 µl of L-PEI (transfection reagent, 1 µg/µl) were added to 250 µl of pure Opti-MEM and mixed well allowed to stand at room temperature for 15 minutes, then added to a 3.5 cm petri dish.
(3) After 4 hours of transfection, changing the medium (medium without double antibody and 10% FBS), and continuing to culture the cells for 24-48 hours, then the samples can be collected and the total cell protein can be extracted. For plasmids expressing secreted proteins, the culture supernatant can be collected, and the transfection effect of the plasmid can be identified by Western Blotting.

### 5. Reporter gene assay

Reporter gene assay (TOPFLASH Reporter Gene Assay) is a common method to analyze whether the classical Wnt signaling pathway is activated and its activation strength. The detection system adopts Promega's dual luciferase reporter gene detection system (Dual-Luciferase^{®} Reporter(DLRTM)Assay System). Specific steps were as follows:
(1) Reagent preparation: 1×PBS; 1×PLB (5×Passive Lysis Buffer (PLB) was diluted with ddH2O before use, the stock solution was stored at -20°C, and stored at 4°C after dilution); LAR II Reagent (10 ml thawed at room temperature) Luciferase Assay Buffer II, then pouring all into the substrate lyophilized powder (Luciferase Substrate), mixed well and used as soon as possible, avoiding repeated freezing and thawing, 100 µl per well of 96-well plate); 1×Stop & Glo^{®} Reagent (dissolving 50×Stop & Glo^{®} Substrate in a brown Stop & Glo^{®} Reagent bottle with Stop & Glo^{®} Buffer, mixed well and used as soon as possible, avoiding repeated freezing and thawing, 100 µl per well of 96-well plate).
(2) Sample preparation: using AD293 cells within two passages to plate in a 48-well plate, making 3 duplicate wells, the number of cells was 30,000 cells/well, and the medium was 300 µl/well. After overnight adherence, we conducted the following operations by examining whether LRP6 or β-catenin knockdown would inhibit the canonical Wnt pathway activated by Wnt3a as an example.

Gene knockdown was performed first, and the transfection system for each well was prepared as follows: 0.4 µl LRP6 or β-catenin siRNA Oligo (0.4 µl Oligo was diluted 1:1 to 1:32 with Annealing Buffer to 6 concentration gradients) /Red (Red as the control) was added to 20 µl of pure Opti-MEM and mixed, 0.6 µl of RNAiMAX (transfection reagent) was added to 20 µl of pure Opti-MEM and mixed, the two were further mixed, and allowed to stand at room temperature for 15 min, all added to the medium.

Plasmid transfection was carried out after 24 h, 160 ng of plasmid was co-transfected in each well, Fugene (transfection reagent) for 0.48 µl/well, Opti-MEM for 8 µl/well. 160 ng of plasmid contains 40 ng SP-TOP (Tcf/Lef-luc) reporter gene expression vector and 1 ng Renilla Renilla luciferase expression vector, and 1 ng Wnt3a plasmid or GFP plasmid (to test the transfection efficiency), and finally filled up to 160 ng with Vector, adding all of them to the medium, and continuing to culture the cells for 24 h before lysing the cells: first removing the medium in the 48-well plate; washing AD293 cells with 1×PBS, removing the washing solution; add 1× PLB was added to a 48-well plate, 65 µl/well, and the cells were fully lysed and mixed.

(3) On-machine operation and data processing: 20 µl of the above cell lysis solution was added to each well of the 96-well plate for detection. The expressions of firefly luciferase (Photinus Pyralis Luciferase) and Renilla Reniformis Luciferase (Renilla Reniformis Luciferase) were obtained by Promega's GloMax^{®} 96 Microplate Luminometer and Dual-Glo^{®} Luciferase Assay System. First adding Luciferase Assay Reagent II (LAR II) to the sample, 100 µl/well, the light signal generated by firefly luciferase lasts for at least 1 min. After quantifying the firefly fluorescence intensity, adding Stop & Glo^{®} Reagent, 100 µl/well to stop the firefly fluorescence and start the Renilla luciferase reaction. After completing the detection of the two luciferases, the firefly luciferase data were normalized according to the Renilla luciferase data.

### 6. Transgenic mice of specific genotypes.

### A. Transgenic mice containing UBC-Cre

A chimeric recombinase (mER-Cre-mER) is produced by fusing the Cre recombinase gene with the ligand-binding domains (LBD) of two estrogen receptors (ER). The expression of this chimeric recombinase is under the control of a specific ubiquitin C (UBC) promoter. Ubiquitin proteins are present in all eukaryotic cells, and thus Cre recombinase is expressed in tissues and organs throughout the body. Cre recombinase can bind to the loxP site and effectively excise the sequence between the two loxP sites to achieve gene knockout. However, the chimeric Cre cannot enter the nucleus spontaneously to exert its activity, and can enter the nucleus only after binding to estrogen. In order to avoid non-specific gene knockout caused by endogenous estrogen, the key amino acids of estrogen LBD were mutated so that it could not combine with physiological estrogen in the body,it can only bind to the exogenous estrogen analog Tamoxifen, the dual expression of Cre recombinase in time and space is realized. The above are the transgenic characteristics of UBC-Cre mice.

### B. Transgenic mice with inducible LRP5/6 or β-catenin systemic knockout

Three homozygous mice, LRP5^{floxp/floxp}, LRP6^{floxp/floxp} and β-catenin^{floxp/floxp}, were constructed similarly, for example, LRP5^{floxp/floxp} or LRP6^{floxp/floxp} homozygous mice, the first is to insert a loxP site on each side of the LRP5 or LRP6 gene region in the genome through gene targeting and homologous recombination, and screening to obtain transgenic mice containing floxp-LRP5-floxp or floxp-LRP6-floxp sites, and they were then bred and backcrossed with wild-type (WT) mice, respectively to obtain LRP5^{floxp/floxp} or LRP6^{floxp/floxp} homozygous mice ^{[131]}. Similarly, β-catenin^{floxp/floxp} homozygous mice can be obtained ^{[134]}. The LRP5^{floxp/floxp}LRP6^{floxp/floxp} transgenic mice used in this study were obtained by continuing the mating of LRP5^{floxp/floxp} and LRP6^{floxp/floxp} mice.

Next, as shown in Figure 20, we generated transgenic mice that could induce systemic co-knockout of LRP5 and LRP6 by breeding LRP5^{floxp/floxp}LRP6^{floxp/floxp} homozygous mice with the above UBC-Cre mice, using the properties of Cre recombinase, the genotype of mice induced by tamoxifen was expressed as LRP5/6^{-/-}(LRP5/6 double knockout). Similarly, transgenic mice with conditional β-catenin systemic knockout can also be obtained, and the genotype after knockout is expressed as β-catenin^{-/-} (β-catenin knockout). In this study, we intraperitoneal injected 1% tamoxib (30 mg/kg/day) into UBC-Cre-LRP5/6^{floxp/floxp} homozygous mice, UBC-Cre-β-catenin^{floxp/floxp} homozygous mice and Ubc-Cre positive mice (control group), respectively, 5 consecutive days of injection, continued to wait 1 or 4 weeks later, detection of LRP5/6 or β-catenin knockout effect in mouse heart by Western Blotting shows that compared with the control mice, the protein expression level of LRP5/6 in the hearts of LRP5/6^{-/-} mice and that of β-catenin in the heart of β-catenin^{-/-}mice were significantly decreased (as shown in Figure 17(A) and Figure 20), indicating that the induction effect of tamoxifen was successful, and LRP5/6^{-/-} and β-catenin^{-/-}mice could be used for further studies, such as modeling of type 1 diabetes.

### C. Transgenic mice that induce systemic overexpression of LRP6N

The transposon system is a transgenic model animal production system with high integration efficiency, high target gene expression probability and suitable for the insertion of larger target fragments. The unique "cut and paste" mechanism of transposon enables the "free" transfer of DNA fragments between the vector and the genome, thereby effectively mediating the integration of exogenous DNA fragments into the genome. During transposition, the transposase effectively recognizes the specific transposon sequences (ITRs) at both ends of the target fragment in the vector, and combines with the end of the transposon to form a short-term hairpin structure, which is "cut" and then detached from the vector and "pasted" to specific sites in the genome. Specifically, the constructed gene vector containing the transposon system and the CAG promoter-floxp-stop codon-floxp-LRP6N-myc cDNA fragment and the CMV promoter-transposase expression vector were subjected to microinjection of mouse fertilized egg nucleus DNA by microinjection technology, the injected fertilized eggs were transplanted into the reproductive system of pseudopregnant mice to make them pregnant and obtain neonatal mice, collect tail tips, extract DNA, use PCR for identification, and screen to prepare transposon-mediated transgenic mice carrying LRP6N gene fragments (CAG-floxp-stop codon-floxp-LRP6N).

CAG is an efficient synthetic promoter commonly used in mammalian expression vectors to drive high-level expression of genes. CAG contains the early enhancer of CMV, the promoter of β-actin and the splice acceptor of β-globin [138]. The LRP6N of the above-mentioned transgenic mice is not expressed under normal conditions, and only after crossing with the corresponding tissue-specific Cre-positive mice, the stop codon is deleted by Cre in specific tissues, therefore, the β-actin promoter of CAG enables the expression of the LRP6N gene fragment in specific tissues. Therefore, as shown in Figure 21, we mated mice containing the LRP6N gene (CAG-floxp-stop codon-floxp-LRP6N) with the UBC-Cre mice described above [133] to breed tamoxifen-inducible transgenic mice overexpressing LRP6N systemically, induced mice are expressed as LRP6N/Tg. In this study, we injected 1% tamoxifen (30 mg/kg/day) intraperitoneally into UBC-Cre-STOPfloxp/floxpLRP6N mice for 5 consecutive days and continued to wait for 4 weeks. Western Blotting detection has found compared with UBC-Cre positive mice (control group), LRP6N/Tg mice have overexpression of LRP6N protein in the heart (as shown in Figure 18(A, B) and Figure 21), indicating that the induction effect of tamoxifen is successful, and LRP6N/Tg mice can be used for further research, such as modeling of type 1 diabetes.

### D. Leptin knockout mice

Leptin knockout mice (Leptin^{-/-} mice, ob/ob mice) are homozygous mice that spontaneously develop an obese phenotype and are often used as an animal model for the study of type 2 diabetes. An obese phenotype was seen at 4 weeks, significant hyperglycemia (a concentration above 13.8 mM) and impaired glucose tolerance developed at 6 weeks. Leptin (leptin) is a protein hormone secreted by adipose tissue, which is involved in regulating the metabolism of sugar, fat and energy in the body, prompting the body to reduce food intake, increase energy release, inhibit the synthesis of fat cells, and then cause weight loss [139]. Due to the lack of leptin ligands, Leptin^{-/-}mice have a high appetite and rapid weight gain, leading to fat deposition and morbid obesity, up to three times the body weight of WT mice, and are not affected by dietary restriction. In addition to the above-mentioned manifestations, Leptin^{-/-} mice were also characterized by insulin resistance, decreased fertility, hypometabolism, impaired wound healing ability, and elevated levels of pituitary and adrenal hormones. Since Leptin^{-/-} mice were sterile, Leptin^{-/-} mice were all produced by mating of Leptin^{+/-}mice.

### 7. Mouse genotype identification

A, tissue genomic DNA extraction:
(1) Cutting the toes to mark the serial number of the mouse, and putting the cut toes into the EP tube for genomic DNA extraction. The scissors after cutting the toes should be wiped clean with alcohol cotton balls to avoid cross-contamination of the genome.
(2) Adding lysate, 50 mM NaOH for 200 (µl/tube, completely cover the sample, cooked in a metal bath at 100°C for 1 h, taking it out after 30 min, carefully flicking it, and mixing the tissue and lysate thoroughly to make the tissue as much as possible be boiled and releasing the genomic DNA, paying attention to tightly cap the EP tube to prevent the lysate from evaporating. Finally, checking whether the toe is completely lysed by NaOH. For tissues that are not sufficiently lysed, the lysis time should be appropriately extended.
(3) After the dissolution is complete, centrifuged at 1000 g for 1 min, adding 20 (µl/tube of Tris-HCl (pH 8.0) to neutralize the lysate, and performing genotype identification after the Vortex shakes and mixes.

### B. Common PCR reaction:

(1) The general PCR reaction system is prepared as follows:

| PCR system with 2 primers (20 µl) | | PCR system with 3 primers (20 µl) | |
|---|---|---|---|
| 2×Easy Taq SuperMix | 10 µl | 2×Easy Taq SuperMix | 10 µl |
| DNA template | 1 µl | DNA template | 1 µl |
| Primer 1 10 µM | 0.8 µl | Primer 1 10 µM | 0.8 µl |
| Primer 2 10 µM | 0.8 µl | Primer 2 10 µM | 0.4 µl |
| | | Primer 3 10 µM | 0.4 µl |
| ddH₂O | 7.4 µl | ddH₂O | 7.4 µl |

(2) Briefly centrifuging the 8 tubes with the added reaction system, putting them into the PCR machine, setting the reaction program according to the PCR reaction conditions of different transgenic mice as shown below, and carrying out PCR amplification.
(2.1)UBC-Cre mice:

| | | | |
|---|---|---|---|
| 94°C | 4 min | | |
| 94°C | 10 s | 35 × cycles | |
| 60°C | 10 s | | |
| 72°C | 45 s | | |
| 72°C | 8 min | | |
| 4°C | ∞ | | |

(2.2)LRP5^{floxp/floxp} LRP6^{floxp/floxp} or β-catenin^{floxp/floxp} mice:

| | | |
|---|---|---|
| 94°C | 4 min | |
| 94°C | 30 s | 35 × cycles |
| 55°C | 30 s | |
| 72°C | 50 s | |
| 72°C | 5 min | |
| 4°C | ∞ | |

(2.3)STOP^{floxp/floxp}LRP6N mice:

| | | |
|---|---|---|
| 95°C | 3 min | |
| 95°C | 30 s | 34 × cycles |
| 58°C | 30 s | |
| 72°C | 2 min | |
| 72°C | 5 min | |
| 4°C | ∞ | |

(2.4)Leptin^{-/-} mice:

| | | |
|---|---|---|
| 95°C | 3 min | |
| 95°C | 30 s | 32xcycles |
| 60°C | 30 s | |
| 72°C | 30 s | |
| 72°C | 3 min | |
| 4°C | ∞ | |

### C. PCR reaction product agarose nucleic acid electrophoresis, identification of mouse genotype:

(1) Preparing an appropriate concentration of agarose solution with 1×TAE buffer according to the size of the DNA fragments to be separated. Heated and melted in a microwave oven, cooled for a while, adding a drop of fluorescent dye (Gel-Red), rotated and mixed well, pouring it into a gel-making plate, and waiting for the solution to solidify at room temperature.
(2) Completely solidified, carefully pulling out the comb, and putting the sample hole side close to the negative electrode and putting it into the electrophoresis tank. Making sure that the 1×TAE buffer in the electrophoresis tank completely covers the gel surface to avoid air bubbles in the wells. Spotting samples into the well, 10 µl/well, and selecting the DNA Marker corresponding to the molecular weight range according to the size of the DNA band to be identified.
(3) Turning on the power and starting electrophoresis. The general voltage is 60-100 V, and electrophoresis is sufficient for 20-40 min.
(4) After electrophoresis, turning off the power. The electrophoresis bands were imaged on a nucleic acid imager, and the size of the bands was judged according to DNA Marker to determine the mouse genotype. Band sizes for specific genotypes of mice are shown in the table below:

| Mouse genotype | DNA band size |
|---|---|
| UBC-Cre | 400 bp |
| LRP5^{floxp/floxp} | 288 bp |
| LRP6^{floxp/floxp} | 411 bp |
| β-catenin^{floxp/floxp} | 400 bp |
| STOP^{floxp/floxp}LRP6N | 835 bp |
| Leptin^{-/-} | 184 bp |

### 8. Two-dimensional echocardiography

Two-dimensional echocardiography was performed using the VisualSonics Vevo 2100 imaging system in this study. The left chest of the mouse was depilated in advance, and the mouse was anesthetized with 1% isoflurane through a vaporizer while supplementing with oxygen. Physical indicators that reflect left ventricular function to be detected include left ventricular internal dimension in end-diastole (LVIDd), left ventricular posterior wall in end-diastole (LVPWd), Interventricular septum in end-diastole (IVSd) (i.e., measured during the period of maximum left ventricular volume), left ventricular internal dimensions in end-systole (LVIDs) (i.e., measured during the period of maximal contraction of the posterior wall of the left ventricle). The calculation method of left ventricular volume is the Teichholtz correction formula: V = [7.0/(2.4+D)] × D³, D represents LVIDd for calculating the left ventricular end-diastolic volume (LVEDV) at end diastole, and D represents LVIDs for calculating the left ventricular end-systolic volume (LVESV) of the end-systole. The calculation formula of left ventricular ejection fraction (LVEF) is: EF = (LVEDV-LVESV)/LVEDV× 100%, and the calculation formula of left ventricular fractional shortening (LVFS) is: FS=(LVIDd-LVIDs)/LVIDd× 100%.

### 9. Type 1 diabetes mouse model

STZ-induced diabetes is a chemical modeling method for diabetes. Its full name is streptozotocin, which is a broad-spectrum antibiotic isolated from achromatic streptomycin. It can selectively damage islet β cells in a short period of time, causing β cell necrosis, resulting in varying degrees of blood insulin drop and blood sugar rise, resulting in insulin-dependent type 1 diabetes mellitus. Compared with other chemical diabetes modeling methods such as Alloxan (ALX) modeling, STZ-induced diabetes hyperglycemia and ketosis are milder, and the diabetes model is also more stable. In this study, we established a type 1 diabetes model induced by intraperitoneal injection of STZ in mice, measured the weight of the mice with an electronic scale, and collected blood from the tail of the mice with a portable blood glucose meter to measure the blood glucose concentration. Modelling criteria are non-fasting blood glucose values above 13.8 mM (250 mg/dl). The modeling steps are as follows:
(1) 8-week-old wild-type male mice were grouped according to the principle of randomization, toes were marked, and body weight and random blood glucose were measured.
(2) Before modeling, the mice were fasted for 12 h, but could not be restrained from water.
(3) Preparing a 2% STZ-citric acid solution, that is, dissolving 2 g of STZ powder in 100 ml of citric acid buffer (0.05 M, pH 4.5), and mixed to form a light yellow transparent liquid. STZ is extremely unstable at room temperature and is easy to sublime. The operation should be carried out on ice. The prepared solution should be protected from light and used in time.
(4) According to the body weight, mice in the model group were injected intraperitoneally with 2% STZ-citric acid solution (160 mg/kg), and the mice in the control group were intraperitoneally injected with the corresponding volume of citrate buffer. 2 h after the injection, the mice were fed back.
(5) Body weight and random blood glucose were measured at different time points after injection, and the mice were anesthetized and the materials were collected.

### 10. Glucose tolerance test

Glucose tolerance test (GTT) is a standard test that shows the body's clearance efficiency of exogenous glucose from the blood, and is used to evaluate the maintenance of glucose metabolism balance in the body. The uptake of glucose by cells from the blood is regulated by insulin, and insulin resistance can lead to impaired glucose tolerance, that is, it takes a longer time to clear the glucose content than the normal body. In this study, Leptin^{-/-} and WT mice were measured for body weight and 6-h fasting blood glucose at the same time every week from the age of 4 weeks. According to the body weight and blood sugar changes of Leptin^{-/-} mice, we selected 6-week-old Leptin^{-/-}mice (experimental group) and WT mice (control group) for glucose tolerance test. Specific steps are as follows:
(1) The mice were fasted for 12 hours (without water), weighed, and blood was collected from the tail to determine the basal blood sugar level.
(2) Making up 20% and 30% D-glucose solutions with sterile PBS and filtered with 0.45 µm filter.
(3) WT mice were intraperitoneally injected with 20% D-glucose solution and Leptin^{-/-} mice were intraperitoneally injected with 30% D-glucose solution according to the body weight, and the doses were both 2 g/kg.
(4) At different time points after injection (15, 30, 60, 90, 120, 240 min), blood was collected from the tail to measure the blood glucose concentration of mice.

### 11. Insulin rescue in vivo

Insulin rescue experiment in vivo was subcutaneous injection of long-acting insulin glargine (Insulin Glargine) or corresponding volume of normal saline (control group) to STZ-induced diabetic mice for 1 week. Specific steps are as follows:
(1) Take out the insulin injection pen and leave it at room temperature for 3 hours to restore the biological activity of insulin. Connect the special needle of the injection pen, and carefully squeeze out 10 µl of insulin liquid (minimum unit dose volume). Carefully aspirate with a pipette and dispense into EP tubes, 2 µl/tube. Before use, add 998 µl of sterile saline to each EP tube to dilute the insulin concentration to 0.0002 U/µl (the original solution concentration is 0.1 U/µl). It should be used in time after preparation.
(2) Mice established in STZ model for 1 week were divided into groups according to the principle of randomization, and their body weight and random blood glucose were measured.
(3) According to the body weight, the insulin rescue group was subcutaneously injected with diluted insulin (1 U/kg/day), once a day at the same time for 7 consecutive days, and the control group was given a corresponding volume of normal saline.
(4) Body weight and random blood glucose were measured at different time points of insulin intervention, and the mice were anesthetized and the materials were collected.

### 12. MDC in vivo rescue

The in vivo rescue experiment of MDC in STZ diabetic mice is to inject MDC (sterilized PBS diluted, 10 mg/kg/day) intraperitoneally into STZ-induced diabetic mice for 3 days, once a day at the same time, and inject the corresponding volume of DMSO (Diluted in sterile PBS) of diabetic mice served as a control group. Body weight and random blood glucose were measured at different time points after injection. After MDC was continuously intervened for 4 days, the mice were anesthetized and the samples were collected.

The in vivo rescue experiment of MDC in Leptin^{-/-} mice was performed by pre-intraperitoneal injection of MDC in 6-week-old Leptin^{-/-} mice (diluted in sterile PBS, 10 mg/kg) or the corresponding volume of DMSO (diluted in sterile PBS). The injected dose of MDC in Leptin^{-/-} mice should be calculated based on the body weight of 6-week-old littermates of WT mice. After 1 h of MDC intervention, 50% D-glucose or the corresponding volume of PBS was injected intraperitoneally. The procedure of injecting D-glucose into Leptin^{-/-} mice was as follows: 12 injections were performed every 2 hours during the 24-hour period, and the injection dose of D-glucose was fixed at the first dose (2 g/kg). After the injection, the mice were anesthetized and the samples were taken.

In the experiment of local injection of Dkkl purified protein into the heart combined with MDC intervention, 10-week-old wild-type male mice were selected, the left chest was depilated, and intraperitoneal injection of MDC (diluted in sterile PBS, 10 mg/kg) or the corresponding volume of DMSO (diluted in sterile PBS) and injected at the same time every day thereafter. After 1 h of MDC intervention, cardiac Dkkl protein injection was performed. The mice were given continuous inhalation anesthesia with 1% isoflurane and oxygenated with a ventilator, and then the fourth intercostal space near the sternum was opened to expose the heart. Using a 200 µl microsyringe, Dkkl (10 µg of Dkkl per heart, dissolved in 30 µl PBS) or an equal volume of PBS was injected locally at three sites in the heart. Of these, two sites are on the border around the left ventricle, and one site enters the myocardium of the left ventricle. After the injection, the wound was sutured, the anesthesia was removed, and the mice were awake. After MDC was continuously intervened for 2 days, the mice were anesthetized and the materials were collected.

In addition to injecting Dkkl protein alone into the heart, we also need to perform experiments of local injection of Dkkl combined with BIM-46187 into mouse hearts. According to the experimental grouping, it was divided into cardiac injections of four different solutions, including PBS, Dkkl, BIM-46187, and a mixture of Dkkl and BIM-46187. The dosage of Dkkl per heart is 10 µg, and the dosage of BIM-46187 is 2.4 µg, diluted with 30 µl PBS and then injected into the heart. The specific operation steps are the same as above. After the injection, the wound was sutured, the anesthesia was removed, and the mice were awake. Two days after the injection, the mice were anesthetized and the materials were collected.

### 13. Tail vein injection of LRP6N plasmid

The LRP6N plasmid (with myc tag) used in this study needs to be identified in advance, that is, AD293 cells were transfected in vitro, and then the supernatant of the cells was collected. After using Western Blotting to prove that the LRP6N plasmid can express the secreted LRP6N protein, the following operations of tail vein injection of the plasmid are carried out. Reagent preparation: ①Hepes solution (50 mM, pH 7.4, 0.22 µ m filtered); ②D-glucose solution (50%, 0.22 µ m filtered); ③L-PEI solution (1 mg/ml, pH 5.0, 0.22 µ m filtered). Specific method: First dissolve 50 µ g of LRP6N or Vector plasmid in 100 µ 1 of Hepes solution containing 5% D-glucose, and let stand for 10 min at room temperature. Then slowly add 65 µl L-PEI to the above solution and mix quickly. After incubation at room temperature for 10 min, the mixture was injected into mice via the tail vein. In the early stage, we found that after 1 day of injection of luciferase plasmid into the tail vein of mice, the luciferin substrate (luciferin) was administered through the tail vein, and the luciferase activity of the whole body was observed by a small animal in vivo imager, which is expressed in the lung, liver and spleen, with the highest expression level in the lung. Therefore, we selected lungs to observe the expression of LRP6N plasmid at different time after tail vein injection. Western Blotting demonstrated that the secreted LRP6N protein expressed by the LRP6N plasmid could be continuously expressed in vivo for at least 6 days. The tail vein injection of LRP6N plasmid should be performed 1 day after STZ induction, and 7 days after STZ modeling, the body weight and random blood sugar were measured, the mice were anesthetized, and the samples were taken.

### 14. Mouse material

(1) The mice were anesthetized by intraperitoneal injection of 2% pentobarbital sodium (45 mg/kg).
(2) Fix the limbs on the operating table with the abdomen facing upward, cut the upper abdomen laterally under the xiphoid process to expose the liver, lift the xiphoid process with a hemostat, cut the diaphragm into the thoracic cavity, cut the ribs along both sides of the xiphoid process, and open it cephalad to expose heart.
(3) Use a 1 ml syringe to quickly puncture the right atrium to draw an appropriate amount of blood and put it into an EDTA anticoagulation tube.
(4) Find the apex of the heart and lift it with forceps, insert the perfusion needle into the apex of the heart, fix the needle tip with a hemostat, cut the right atrium, and start perfusion.
(5) Physiological saline perfusion: quickly put the catheter of the perfusion pump into the physiological saline, and start perfusion until the liquid flowing out from the right atrium is colorless, and the liver of the mouse is pale, the limbs are white, and the intestinal tract is swollen.
(6) Paraformaldehyde perfusion: Then carefully move the catheter of the perfusion pump to 4% paraformaldehyde to start perfusion. If you see that the limbs of the mouse are suddenly tense, the tail is curled, and the tissues and organs are gradually hardened, the immobilization effect is achieved.
(7) The materials were collected and the organs were weighed. Further processing: For tissue samples extracted from protein or RNA, after perfusion with normal saline, quickly freeze in liquid nitrogen and store at -80°C; for tissue samples for frozen section preparation, after perfusion with normal saline, aspirate the liquid adhering to the sample, directly embedded in OCT, then quickly frozen in liquid nitrogen, and stored at -80 °C; for tissue samples for paraffin sectioning, after perfusion with 4% paraformaldehyde, remove the specimen and put it in 4% paraformaldehyde , and continued to fix for 24 h on a shaker at 4°C for subsequent paraffin embedding and section staining.

### 15. Protein extraction from cells and tissues

### Total protein extraction from cells and tissues

(1) Prepare the lysate for total protein extraction according to the following table, mix well and put it on ice:

| component | | Dosage (1000 µl system) |
|---|---|---|
| | RIPA(2x) | 500 µl |
| | Protease inhibitor (100x) | 10 µl |
| | NaF(0.5 M) | 100 µl |
| | β-Glycerophosphate(0.5 M) | 40 µl |
| | Na₃VO₄(0.2 M) | 5 µl |
| | ddH₂O | 345 µl |

(2) The confluence of adherent cells in 3.5 cm dishes reaches 90% and can be used for total protein extraction. First remove the culture medium with a pipette, then wash twice with PBS, blot dry the PBS residue, and place it on ice.
(3) Add 150 µl of total protein lysate to each cell sample to make the lysate fully contact with the cells, scrape off the cell lysate with a scraper, collect it into a pre-cooled EP tube, and take an ice bath for 15 min.
(4) Add 500 µl of pre-cooled sterilized PBS (containing protease and phosphorylase inhibitors at a final concentration of 1 mM) per 100 mg of tissue, and chop the tissue pieces into pieces as much as possible in PBS. 4°C, 12000 rpm, 5 min, discard the PBS supernatant and keep the pellet. Add 150 µl of total protein lysate to each sample, and use a homogenizer to fully homogenize the tissue pellet on ice for 15 min.
(5) 4°C, 14000 g, 10 min, carefully collect the total protein supernatant to a new EP tube, and store at -80°C for later use.

### Membrane protein extraction from cells and tissues

(1) PMSF (final concentration of 1 mM) was added to the membrane protein extraction solution A and B, respectively.
(2) The confluence of adherent cells in 3.5 cm dishes reaches 90% and can be used for membrane protein extraction. First remove the culture medium with a pipette, then wash twice with PBS, blot dry the PBS residue, and place it on ice.
(3) Add 500 µl of pre-cooled sterilized PBS (containing PMSF with a final concentration of 1 mM) to each sample, scrape the cells with a scraper and collect them into pre-cooled EP tubes. Tissue pieces were minced as much as possible in PBS.
(4) 4°C, 12000 rpm, 5 min, discard the PBS supernatant and keep the pellet.
(5) Add 100 µl (cell) or 200 µl (tissue) A solution to each sample, and resuspend the cell pellet with a pipette to completely disperse the pellet. The tissue pellet should be fully homogenized on ice with a homogenizer, and the ice bath should be used for 15 min.
(6) Quickly freeze the sample with liquid nitrogen, then put it in a 37°C water bath, take it out when the sample is thawed to almost no ice crystals, repeat freezing and thawing 3 to 5 times, do not make the sample thaw too much, and put it back on ice after the end.
(7) Vortex at the highest speed for 5 s, 4°C, 700 g, 10 min. Carefully collect the supernatant into a new EP tube without touching the pellet.
(8) Continue centrifugation of the supernatant from step "7" at 14000 g for 30 min at 4°C. Carefully collect the supernatant into a new EP tube without touching the precipitate. Extract the plasma protein and store it at -80°C for later use.
(9) 4°C, 14000 g, 1 min, suck up the supernatant as much as possible to ensure that the membrane protein is not mixed into the plasma protein component.
(10) Add 50 µl (cells) or 100 µl (tissue) B solution to each sample, resuspend the pellet in Vortex at the highest speed for 10 s, ice bath for 10 min, and repeat twice to fully extract membrane proteins.
(11) 4 °C, 14000 g, 5 min, carefully collect the supernatant into a new EP tube, extract the membrane protein, and store at -80 °C for later use.

### Nuclear protein extraction from cells and tissues

(1) PMSF (final concentration of 1 mM) was added to the plasma protein extraction solution A and the nuclear protein extraction solution, respectively.
(2) The confluence of adherent cells in 3.5 cm dishes reaches 90% and can be used for nuclear protein extraction. First remove the culture medium with a pipette, then wash twice with PBS, blot dry the PBS residue, and place it on ice.
(3) Add 500 µl of pre-cooled sterilized PBS (containing PMSF with a final concentration of 1 mM) to each sample, scrape the cells with a scraper and collect them into pre-cooled EP tubes. Tissue pieces were minced as much as possible in PBS.
(4) 4°C, 12000 rpm, 5 min, discard the PBS supernatant and keep the pellet.
(5) Each cell sample: add 200 µl of solution A. For each tissue sample: solution A and solution B were mixed at a volume ratio of 20:1 (containing PMSF with a final concentration of 1 mM) to prepare 200 µl of tissue homogenate and added to the tissue.
(6) Vortex at the highest speed for 5 s to completely disperse the precipitate. The tissue pellet should be fully homogenized on ice with a homogenizer, and the ice bath should be used for 15 min.
(7) Tissue: 4°C, 1500 g, 5 min. Aspirate the supernatant into a pre-cooled EP tube, and extract part of the plasma protein. Tissue Precipitation then, from step "5", plasma and nucleoprotein extraction was carried out according to the cell treatment method.
(8) Add 10 µl of solution B to each sample. Maximum speed Vortex 5 s, ice bath for 1 min.
(9) Maximum speed Vortex 5 s, 4°C, 14000 g, 5 min.
(10) Aspirate the supernatant into a pre-cooled EP tube, do not touch the precipitate, extract the plasma protein, and store it at -80°C for later use. The plasma protein obtained by tissue extraction can be combined with the plasma protein in step "7".
(11) Aspirate the residual supernatant completely, and add 50 µl of nuclear protein extract to each sample.
(12) Vortex at the highest speed for 30 s to completely disperse the precipitation, put it back on ice, and then Vortex at high speed for 30 s every 2 min for a total of 30 min.
(13) 4°C, 14000 g, 10 min.
(14) Aspirate the supernatant into a pre-cooled EP tube, extract the nucleoprotein, and store it at -80°C for later use.

### Determination and calibration of protein concentration

### A. The determination of protein concentration adopts BCA method, and the specific steps are as follows:

(1) Dissolve and mix the protein sample to be tested on ice. First add 18 µl ddH₂O to each well of the 96-well plate, then add 2 µl of the sample, and only add 20 µl ddH₂O to the blank well. Two replicate wells were made for each sample.
(2) Protein concentration determination solution A and B solution were prepared according to the volume ratio of 50:1, 200 µl/well, the 96-well plate was gently shaken and mixed, and then placed in a 37°C incubator for 30 min.
(3) Take out the 96-well plate, zero-adjust the blank well, and read the OD value of each well at a wavelength of 562 nm.

### B. The calibration of protein concentration adopts relative quantitative method, and the specific steps are as follows:

(1) The sample volume with the smallest OD value (a µl) is used as the calibration volume, and the balance volume of other samples is [minimum OD value×a µl]/Other sample OD value, and then use ddH₂O to make up the other sample volumes to the calibration volume (a µl), thereby adjusting the OD value of each sample to the level of the sample with the smallest OD value.
(2) Add 0.25 times the volume of protein solution 5× loading buffer to each sample, mix well, cook in a metal bath at 100°C for 5 min, centrifuge briefly, and store at -80°C for later use.

### 16. Western blotting

### A. Preparation of SDS-PAGE gel:

Protein electrophoresis gels are double-layered gels, including an upper stacking gel and a lower separating gel. The concentration of the stacking gel is 5%, and the concentration of the separating gel is determined according to the molecular weight of the target protein. In this study, large molecular weight proteins (LRP5/6, β-catenin, β -arrestin1/2) used 8%-5% double-layer gel, and small molecular weight proteins (yH2AX, p53, p21, Bcl-2, Bax, Cleaved Caspase- 3) used 12%-5% double-layer gel. In addition, the number of wells (10 wells or 15 wells) and thickness (1 mm or 1.5 mm) of the preparation gel were selected according to the number and volume of protein samples loaded. The gel formulation is shown in the experimental material section, and the preparation steps are as follows:
(1) Rinse the glass plate with tap water. After drying in the oven, put it into the glue rack to prevent liquid leakage at the bottom.
(2) First prepare a separating gel, add ddH2O, 30% Acrylamide, Tris-HCl, 10% SDS, 10% APS, TEMED in sequence, and inject the gel after mixing. The amount of each gel is 7.5 ml to avoid bubbles. Add n-butanol to level the liquid surface and let it solidify for 30 min.
(3) Reconstitute the concentrated gel (the method is the same as that of the separating gel), and the amount of each gel is 2 ml to avoid the generation of air bubbles. Quickly insert a clean pore-forming comb and let it solidify for 30 min.

### B, protein electrophoresis:

Different protein molecules move at different speeds in the electrophoresis gel due to the difference in protein molecular weight and charge, so that various protein molecules can be distinguished in the gel. Specific steps are as follows:
(1) Clamp the prepared gel into the clip, put it into the electrophoresis tank, pour 1× electrophoresis buffer, completely immerse the sample loading hole of the gel, and gently remove the hole-forming comb to avoid deformation of the gel hole.
(2) Dissolve the mixed protein sample on ice, cook in a metal bath at 100°C for 5 min, centrifuge briefly and put it back on ice to prepare for loading.
(3) Sample loading: Arrange all protein samples in the recorded loading order, and pipette the same amount of samples into the gel hole. When loading the sample, the pipette tip is attached to the higher side glass, and the position along the channel is as low as possible. When resistance is encountered, it can be added dropwise. Since the sample contains loading buffer, the sample solution can sink to the bottom of the hole by itself; Each time a sample is completed, in order to avoid cross-contamination, a new pipette tip should be replaced for the next sample loading; the sample volume should be moderate, with a maximum of 40 µl per well for 10-well gel and a maximum of 20 µl per well for 15-well gel. After loading, an appropriate amount of pre-stained protein markers (8 µl on the left, 4 µl on the right) were added to the wells on both sides of the sample, and the left and right order of the loading was easily distinguished by the depth of the Marker color.
(4) Electrophoresis: Cover the lid of the electrophoresis tank, turn on the power, start electrophoresis at room temperature, set the electrophoresis conditions: first 80 V, 30 min, after the sample runs off the stacking gel and is pressed into a horizontal straight line (judged by bromophenol blue), and then 120 V for 90 min to separate the dyes of different molecular weights in the Marker in the gel. According to the molecular weight of the Marker, judge whether the protein molecules of different sizes are sufficiently separated in the gel, terminate the electrophoresis in time, and ready to transfer.

### C, gel electroporation:

0.45 µm PVDF membrane was used for the transmembrane of the target protein larger than 20 kDa, and 0.2 µm PVDF membrane was used for the protein less than 20 kDa. PVDF membranes need to be treated with methanol to activate the positively charged groups on the membrane, making it easier to bind negatively charged proteins. Specific steps are as follows:
(1) Pour the pre-cooled 1× electroporation buffer into the electroporation tank, and soak the membrane transfer splint and its contents in 1 × electroporation buffer for later use.
(2) Prepare the PVDF membrane according to the gel size (5.5×8.5 cm²), cut the corners and make a mark to distinguish the front and back of the membrane. After activation in methanol for 1 min, put it in 1 × electroporation buffer for use.
(3) After electrophoresis, rinse the gel plate with water, pry it open with a scraper, remove the gel, open the membrane transfer splint, and follow the order of sponge pad →filter paper→gel→PVDF membrane→filter paper→sponge pad , put the contents in the middle of the black surface of the transfer film splint, and gently flatten it to close the white surface of the transfer film splint. Insert the electroporation tank (black side faces black side, white side faces red side) and fill up with electroporation buffer. During the transfer process, air bubbles should be avoided between the PVDF membrane and the gel, and a scraper can be used to gently remove the tiny air bubbles in the gap.
(4) electroporation: put the electrotransfer tank into ice cubes, cover the lid of the electrotransfer tank, turn on the power to start the electroporation, and set the electroporation condition: 100 V, 120 min. During electroporation, if the current is too large or the electroporation buffer is overheated, a new pre-cooled electroporation buffer should be replaced in time to prevent membrane transfer failure due to overheating.

### D. blocking:

The blocking solution (5% skim milk) can bind to the non-specific binding sites on the PVDF membrane, reducing the background of the PVDF membrane and ensuring the specific binding of the primary antibody to the target protein. Specific steps are as follows:
(1) After the electroporation, open the membrane transfer splint, take out the PVDF membrane with tweezers, quickly put it into the blocking solution, and slowly shake it on a shaker for 1 h at room temperature.
(2) Wash the membrane. Pour off the blocking solution, put the PVDF membrane on a shaker, wash three times with TBST for 5 min each time, and prepare for primary antibody incubation after washing.

### E. Antibody (primary and secondary) incubation:

By incubating the primary antibody, the protein or polypeptide on the PVDF membrane reacts specifically with the corresponding primary antibody, and then by incubating the secondary antibody (horseradish peroxidase (HRP) labeling), the primary antibody is combined with the secondary antibody, Through the cascade amplification reaction between the exposure liquid substrate and the HRP linked to the secondary antibody, the detectable chemiluminescent protein band is displayed, so that the expression level of the specific target protein can be judged. Specific steps are as follows:
(1) Dilution of the primary antibody: Before using the primary antibody, centrifuge briefly, and use the primary antibody diluent to dilute the primary antibody according to the multiple recommended by the antibody specification.
(2) Incubation of primary antibody: According to the size of the marker, cut out the corresponding part of the PVDF membrane containing the distribution of the target protein to be incubated. Different target proteins should be incubated with their corresponding specific primary antibodies. Incubation time depends on the affinity of the antibody to the protein and the abundance of the protein. Put the diluted primary antibody and PVDF membrane (with the protein distribution side up) in the incubation box together to cover the membrane evenly, so that the primary antibody and the membrane can be evenly combined. Incubate overnight at 4°C with gentle shaking of the shaker. For protein samples with low affinity or low concentration, it is recommended to use a higher antibody dilution concentration and a longer incubation time (generally no more than 18 h) to ensure its specific binding.
(3) Wash the membrane: Recover the incubated primary antibody to a centrifuge tube (usually reused three times), and store at 4°C. The PVDF membrane was placed on a shaker and washed three times with TBST for 5 min each time. After washing, the secondary antibody was prepared for incubation.
(4) Dilution of the secondary antibody: Before using the secondary antibody, centrifuge briefly, and dilute the secondary antigen solution with TBST according to the multiple recommended in the antibody specification.
(5) Incubation of the secondary antibody: Put the diluted secondary antibody and PVDF membrane (the side with the protein distribution up) together in the incubation box, and incubate for 1-2 h at room temperature by shaking the shaker slowly.
(6) Wash the membrane. The secondary antibody was discarded (not recovered), and the PVDF membrane was placed on a shaker and washed three times with TBST for 5 min each time. After washing, it was ready for exposure and development.

### F. Exposure and development:

The protein bands were developed by chemiluminescence. The HRP linked to the secondary antibody is a highly sensitive enzyme, which can catalyze the ECL exposure liquid substrate to produce a luminescent substance. The exposure function can convert the luminescent signal into a grayscale signal of the band on the picture, and the grayscale of the band can reflect the expression level of the target protein. Specific steps are as follows:
(1) Preparation of exposure solution: Mix equal volumes of ECL exposure solution A and B, and protect from light with tin foil paper.
(2) Blot dry the TBST paper on the PVDF membrane, lay it flat on a layer of transparent film (the side with the protein distribution is facing up), apply the exposure solution evenly on the membrane, react for about 1 min, and aspirate the exposure fluid, put the film directly under the lens of the exposure machine, adjust the field of view, and use Quantity One 4.4 software to expose the PVDF film. The exposure conditions of the target protein will vary depending on the antibody titer, so that you need to explore the best parameters by yourself. Finally, the exposure data was exported to TIFF image format and saved. If necessary, Image-Pro Plus 6.0 software could be used for quantitative analysis of gray value of protein bands.

### 17. Total RNA extraction and real-time quantitative PCR

### A. Total RNA extraction:

It is best to perform total RNA preparation immediately after sample collection. If it cannot be extracted in time, it should be frozen in liquid nitrogen and stored at -80°C. During tissue RNA extraction, samples should be quickly broken by grinding with liquid nitrogen, and do not thaw first to avoid RNA degradation caused by endogenous RNases. In addition, in order to prevent RNA from being degraded by exogenous RNase during the extraction process, pay attention to the use of RNase-free pipette tips and EP tubes; grinding tools need to be sterilized at high temperature and high pressure; wear disposable masks and gloves; avoid talking while operating etc.
(1) Add 1 ml of Trizol per 50-100 mg of tissue to lyse the samples ground in liquid nitrogen, and repeatedly pipet with a pipette to lyse the tissue samples.
(2) Transfer the Trizol lysate to an EP tube and place at room temperature for 5 min.
(3) Add 0.2 ml of trichloromethane per 1 ml of Trizol, cover the cap of the EP tube, shake vigorously in your hand for 15 s, place at room temperature for 3 min, 12000 g, 4 °C, 15 min.
(4) Take the upper aqueous phase and place it in a new EP tube, add 0.5 ml of isopropanol to each 1 ml of Trizol, invert up and down and mix, place at room temperature for 10 min, 12000 g, 4 °C, 10 min, discard the supernatant and keep precipitate.
(5) Add 1 ml of 75% ethanol prepared with DEPC water per 1 ml of Trizol for washing, vortex to mix, 7500 g, 4 °C, 5 min, discard the supernatant, and keep the precipitation. Do the same operation and wash again, a total of two times.
(6) Let the precipitated RNA dry naturally at room temperature.
(7) Add 50 µl DEPC water and place on ice for 5-10 min to dissolve the precipitate and extract to obtain RNA.
(8) Use NanoDrop to measure the concentration (µg/ml) and purity (OD260/OD280) of RNA samples, and the purity is usually 1.8-2.0.

### B. Real-time quantitative PCR:

(1) Removal of genomic DNA (gDNA) in RNA samples. Prepare the reaction solution on ice according to the following table, prepare the Master Mix according to the number of reactions + 2, and then dispense it into each reaction tube, and finally add the RNA sample. The reaction solution was reacted at 42°C for 2 min, and after that, it was stored on ice, and the obtained RNA without gDNA was used for reverse transcription.

| component | Dosage (10 µl system) | |
|---|---|---|
| gDNA Eraser Buffer(5 ×) | 2 µl | Master |
| gDNA Eraser | 1 µl | Mix(3 µl) |
| RNA sample | 1 µg | |
| RNase Free ddH₂O | Make up to 10 µl | |

(2) Reverse transcription reaction. Prepare the reaction solution on ice according to the table below, prepare the Master Mix according to the number of reactions + 2, and then dispense into each reaction tube and mix gently. The reaction solution was reacted at 37 °C for 15 min; 85 °C for 5 s. After that, it was stored on ice, and the obtained cDNA template was used for SYBR Green qPCR reaction.

| component | Dosage (20 µl system) | |
|---|---|---|
| RNA without the gDNA | 10 µl | |
| RNase Free ddH₂O | 4 µl | Master Mix(10 µl) |
| PrimeScript Buffer 2(5×) | 4 µl | |
| RT Primer Mix | 1 µl | |
| PrimeScript RT Enzyme Mix I | 1 µl | |

(3) Preparation of PCR reaction system. Prepare the reaction solution on ice as shown in the table below. It is recommended that the amount of RNA sample equivalent to the amount of cDNA template in 20 µl of the reaction solution should not exceed 100 ng.

| component | Dosage (20 µl system) |
|---|---|
| SYBR^{®} Premix Ex Taq II(2×) | 10 µl |
| PCR Forward Primer(10 µM) | 0.8 µl |
| PCR Reverse Primer(10 µM) | 0.8 µl |
| ROX Reference Dye II(50×) | 0.4 µl |
| cDNA template | 2 µl |
| Sterilized ddH₂O | 6 µl |

(4) On-board testing and result analysis

The sample for detecting a certain gene needs to do 3 replicate wells to exclude the sampling error. The loaded 96/384-well plate was sealed with a clear film and centrifuged briefly to remove the residue from the lower tube wall. Put it into the PCR machine and set the reaction conditions: Step 1: pre-denaturation (95°C for 30 s); Step 2: PCR reaction (95°C for 5 s, 60°C for 34 s) 40× cycle. At 60°C, a fluorescence detection point was set and amplification was performed. If necessary, a melting curve can be added after the reaction to detect the specificity of the amplified product. The final CT value was obtained, and the expression of a certain gene in different samples was calculated by the 2^{-ΔΔCT} method.

### 18. Tissue frozen sections and immunofluorescence staining

(1) Take out the OCT embedding block from the -80°C refrigerator, flatten the bottom of the specimen with a safety blade, and quickly adhere it to the circular support, and place it on the freezing stage of the freezing microtome at -24°C. After the embedded block is completely adhered to the support, it is fixed on the specimen table, and the unnecessary OCT is quickly cut off until the tissue block to be cut is exposed.
(2) Adjust the thickness of the slices to 6 µm, place them at room temperature for 30 min after cutting to prevent the slices from falling off, and store at -20°C for later use.
(3) Take out the frozen sections from the -20°C refrigerator, put them at room temperature for 30 minutes, fix them with pre-cooled 4% paraformaldehyde at room temperature for 20 minutes, wash with PBS for 3 × 10 minutes, and dry the liquid around the samples with paper.
(4) 0.2% Triton X-100 (diluted in PBS) to permeate the cell membrane for 10 min, and wash with PBS for 3×10 min.
(5) circled using immunohistochemical pen, blocked with 5% BSA for 1 h at room temperature.
(6) Add the primary antibody (diluted with 1% BSA) dropwise, cover it evenly with parafilm, put it in a humid box to prevent the surface from drying, and incubate at 4°C overnight.
(7) Wash with PBST for 3×10 min, and dry the liquid around the sample with paper.
(8) Add Cy3 fluorescent secondary antibody (diluted with 1% BSA) dropwise, cover it evenly with parafilm, and incubate at room temperature for 1 h in the dark.
(9) Wash in PBST for 3 × 10 min, protect from light, and dry the liquid around the sample with paper.
(10) DAPI staining (2 µg/ml, diluted in PBS) for 10 min, protect from light.
(11) Wash with PBST for 3×10 min, protect from light, and dry the liquid around the sample with paper.
(12) Add anti-fluorescence quencher dropwise, and mount the slide.
(13) Laser scanning confocal microscope imaging (63× oil immersion lens), Cy3-labeled red fluorescence (target protein) and DAPI-labeled blue fluorescence (nucleus) can be seen.

### 19. Tissue paraffin section and HE staining

### A. Preparation of tissue paraffin sections:

(1) Take out the tissue specimens fixed with 4% paraformaldehyde for 24 hours, connect the label and the tissue with a pin, rinse with running water overnight to remove the stationary liquid, and dehydration was carried out with gradient ethanol prepared with ddH2O.
(2) Dehydration: 70% ethanol for 1 h → 80% ethanol for 1 h → 90% ethanol for 1 h → 95% ethanol for 1 h (×2) → 100% ethanol for 1 h (×2).
(3) Xylene was transparent for 30 min (×2).
(4) waxdip: soak the melted paraffin at 65°C for 3 to 4 hours.
(5) Embedding: Put the tissue in a self-made embedding box, add melted paraffin, adjust the position of the tissue with a pin, and carefully place the embedding box on a -20°C cooling table. After most of the paraffin solidifies, stick the label on the paraffin block, mark the tissue specimen, and pry the wax after 10 min.
(6) Sectioning: Trim the paraffin block with a safety blade, fix it on a microtome, adjust the thickness of the section to 6 µm, flatten the tissue piece with a brush, transfer it to a 40°C water bath, and then remove the tissue pieces from the water bath with anti-dropping slides.
(7) Put the slides on a 37°C oven for overnight and store at room temperature.

### B. HE staining:

(1) Bake the above slides at 65° C for 5 min, and start dewaxing: xylene for 10 min → xylene for 5 min.
(2) Hydration: 100% ethanol for 5 min (×2)→95% ethanol for 5 min (×2)→85% ethanol for 3 min→75% ethanol for 2 min→ddH2O for 1 min.
(3) Hematoxylin staining for 5 min. Wash with water for 15 min.
(4) Differentiation in 1% hydrochloric acid alcohol for 30 s. Wash with water for 15 min.
(5) ddH₂O 2 min→75% ethanol for 2 min→85% ethanol for 2 min.
(6) Eosin staining for 15 s.
(7) Dehydration: 95% ethanol for 5 min (×2)→100% ethanol for 5 min (×2).
(8) Xylene was transparent for 5 min (×2).
(9) Covering: drop a drop of neutral gum in the middle of the slide glass, place the cover glass on the neutral gum, and press slowly so that the neutral gum fills the entire space covered by the cover glass to avoid air bubbles. After 24 h, the neutral gum on the surface was wiped off with xylene. Place in a fume cupboard for 24 h to fully evaporate the xylene.
(10) Use low magnification (12.5×) and high magnification (400×) to observe and take pictures under an optical microscope, respectively.

### 20. Determination of plasma Dkkl (ELISA method)

(1) Sample preparation: Store the freshly obtained mouse blood in an EDTA anticoagulation tube, stand at room temperature for 10 min, centrifuge at 1000 g for 15 min, and collect the supernatant, namely plasma. If it cannot be detected immediately, it should be subpackaged and stored at -80°C to avoid repeated freezing. If precipitation occurs during storage, it should be fully thawed at room temperature before use, and centrifuged again.
(2) All reagents were returned to room temperature for 30 min and mixed well. Streptavidin-HRP (1:200), biotinylated goat anti-mouse Dkkl detection antibody (1:180), recombinant mouse Dkkl standard (1:100), plasma samples (1:4) were diluted to the indicated concentration first using reagent diluent; goat anti-mouse Dkkl coating antibody (1:180) was first diluted to the indicated concentration with coating buffer. The diluted reagents were used in the following steps.
(3) Standard: Use reagent diluent to dilute the standard to 7 concentration gradients including 1350, 675, 337.5, 168.8, 84.4, 42.2, 21.1 pg/ml.
(4) Coating of the sample plate: Immediately add 100 µl of Dkkl-coated antibody per well to the 96-well sample plate for antibody coating, seal it with a sealing film, and incubate at room temperature overnight.
(5) Wash the plate: Carefully peel off the sealing film, discard the liquid and spin dry, fill each well with the diluted washing solution, let it stand for 30 s and discard, pat dry with paper thoroughly, and thoroughly wash the sample plate 3 times .
(6) Blocking: Add 300 µl of reagent diluent to each well and incubate for 1 h at room temperature.
(7) Repeat step "4".
(8) Sample loading: Add 100 µl ddH₂O (blank well), Dkkl standard or plasma sample to each well, cover the sample plate, and incubate at room temperature for 2 h.
(9) Repeat step "4".
(10) Detection: Add 100 µl Dkk1 detection antibody to each well, cover the sample plate, and incubate at room temperature for 2 h.
(11) Repeat step "4".
(12) Add 100 µl streptavidin-HRP to each well, cover the sample plate, and incubate at room temperature for 20 min in the dark.
(13) Repeat step "4".
(14) Add 100 µl of developing-coloring solution to each well (mix substrate A and substrate B at a ratio of 1:1, use within 15 minutes), cover the sample plate, and incubate at room temperature for 20 minutes in the dark.
(15) Add 50 µl of stop solution to each well, and gently shake the sample plate to mix the liquid.
(16) Plate reading: zeroing with blank holes, and read the OD value of each well at a wavelength of 450 nm.
(17) Statistics: According to the concentration of the standard substance (abscissa) and its OD average value (ordinate), a standard curve and a linear regression equation are generated, and the Dkkl concentration (pg/ml) in the sample loading microwell is calculated by the formula, then multiplied by the dilution factor to obtain the actual concentration (pg/ml) of Dkk1 in the plasma.

### 21. Determination of plasma 8-OHdG (ELISA method)

(1) The sample preparation is the same as the determination of plasma Dkkl (ELISA method).
(2) Grouping: The kit was placed at room temperature for 30 min. Remove the ELISA PLATE and determine the required number of plates based on the number of standards and plasma samples. Set up blank wells (Blank), non-specific binding detection wells (Non-Specific Binding, NSB), standard substance/plasma sample group. To reduce experimental error, two replicate wells were set.
(3) Standard: Use standard dilution to double dilute the standard to 8 concentration gradients including 20, 10, 5, 2.5, 1.25, 0.625, 0.313, 0.157 ng/ml.
(4) Add sample: Add the required reagents to the microwells with different settings according to the following table:

| Microwell settings | Standard Diluent | Standards/plasma | AChE enzyme labeling solution | 8-OHdG primary antibody |
|---|---|---|---|---|
| Blank | - | - | - | - |
| NSB | 100 µl | - | 50 µl | - |
| Standards/plasma | - | 50 µl | 50 µl | 50 µl |

(5) Incubation: After sealing the ELISA plate with a sealing film, incubate at 4°C for 18 h.
(6) Wash the plate: Carefully peel off the sealing film, discard the liquid and spin dry, fill each well with the diluted washing solution, let it stand for 30 s and discard, thoroughly pat dry with paper and thoroughly wash the ELISA plate 5 times.
(7) Color development: 200 µl of color development solution (reaction substrate containing AChE) was added to each well. Reseal the ELISA plate with a sealing film, place it on a shaker, and react at room temperature for 90-120 min in the dark.
(8) Termination: After the color reaction is over, gently peel off the sealing film to prevent the liquid from splashing out of the well, and read the plate immediately.
(9) Plate reading: zeroing with blank holes, and read the OD value of each well at a wavelength of 412 nm.
(10) Statistics: The OD values of the standard and plasma samples must be corrected by subtracting the average OD value of the NSB wells. According to the concentration of the standard substance (abscissa) and its corrected OD average value (ordinate), a standard curve and a linear regression equation were generated, and the 8-OHdG concentration (ng/ml) in the loading microwell was calculated by the formula, and then multiply by the dilution factor to obtain the actual concentration (ng/ml) of 8-OHdG in plasma.

### 22. Statistical analysis

In this study, statistical analysis of data was done by GraphPad Prism 5 software, and all data were expressed as mean ± standard deviation. Unpaired two-tailed t-test (Student's t-test) between two groups of samples and One-way ANOVA between multiple groups of samples (one-way analysis of variance with Bonferroni's *post hoc* test, one-way ANOVA with Bonferroni's post-test comparison) were used to assess the statistical difference between the two groups of data. For all experiments, differences between groups were statistically significant when the P value was less than 0.05. The ^{∗} symbol shown in the figure means the significance of the P value: ^{∗}P < 0.05, ^{∗∗}P < 0.01, ^{∗∗∗}P < 0.001.

### Example 1 Dkkl upregulation may be associated with cachexia-related tumor death.

Through TCGA analysis, the expression of Dkkl is increased in clinical patients with various tumors, and high expression of Dkkl will significantly reduce the survival time of patients (Figure 1a). Upregulation of Dkkl in blood is also observed after subcutaneous implantation of CT26 and LLC tumor cells in mice by ELISA analysis (Fig. 1b). Real-time PCR analysis shows that Dkkl expression is highest in CT26 tumor cells compared to organs such as muscle, kidney, heart, and liver (Figure 1c), suggesting that implanted tumors may release Dkkl and increase local appreciation of circulating Dkkl. Even, implantation of CT26 tumors causes increased Dkkl expression in muscle and kidney (Fig. Id). Notably, kidneys express almost negligible Dkkl even after CT26 tumor implantation (Fig. 1c, d). All CT26 tumor-bearing mice died about 80 days after tumor implantation, and injection of Dkkl could significantly shorten the survival period of tumor-bearing mice (Dkkl was injected at 2-day intervals from day 40) (Fig. 1e), indicating that DKK1 can directly affect cancer death. However, neutralizing antibodies to Dkkl can extend the lifespan of CT26 tumor-bearing mice (Fig. If). Since CT26 tumor cells do not metastasize, cancer death in this model is not due to metastasis. Notably, CT26 tumor-bearing mice develops severe cachexia, accompanied by persistent body weight loss and decreased muscle mass (Fig. 1g). Thin muscle fibers and blank areas are shown by HE staining, suggesting muscle atrophy and some muscle hydrolysis (Fig. 1h). Interestingly, the kidney weight remains unchanged, and creatinine and urea nitrogen (Fig. 1i), two indicators of renal function, also do not change, did not change in HE staining and blood, indicating that seeding CT26 cells have no effect on the kidney. These results suggest that CT26 tumor seeding often affects organs other than the kidney. Although an increase in Dkkl is also observed in LLC cell tumor-bearing mice (Fig. 1b), LLC cells have a strong ability to migrate. Therefore, to elucidate the underlying mechanism of Dkk1's deleterious role in metastasis-independent tumor death, we focused on CT26 tumor-bearing mice in our next experiments.

### Example 2 Downregulation of membrane proteins LRP6 and Kremen2 can cause tumor cachexia

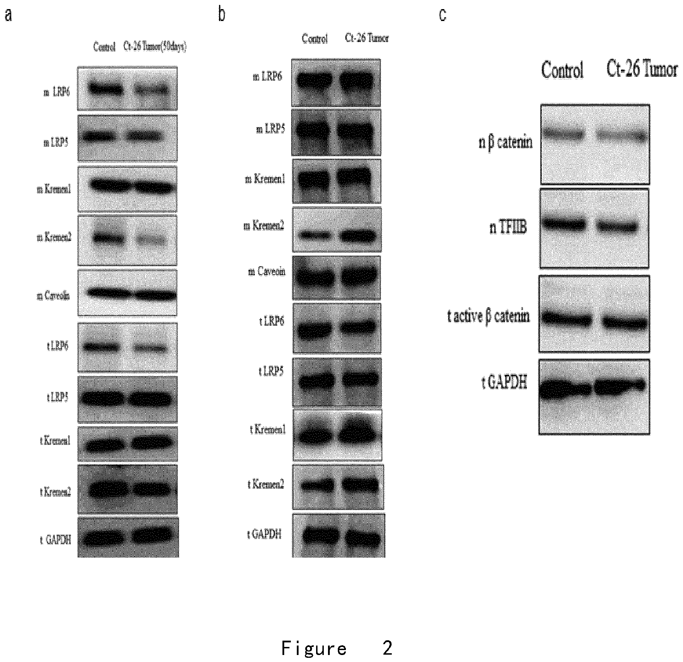

Muscle wasting, a key feature of tumor cachexia, is a multifactorial disease that negatively impacts patient outcomes and quality of life. Skeletal muscle wasting is considered a meaningful prognostic factor during cancer development, regardless of body mass index (BMI), and is associated with increased incidence of chemotherapy toxicity, shorter time to tumor progression, poorer surgical outcomes, physical impairment, and shorter survival. Furthermore, Kremen1/2 is necessary receptors for Dkkl-mediated LRP5/6 submembrane. Therefore, to explore the underlying mechanism of Dkk1's deleterious role in cancer death, we first examined the expression of Dkkl-binding proteins LRP5/6 and Kremen1/2 in CT26 tumor-bearing mice. On the 50th day after tumor implantation, Dkkl in systemic circulation is significantly increased (Figure 1b), and muscle atrophy also occurs (Figure 1g and Figure 1h). Membrane protein and total protein of LRP6 and Kremen2 are significantly down-regulated in muscle, while LRP5 and Kremen1 are not significantly down-regulated (Fig. 2a). In contrast, even at the final stage of cachexia, LRP6 expression in the kidney is unchanged, whereas Kremen2 expression is significantly elevated (Fig. 2b). Based on the fact that tumor implantation has no effect on the kidneys (Example 1i), suggesting that the regulation of LRP6 and Kremen2 on the membrane may be responsible for Dkkl-induced organ damage. Furthermore, the expression of activated β-catenin and nuclear β-catenin is not altered (Fig. 2c), suggesting that the mechanism of LRP6 alteration in the development of cachexia is independent of β-catenin.

### Example 3 Reversal of membrane downregulation of LRP6 and Kremen2 prevents tumor cachexia

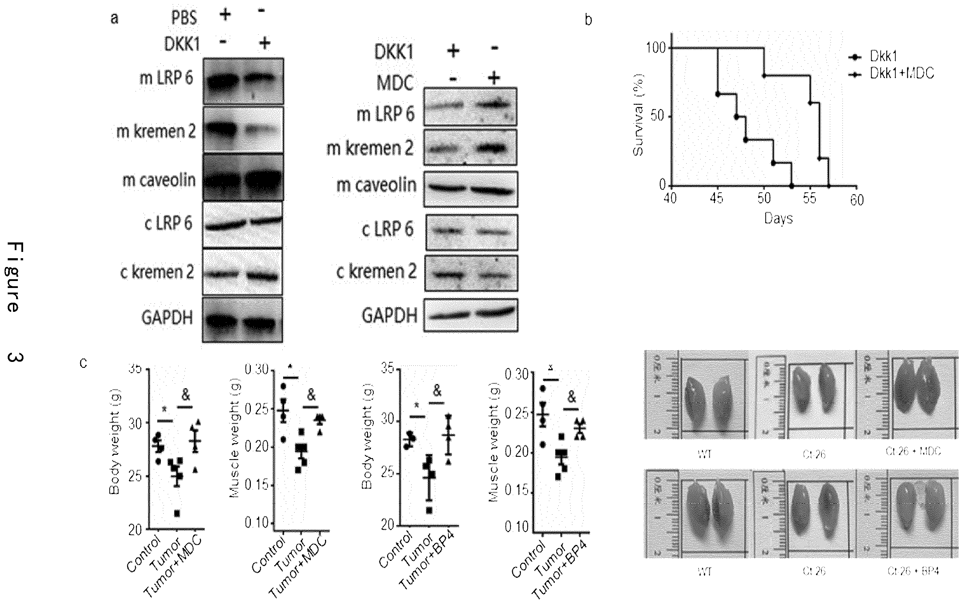

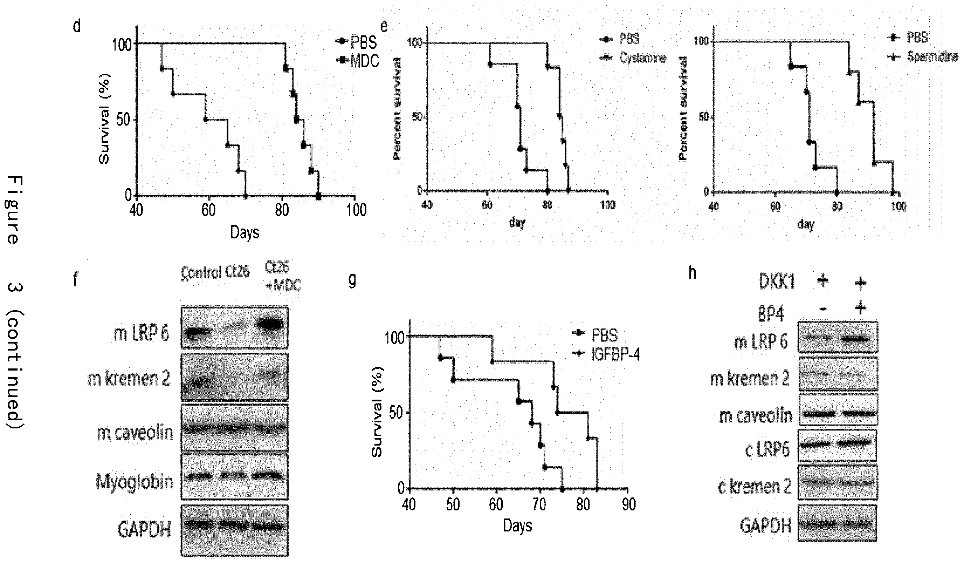

To verify that the down-regulation of membrane LRP6 is involved in the development of tumor cachexia, we used a small molecule chemical drug MDC to prevent Dkkl-induced down-regulation of membrane LRP5/6. Intramuscular injection of Dkkl into the lower limbs of normal mice causes the endocytosis of LRP6 and Kremen2 on the membrane (Fig. 3a), while simultaneous injection of MDC prevents this phenomenon (Fig. 3a). More importantly, MDC could inhibit the rapid death of tumor-bearing mice induced by Dkkl injection (Fig. 3b). Surprisingly, injection of MDC at the same time as tumor implantation reverses downregulation of LRP6 and Kremen2 on membrane and completely prevents the loss of body weight and muscle weight (Fig. 3c). MDC intervention prolongs the survival of CT26 mice even 40 days after tumor implantation (Fig. 3d). Further use of the clathrin-TG2-related endocytosis inhibitors Cystamine and Spermindine observes similar results with MDC in prolonging the survival of tumor-bearing mice (Fig. 3e). Combined with the results of muscle protein level detection (Figure 3f), it shows that preventing Dkkl-induced downregulation of LRP6 and Kremen2 on the membrane can prevent tumor cachexia and prolong survival. Interestingly, the recombinant mutant IGFBP4/H95P protein, which lost its IGF-binding ability, can bind to LRP6 on the membrane and prevent Dkkl signaling, and also prolongs the survival of tumor-bearing mice (Fig. 3g). As with MDC, simultaneous injection of IGFBP-4/H95P completely prevents the decrease in body weight and muscle weight (Fig. 3c), the mechanism is also to inhibit downregulation of membrane LRP6 expression induced by Dkkl (Fig. 3h)

### Example 4 Changes in GPCR expression profile after LRP5/6 deletion (transcriptome sequencing)

The Wnt co-receptors LRP5/6 are involved in the activation of the Wnt/β-catenin pathway. Since LRP5/6 activates β-catenin into the nucleus, in order to explore whether the close effect of LRP5/6 itself on GPCRs is widespread, it is necessary to exclude the interference of β-catenin signaling downstream of LRP5/6, that is, the corresponding β-catenin experiment should be used as the experimental control for LRP5/6. First, we used RNAi technology to carry out gene knockdown experiments of LRP5/6 and β-catenin in several cells (HepG2, Hela, U2OS, HUVEC), respectively. It is observed under the microscope that knockdown of LRP5/6 gene causes the deterioration of the above cell status and growth arrest. Especially in HepG2 cells, it is most obvious. Then, WB detection shows that the yH2AX signal is up-regulated after LRP5/6 knockdown, suggesting that cells with knockdown of LRP5/6 gene have different degrees of DNA damage, while the control group and knockdown of β-catenin gene do not have this phenomenon, indicating that LRP5/6 can specifically protect the balance of cell homeostasis independent of β-catenin. Considering the possibility that a large number of GPCRs can interact with LRP5/6 in a single cell, we have hypothesized that knocking down the LRP5/6 gene will simultaneously affect these GPCRs that interact with LRP5/6, which in turn the regulation of positive and negative feedback loops leads to changes in the expression of these GPCRs at the transcriptional level, ultimately altering GPCR signaling, disrupting cellular homeostasis, and causing changes such as cell growth arrest and DNA damage. In order to test this hypothesis, considering that HepG2 cells are significantly affected by LRP5/6 gene knockdown, genome-wide transcriptional changes in HepG2 cells after knockdown of LRP5/6 or β-catenin are analyzed, try to investigate the effect of knockdown of LRP5/6 gene itself on GPCR expression level.

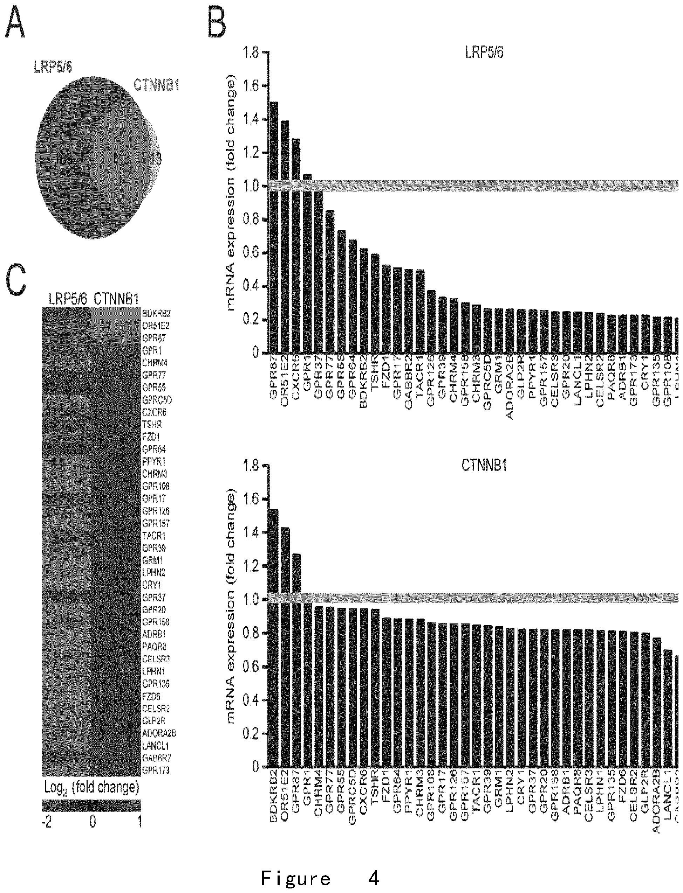

RNA-Seq analysis of HepG2 cells shows that compared with knockdown of β-catenin gene, knockdown of LRP5/6 gene leads to a significant number and extent of GPCRs with altered expression levels (Fig. 4, AC). This suggests that knockdown of the LRP5/6 gene can specifically cause changes at the transcriptional level of the GPCRs interacting with it via a feedback loop. These results demonstrate a broad and close relationship between LRP5/6 and GPCRs, which is independent of the Wnt/ β -catenin pathway, and provide a basis for our study of the molecular mechanisms of LRP5/6 regulation of cellular and diabetic heart damage.

### Example 5 LRP6 extracellular segment (LRP6N) can reverse LRP5/6 deletion-induced DNA damage

Initially, we have found that by knocking down LRP5/6 or β-catenin in HUVEC cells, we have observed that knockdown of LRP5/6 have resulted in DNA damage (up-regulation of yH2AX signaling), this phenomenon does not occur in both the control group and knockdown of β-catenin (Fig. 5B). In addition to simultaneous knockdown of LRP5/6, WB results show that compared with knockdown of β-catenin, knockdown of LRP5 or LRP6 alone in HUVEC cells with different concentration gradients of siRNA can also lead to DNA damage (up-regulation of yH2AX signal) (Fig. 5A). These results suggest that knockdown of LRP5/6 gene induces homeostatic disturbance in HUVEC cells, and this function of LRP5/6 is independent of β-catenin. Interestingly, by pre-adding a soluble protein of the extracellular segment of LRP6 (LRP6 ectodomain, LRP6N) to HUVEC cell culture medium, it is found that knockdown of LRP5/6-induced upregulation of yH2AX signaling is significantly attenuated by LRP6N (Fig. 5B). Since the secreted LRP6N protein acts on the cell membrane surface, the rescue of LRP6N protein in DNA damage induced by knockdown of LRP5/6 suggests that the deletion of LRP5/6 on the cell membrane is the fundamental cause of DNA damage in cells.

H₂O₂ can simulate an abnormal culture environment in a disease state in vitro. The peroxide of H₂O₂ can directly attack the DNA double-strand of cells and cause oxidative DNA damage without causing changes in the expression of LRP5/6 and β-catenin, and the degree of DNA damage induced by H₂O₂ is concentration- and time-dependent (Fig. 5C). In order to further investigate the biological role of LRP5/6 in maintaining cell homeostasis, we have further treated HUVEC cells based on the above experiments using an adverse stimulus (H₂O₂) that mimics the disease state. When we stimulated HUVEC cells with a low concentration of H₂O₂ (50 µM), we have found that compared with the control group and knockdown of β-catenin, the up-regulated yH2AX signaling by knockdown of LRP5/6 is further activated by H₂O₂, and this change is significantly attenuated by pretreatment with exogenous LRP6N protein (Fig. 5B). In contrast, LRP6N protein has no effect on H₂O₂-activated yH2AX signaling in control and β-catenin-knockdown HUVEC cells (Fig. 5B). These results suggest that the loss of LRP5/6 at the cell membrane attenuates the resistance of HUVEC cells to oxidative stress, and this phenomenon is independent of β-catenin.

### Example 6 MESD deletion enhances oxidative stress effects

As a chaperone protein of LRP5/6, MESD protein can help it transfer from the cytoplasm to the cell membrane and become mature LRP5/6 to exert biological functions. WB results show that knockdown of MESD gene in HUVEC cells can promote the reduction of LRP5/6 on the cell membrane (Fig. 6A), but no obvious cell damage occurs (Fig. 6B). Further studies show that yH2AX signaling in knockdown MESD cells is greatly enhanced by treatment with low concentration of H₂O₂ (50 µM), and pretreatment with exogenous LRP6N protein significantly attenuats this change (Fig. 6B). These results strongly suggest that intact membrane LRP5/6 is critical for enhancing the ability of cells to resist oxidative stress.

### Example 7 Dkkl can induce DNA damage effect

The secreted protein Dkkl induces the endocytosis of LRP5/6 on the cell membrane and inhibits the Wnt/β-catenin signaling pathway. Since the blood Dkkl level is significantly up-regulated in patients with many chronic diseases (such as diabetes, chronic myocardial ischemia, malignant tumor, etc.) in adulthood, it is associated with poor prognosis, but the mechanism is still unclear. To further explore the mechanism, different time and concentration gradients of purified Dkkl protein were added to HUVEC cells, and it is found that Dkkl can rapidly induce the endocytosis of LRP5 (100 ng/ml from 15 min) and LRP6 (100 ng/ml from 5 min) on the cell membrane (the total protein expression level is unchanged, while the membrane protein expression level is down-regulated) (Fig. 7A). Notably, Dkkl (100 ng/ml) significantly activats yH2AX within 5 min and peaked at 15 min (Fig. 7A). This suggests the possibility that Dkkl can cause cellular DNA damage while endocytosing LRP5/6. Second, we have also found that Dkkl (100 ng/ml) has down-regulated the expression level of β-catenin in the nucleus after 60 min (the total protein expression level is unchanged, while the nuclear protein expression level is down-regulated), however, higher doses of Dkkl does not down-regulate nuclear β-catenin expression within 30 min, suggesting that Dkkl does not induce cellular DNA damage responses by inhibiting β-catenin signaling (Fig. 7A). In addition, as shown in Figure 6B, greater reduction in β-catenin by using siRNA alone also does not alter yH2AX signaling, further supporting this opinion. To further demonstrate the intrinsic relationship between LRP5/6 membrane endocytosis and yH2AX activation, we pretreated HUVEC cells with MDC, an inhibitor of Dkk1 endocytosis of LRP5/6, as shown in Figure 7B, and have found that Dkkl-induced yH2AX activation is attenuated by MDC and similar to direct knockdown of LRP5/6, pretreatment with exogenous LRP6N protein also inhibits Dkkl-induced yH2AX activation (Fig. 7B). These results suggest that the secreted LRP6N protein can replace endogenous LRP5/6 on the cell membrane to protect cellular homeostasis. Based on the above data, it can be seen that the down-regulation of LRP5/6 on the membrane induced by Dkkl is the initial cause of cellular DNA damage, that is, Dkkl-induced cell damage is achieved by downregulating LRP5/6 on the membrane rather than by preventing nuclear translocation of β-catenin.

In addition, in order to investigate whether LRP5/6 on the membrane also has the above-mentioned protective effect on cells in a bad environment, we further stimulated HUVEC cells with low concentration of H₂O₂ (50 µM) on the basis of the above experiments. Experiments show that H₂O₂ itself can activate yH2AX signaling without affecting the expression of LRP5/6 on the cell membrane, and when Dkkl induces the downregulation of LRP5/6 on the membrane, H₂O₂ can strongly upregulate yH2AX signaling (Fig. 7B). Notably, MDC or LRP6N by themselves does not elicit yH2AX responses, and also does not prevent H₂O₂-induced yH2AX activation, but they have significantly attenuated the yH2AX signaling that is enhanced by H₂O₂ induction in the presence of Dkkl (Fig. 7B). These results further suggest that intact membrane LRP5/6 is critical for resistance to oxidative stress and maintenance of cellular homeostasis.

### Example 8 Dkk1 activates β-arrestin1/2 signaling through endocytosis of LRP5/6

The above RNA-Seq analysis has demonstrated a close relationship between LRP5/6 and most GPCRs, so that it is not difficult to speculate that the downregulation of LRP5/6 on the membrane may lead to an imbalance in the homeostasis of the GPCR membrane, which further cause complex changes in downstream signaling pathways. As mentioned earlier, β-arrestin1/2, like various G proteins, are general direct downstream targets of GPCRs, but unlike GPCRs and G proteins, β-arrestin1/2 can rapidly translocate to the cell membrane and directly binds to the C-terminus of activated GPCRs and is specifically involved in the regulation of general GPCR signaling through transport between its own cell membrane and the cytoplasm/nucleus, therefore, a general consequence of altered GPCR signaling is intracellular translocation of β-arrestin1/2. To test whether widespread GPCR signaling disturbances correlate with LRP5/6 downregulation, we examined the specific effects of Dkkl-induced membrane endocytosis of LRP5/6 on β-arrestin1/2 signaling alterations, that is, the relationship between LRP5/6 and GPCRs on the membrane was further elucidated by the signaling of intracellular β-arrestin1/2.

We have first analyzed the altered translocation of β-arrestin1/2 after Dkkl stimulation of HUVEC cells. The results show that Dkkl can rapidly up-regulate β-arrestin1/2 in HUVEC cytoplasm and down-regulate β-arrestin1/2 on the cell membrane while endocytosing LRP5/6 (Fig. 8A) and pretreatment with MDC, an inhibitor of Dkk1 endocytosis of LRP5/6, can prevent the above translocation changes of β-arrestin1/2 (Fig. 8B). Similar rapid changes in LRP5/6 and β-arrestin1/2 are induced by Dkkl stimulation within a short time (15 min), suggesting that Dkkl first binds and rapidly induces the endocytosis of LRP5/6 on the cell membrane, which then leads to altered translocation of β-arrestin1/2, suggesting a direct and close relationship between LRP5/6 and GPCRs on the membrane (Fig. 8A). In general, when GPCRs are activated, translocation of the inner plasma membrane G proteins relative to these GPCRs occurs first, followed by translocation of intracellular β-arrestin1/2 relative to these GPCRs. To this end, we pretreated HUVEC cells with the small molecule BIM-46187 (1 µM for 30 min), which is a general inhibitor of G protein and can directly bind to the alpha subunit of G protein, thereby preventing the formation of ligand-activated GPCR and G protein complexes and the subsequent conformational changes of G proteins, because such binding prevents the normal interaction between the receptor and the G protein heterotrimer, thereby inhibiting the GDP/GTP exchange of the α subunit of the G protein, resulting in extensive inhibition of G protein and GPCR signaling. Indeed, we have found that BIM-46187 is able to prevent Dkkl-induced translocation of β-arrestin1/2 in HUVEC cells (Fig. 8C), suggesting that Dkkl-induced downregulation of LRP5/6 at the membrane affects GPCR signaling, which in turn leads to changes in the translocation of β-arrestin1/2, the general downstream target of GPCRs. It is worth noting that the downregulation of LRP5/6 by RNAi technology is a long-term process and may be secondary to unpredictable secondary effects. Therefore, knockdown of LRP5/6 but not β-catenin in HUVEC cells up-regulates the expression levels of β-arrestin1/2 (Fig. 8D), it is possible that the feedback system indirectly initiates the above changes at the transcriptional level due to the close relationship between LRP5/6 and GPCRs. Taken together, these results imply that Dkkl can affect GPCR signaling by inducing LRP5/6 endocytosis, and further demonstrate that LRP5/6 is indeed closely associated with GPCRs.

### Example 9 β-arrestin1/2 mediates cellular DNA damage caused by downregulation of LRP5/6 on the membrane

It is currently known that β-arrestin1/2, the direct downstream target of GPCR, can not only participate in GPCR signal transmission through the transport between the cell membrane and the cytoplasm/nucleus, but also mediate DNA damage response. Therefore, on the one hand, the endocytosis of LRP5/6 on the HUVEC cell membrane by Dkkl rapidly induces the transfer of β-arrestin1/2 from the cell membrane to the cytoplasm, which in turn leads to the activation of cellular yH2AX; On the other hand, inhibiting the endocytosis of LRP5/6 by Dkkl by MDC also prevents the translocation of β-arrestin1/2 into cells and the activation of yH2AX. Similarly, knockdown of β-arrestin1/2 in HUVEC cells (Fig. 9A) not only blocks GPCR-β-arrestin1/2 signaling, but also inhibits yH2AX activation induced by Dkk1 stimulation (Fig. 9B). It is worth noting that knockdown of β-arrestin1/2 also has obvious rescue effect for the enhanced yH2AX signal induced by successive stimulation of Dkkl and low concentration of H₂O₂ (50 µM), but knockdown of β-arrestin1/2 does not protect H₂O₂ itself induced cellular DNA damage (Figure 9B), suggesting a specific role for β-arrestin1/2 in promoting Dkkl-induced DNA damage. In addition, Dkkl stimulation-induced yH2AX activation is also inhibited by pretreatment with a general inhibitor of G protein, BIM-46187 (1 µM, 30 min) (Fig. 9C). Based on the above experimental results, it can be seen that the Dkk1-LRP5/6 axis mediates cellular DNA damage and homeostatic imbalance caused by LRP5/6 endocytosis by activating GPCR-β-arrestin1/2 signaling (altered β-arrestin1/2 translocation).

It is worth mentioning that, similar to the above-mentioned results of knockdown of β-arrestin1/2 inhibiting Dkkl on cell damage, knockdown of β-arrestin1/2 in HUVEC cells can also inhibit DNA damage and impairment of anti-oxidative stress caused by knockdown of LRP5/6 and this protective effect is independent of β-catenin (Fig. 9D). All the above results demonstrate that the direct downstream molecular targets of GPCRs, β-arrestin1/2, are direct regulators of cellular DNA damage and homeostatic imbalance induced by LRP5/6 deletion.

### Example 10 G protein agonists are able to induce DNA damage responses

The above experimental results have revealed an important function of the Dkk1-LRP5/6-GPCR-β-arrestin1/2 pathway in Dkkl-induced cellular DNA damage. Next, in order to further verify the close relationship between GPCR signaling disturbance and cellular DNA damage, we used G protein agonists to interfere with the direct downstream target G proteins of GPCRs to observe whether cellular DNA damage can also be induced. Experiments have showed that CTX (cholera toxin) can continuously activate the α subunit of Gs protein, which stimulate HUVEC cells to significantly upregulate yH2AX in a dose- and time-dependent manner ( FIG. 10A ). Notably, PTX (pertussis toxin) rapidly inhibits the Gi protein alpha subunit, while long-term stimulation can activate the Gs protein alpha subunit, and yH2AX is activated by different doses of PTX in both short- and long-term stimulation (Fig. 10B). These results suggest that affecting the function of different G proteins can induce DNA damage responses. Thus, as a general downstream target of GPCRs, disruption of G protein homeostasis broadly alters GPCR signaling, which in turn leads to imbalances in cellular homeostasis.

### Example 11 LRP5/6 gene knockout induces cardiac injury in mice

We used 8-week-old LRP5/6 or β-catenin systemic knockout (LRP5/6-/- or β-catenin-/-) mice induced by intraperitoneal injection of tamoxifen of the effects on LRP5/6 in adult tissues to be elucidated. They were obtained by mating LRP5/6^{floxp/floxp} or β-catenin^{floxp/floxp} mice, respectively, with mice containing Cre recombinase (UBC-Cre/ESR1) whose expression is under the control of a systemically expressed UBC promoter, systemic knockout of genes can be achieved. Taking the heart as an example, Western Blotting has confirmed from the protein level that specific genes are successfully knocked out in the hearts of LRP5/6-/- or β-catenin-/- mice induced by tamoxifen for 1 week (FIG. 11A).

During the experiment, compared with control mice, LRP5/6-/- mice induced by tamoxifen for 35 weeks show a reduction in body weight and weight of various organs including heart, lung, kidney, spleen, skeletal muscle and fat (Fig. 11B), β -catenin-/- mice do not show these changes, suggesting that LRP5/6 deletion specifically induces severe damage to the body and multiple organs. Subsequently, we selected the heart of interest as the research object of organ damage, and further investigated the effect of LRP5/6 knockout on the heart. We have found that yH2AX signaling is significantly increased in the hearts of LRP5/6-/- but not β-catenin-/- mice induced by tamoxifen for 1 week, and p53 and p21 expression levels are also significantly up-regulated. However, DNA damage in the hearts of LRP5/6-/- mice does not induce apoptosis (Fig. 11A). In addition, two-dimensional echocardiography shows impaired cardiac physical function, including reductions in left ventricular ejection fraction (EF%) and left ventricular fractional shortening (FS%), in LRP5/6-/- mice induced by tamoxifen for 35 weeks (Figures 11C and 11E); real-time quantitative PCR analysis also shows that LRP5/6-/- mice heart failure markers ANP and BNP are significantly upregulated (Figure 11D), these results further suggest that LRP5/6 knockout can cause heart damage. In contrast, there is no significant difference between β-catenin^{-/-} mice and littermate control mice expressing only Cre recombinase until 35 weeks after knockout. Taken together, short-term knockout of LRP5/6 can specifically induce the DNA damage response and cell cycle arrest in adult mouse heart, while long-term LRP5/6 deletion causes accumulation of DNA damage, which in turn leads to impaired cardiac function. In conclusion, the above experiments preliminarily demonstrate that LRP5/6 can protect the normal function and homeostasis of the adult mouse heart.

### Example 12 LRP5/6 knockout activates β-arrestin1/2 signaling in mouse heart

Cellular experiments show that β-arrestin1/2, the general downstream targets of GPCRs, can not only participate in GPCR signaling but also mediate DNA damage responses through transport between the cell membrane and the cytoplasm/nucleus. Similarly, to demonstrate whether the above-mentioned damage changes in LRP5/6-/- mouse hearts are related to altered GPCR-β-arrestin1/2 signaling, we examined β-arrestin1/2 in LRP5/6-/- mouse hearts. As shown in Figure 12A, LRP5/6-/- but not β-catenin-/- mice are able to induce altered translocation of β-arrestin1/2 in the heart (including downregulation of β-arrestin1/2 in the cell membrane, upregulation of β-arrestin1/2 in cytoplasm and β-arrestin1 in nucleus), indicating that β-arrestin1/2 can mediate the cardiac DNA damage caused by LRP5/6 knockout, and also provide direct evidence for the close relationship between LRP5/6 and GPCRs. In addition, real-time quantitative PCR also shows (Fig. 12B) that LRP5/6-/- but not β-catenin-/- mice induces changes in the levels of multiple important GPCR genes in the heart, further supporting the idea that LRP5/6 is closely related to multiple GPCRs. In conclusion, the above results preliminarily indicate that LRP5/6 has the biological function of maintaining the stability of GPCR-β-arrestin1/2 signaling in adult mice independent of β-catenin, thereby preventing the biological function of cardiac DNA damage and dysfunction.

### Example 13 Hyperglycemia induces up-regulation of blood Dkkl in diabetic mice

Our ELISA assays show that blood Dkkl levels are significantly increased in STZ-induced type 1 diabetic mice (Fig. 13A). Interestingly, treatment of diabetic mice with daily insulin administration not only reduces STZ-induced blood glucose elevation (FIG. 13B), but has also brought high blood Dkkl concentrations back to basal levels (FIG. 13A). These results suggest that STZ-induced diabetic hyperglycemia can specifically upregulate blood Dkkl, and insulin can restore the elevated blood Dkkl level to normal by virtue of its hypoglycemic effect. Since STZ-induced diabetic mice resemble diabetic patients with high Dkkl expression in their blood, we used this mouse model to investigate the mechanism underlying the deleterious effects of Dkkl on diabetes.

### Example 14 The close relationship between the down-regulation of LRP5/6 on the membrane of diabetic mice and cardiac injury

As previously mentioned, Dkkl has dual biological functions of inhibiting Wnt/β-catenin signaling and LRP5/6 on the endocytic membrane. To determine what role high blood levels of Dkkl play in diabetes, we investigated the expression of β-catenin and LRP5/6 in the hearts of STZ-induced type 1 diabetic mice. As shown in Figure 14A, at different time points of STZ modeling, we have found no changes in cardiac nuclei and overall β-catenin, suggesting that the Wnt/β-catenin signaling pathway is not involved in the pathogenesis of diabetes. It is worth noting that accompanied by up-regulation of blood glucose concentration (significantly increased on the 5th day of STZ modeling) and blood Dkkl, the expression level of LRP5/6 on the cardiac membrane is significantly down-regulated on the 5th day of modeling and becomes more pronounced on the 7th day of modeling, but the overall LRP5/6 expression level remains unaffected, suggesting that endocytosis of LRP5/6 in the cardiac membrane may be associated with hyperglycemia-induced upregulation of blood Dkkl in STZ-induced type 1 diabetic mice (Fig. 14A). In addition, γ H2AX signal indicating cardiac DNA damage is also significantly increased from day 5 of STZ modeling and peaked on day 7 (Fig. 14A). Blood 8-OHdG is also significantly up-regulated on day 7 of STZ modeling (Fig. 14C), further confirming the occurrence of diabetic DNA damage. The temporal consistency of these changes in STZ diabetic mice implies that the downregulation of LRP5/6 in cardiac membranes and the development of DNA damage are closely related to hyperglycemia-upregulated Dkkl. At the same time, as shown in Figure 14D, diabetic mice on day 7 of STZ modelling also show marked upregulation of the levels of other injury markers (including cardiac p53 and p21, Bax/Bcl-2 and Cleaved Caspase-3), suggesting that diabetes contributes to severe cardiac DNA damage, ultimately leading to apoptosis.

It is worth noting that, as shown in Fig. 14B, after daily insulin treatment of STZ-induced diabetic mice, the blood glucose concentration has decreased from the first day of treatment and remained at a low level throughout the subsequent time, at the same time, blood Dkkl is also at the basal level due to the decrease of blood glucose concentration. On day 3 of insulin treatment, LRP5/6 on the membrane of the heart is started to be up-regulated, accompanied by a decrease in yH2AX signaling and these changes become more pronounced on day 7 of treatment and as shown in Figures 14C and 14E, the expression of other injury markers, including blood 8-OHdG; cardiac p53 and p21, Bax/Bcl-2 and Cleaved Caspase-3, is also greatly attenuated in the diabetic mice on day 7 of insulin treatment. Considering that weight loss is a hallmark sign of multiple organ damage in type 1 diabetes, we have also found that the hypoglycemic effect of insulin has promoted the gradual recovery of the weight loss in diabetic mice on the basis of the above changes (Fig. 14F). The above results show that the temporal consistency of insulin treatment on the downregulation of LRP5/6 on the cardiac membrane and the reversal of diabetic injury further suggests that diabetic cardiac injury is closely related to the tightly regulated blood Dkkl and membrane LRP5/6 expression levels.

### Example 15 Dkkl induces heart injury from diabetes by inducing LRP5/6 membrane endocytosis

To further demonstrate the correlation of LRP5/6 membrane endocytosis and DNA damage in the heart of diabetic mice with the up-regulation of Dkkl levels, we used MDC, an inhibitor that prevents Dkkl from endocytosing LRP5/6 on the membrane, and observed the role of membrane Dkk1-LRP5/6 axis in diabetic heart injury (heart injury from diabetes). As shown in Figure 15A, we have found that STZ-induced downregulation of LRP5/6 in diabetic cardiac membranes can be inhibited by MDC, while overall LRP5/6 and nuclear or overall β-catenin expression levels remain unchanged, indicating that Dkkl specifically mediates the membrane endocytosis of LRP5/6 in diabetic hearts, but does not affect the canonical Wnt pathway. Meanwhile, as shown in Figures 15B and 15C, we show by immunofluorescence staining and Western Blotting, respectively, that yH2AX activation in diabetic hearts can also be inhibited by MDC, similar to the rescue of DNA damage by insulin. In addition, MDC has also prevented the elevation of 8-OHdG in the blood of diabetic mice (FIG. 15D). These results suggest that DNA damage in diabetic mice is mainly regulated by Dkkl-induced downregulation of LRP5/6 at the membrane. As shown in Figures 15C and 15E, MDC also inhibits the upregulation of p53, p21, Bax/Bcl-2 and Cleaved Caspase-3 in diabetic hearts, as well as weight loss in diabetic mice. HE staining of the heart shows that STZ modeling causes significant damage to the structure of the diabetic myocardium, including the disorder and rupture of myocardial fibers, and the expansion of interstitial spaces, all of which can be prevented by insulin (positive control) and MDC (Figure 15F). Importantly, MDC prevents the above damage without altering blood glucose concentrations in diabetic mice (Fig. 15G), suggesting that hyperglycemia-induced diabetic damage is essentially due to downregulation of LRP5/6 on the membrane. These results strongly suggest the possibility that Dkk1 contributes to diabetic heart damage by inducing downregulation of LRP5/6 at the membrane. To demonstrate the direct relationship between Dkkl and cardiac injury, we injected Dkkl purified protein into myocardium, and the results show that Dkkl can directly induce the down-regulation of LRP5/6 and the up-regulation of yH2AX, p53 and p21 on the cardiac membrane without affecting β-catenin signaling, and these changes can also be reversed by MDC pretreatment (Fig. 15H), suggesting that Dkkl can directly downregulate LRP5/6 on the membrane to induce cardiac DNA damage and cell cycle arrest. In addition, as shown in Figure 151, we have found in the experiment of local injection of Dkkl purified protein combined with BIM-46187 into mouse heart that BIM-46187 (a general inhibitor of G protein) can also effectively inhibit Dkkl-induced cardiac injury through LRP5/6 membrane endocytosis, including yH2AX, p53 and p21, this result suggests that Dkk1-LRP5/6 axis-mediated cardiac DNA damage and cell cycle arrest may be associated with GPCR signaling activation by downregulation of LRP5/6 at the membrane.

In conclusion, the above results preliminarily suggest a molecular mechanism of LRP5/6-independent regulation of β-catenin in diabetic heart damage, that is, the hyperglycemic phenotype of diabetes induces the upregulation of blood Dkkl, which leads to the downregulation of LRP5/6 on the membrane, namely the Dkk1-LRP5/6 axis is altered, which in turn affects the stability of GPCR signaling, causing cardiac DNA damage, cell cycle arrest, and even apoptosis and other homeostatic imbalances, which ultimately lead to diabetic heart damage.

### Example 16 Down-regulation of LRP5/6 on the membrane of Leptin-/- mice leads to diabetic heart damage

Obesity is considered to be a high risk factor for the development of type 2 diabetes, and the prevalence of type 2 diabetes has been on the rise in recent years with the sharp increase in the number of obesity. As mentioned earlier, Leptin-/- mice displayed an obese phenotype with severe hyperglycemia, therefore, we used it as a mouse model to simulate type 2 diabetes to investigate the relevant role of LRP5/6 in it. As shown in Figures 16A and 16B, 5-week-old Leptin-/- mice have significantly increased body weight and blood glucose compared to littermate Leptin+/- or WT mice. As shown in Figure 16C, when the blood glucose concentration of 6-week-old Leptin-/- mice has reached the highest level, LRP5/6 on the membrane of the heart is significantly down-regulated, and the expression of yH2AX, p53, and p21 is also slightly up-regulated, but no nuclear translocation of β-catenin has occurred. Notably, the expression levels of LRP5/6 on cardiac membranes are similar between 4-week-old Leptin-/-, Leptin+/- and WT littermates (Fig. 16D), while, the blood glucose levels of 4-week-old Leptin-/- and WT mice is also very similar, but body weight levels in Leptin-/- mice have increased significantly (Figures 16A and 16B), suggesting that downregulation of LRP5/6 in the membranes of 6-week-old Leptin-/- mice is a caused by high blood sugar rather than high body weight (the obesity factor). In addition, as shown in Figure 16E, ELISA test shows that the blood Dkkl level of 6-week-old Leptin-/- mice is significantly up-regulated, indicating that Dkk1 may be involved in inducing the down-regulation of LRP5/6 and DNA damage on the cardiac membrane of type 2 diabetes.

Considering the generally impaired glucose tolerance in diabetic patients, we performed glucose tolerance tests on 6-week-old Leptin-/- mice. The results are shown in Figure 16F, the blood glucose of Leptin-/- mice has maintained a high level for up to 2 h after glucose injection, but the blood glucose of WT mice has decreased immediately within 15 min, indicating that there is a delay in the clearance of blood glucose in Leptin-/- mice. Interestingly, as shown in Figures 16H, 161 and 16J, in vivo injection of glucose (every 2 h for 24 h) has significantly induced downregulation of LRP5/6 in the cardiac membrane of Leptin-/- mice, activation of yH2AX and blood 8 -OHdG is up-regulated, and cardiac p53, p21 and Bax/Bcl-2 are up-regulated, while nuclear β-catenin has no significant change. To verify whether these changes are related to Dkkl, firstly, we have found that blood Dkkl levels in Leptin-/- mice injected with glucose are significantly higher than those injected with PBS by ELISA (Fig. 16G); Second, MDC has significantly reversed the down-regulation of LRP5/6 on cardiac membranes and the up-regulation of the aforementioned molecular markers of injury induced by glucose injection (Figures 16H, 161 and 16K). These results demonstrate a deleterious role of blood Dkkl, which is directly upregulated by hyperglycemia, in promoting endocytosis of LRP5/6 at the membrane and in diabetic heart injury, while also providing a novel mechanism that may well explain the importance of restricting glucose intake in patients with metabolic syndrome for ameliorating diabetic damage.

In conclusion, the in vivo results of the above-mentioned mouse models of type 1 and type 2 diabetes collectively demonstrate that hyperglycemia induces upregulation of Dkkl in the blood and downregulation of LRP5/6 on the cell membrane, which in turn leads to a DNA damage response, cell cycle arrest, and even apoptosis in the hearts of diabetic mice. Therefore, the integrity of LRP5/6 on the cell membrane is crucial for maintaining tissue and organ homeostasis. In addition, MDC may hold promise as a clinical drug for organ damage in patients with type 1 and type 2 diabetes by virtue of its role in stabilizing the expression of LRP5/6 on the membrane.

### Example 17 LRP5/6 gene knockout aggravates diabetic heart damage

Cellular experiments show that knockdown of the LRP5/6 gene can significantly increase the DNA damage of HUVEC cells in a poor culture environment (H₂O₂). It can be seen that the presence of LRP5/6 does help maintain cell homeostasis. Similarly, to further explore the function of LRP5/6 in β-catenin-independent maintenance of adult body homeostasis, we modeled STZ-induced diabetes in systemic LRP5/6-/- or β-catenin-/- mice 4 weeks after tamoxifen induction to investigate whether the loss of LRP5/6 in vivo can aggravate diabetes-induced cardiac damage. As shown in Figure 17A, STZ-induced systemic LRP5/6-/- diabetic mice for 1 week has more severe cardiac molecular damage than systemic non-knockout diabetic mice, including significantly up-regulated yH2AX, p53, p21, Bax/Bcl-2 as well as Cleaved Caspase-3 (Fig. 17A); and, diabetic mice with systemic LRP5/6-/- has developed markedly impaired cardiac physical function at 2 weeks of STZ induction (including EF% and FS% reduction, Figures 17B and 17E). It is worth mentioning that, compared with the systemic non-knockout diabetic mice, the above-mentioned molecular indicators of cardiac injury and cardiac physical function do not show significant changes in the systemic β-catenin-/- diabetic mice (Figures 17B and 17E ). In addition, compared with the systemic non-knockout diabetic mice, the systemic LRP5/6-/- diabetic mice also shows a more pronounced weight loss at 1 week of STZ induction (Fig. 17C), but there is no difference in blood glucose levels between the two (Fig. 17D). The above results collectively show that complete deletion of LRP5/6 can significantly aggravate diabetic heart and body damage, and this phenomenon is not affected by β-catenin signaling and blood glucose levels; it further proves that LRP5/6 itself has a general biological function to maintain the homeostasis of the adult body independent of β-catenin.

### Example 18 LRP6 extracellular segment (LRP6N) prevents diabetic heart damage

Given the fact that exogenous LRP6N protein was able to prevent LRP5/6 knockdown-induced cellular DNA damage in vitro, we further investigated whether LRP6N could also prevent diabetic heart damage in vivo. First, as shown in Fig. 18A, we used transposon technology to construct transgenic mice carrying LRP6N gene fragments, and through the conditional Cre-loxP recombinase system, mice were induced to overexpress LRP6N protein (with myc tag) systemically. In the hearts of LRP6N/Tg mice 4 weeks after tamoxifen induction, no other molecular changes are observed except for LRP6N overexpression (Fig. 18B). However, as shown in Figures 18C and 18D, LRP6N/Tg mice prevents STZ modeling-induced upregulation of yH2AX, p53, p21, Bax/Bcl-2 and Cleaved Caspase-3 in diabetic hearts and blood 8-OHdG (Figure 18C and 18D). At the same time, LRP6N/Tg mice has also inhibited the STZ model-induced downregulation of LRP5/6 on endogenous membranes (Fig. 18C). Similarly, as shown in Figures 18E and 18F, injection of the LRP6N plasmid (myc-tagged) via the tail vein of mice has also attenuated increases in yH2AX, p53, p21, Bax/Bcl-2 and Cleaved Caspase-3 in diabetic hearts (Figure 18E and 18F). Furthermore, as shown in Figure 18G, compared with the respective control groups, the knockout of endogenous LRP5/6 or β-catenin in mice and the overexpression of exogenous LRP6N (such as transgenic or in vivo transfection plasmids) do not change the blood glucose concentration of the body, indicating that changes in the expression of LRP5/6 or β-catenin in vivo do not have any effect on blood glucose metabolism (Fig. 18G). It is worth emphasizing that since the secreted LRP6N protein exerts a protective role at the cell membrane surface, these in vivo results further suggest that diabetic heart injury is directly attributable to downregulation of LRP5/6 at the membrane independent of β-catenin signaling and blood glucose levels. It also shows that LRP6N can replace endogenous LRP5/6 to prevent the occurrence of diabetic heart damage.

As shown in Figure 18H, overexpression of LRP6N in LRP6N/Tg mice and tail vein-injected mice with LRP6N plasmid can effectively prevent STZ-induced diabetes weight loss (Figure 18H). these results suggest that LRP6N has the ability to generally prevent diabetic damage. As previously mentioned, the wasting phenotype caused by multiple organ damage in type 1 diabetic mice is also suppressed by insulin and MDC. Since LRP6N overexpression and MDC intervention do not alter blood glucose levels, soluble LRP6N protein and MDC can be used as potential therapeutic options for diabetic damage, which can protect the body and organs even under hyperglycemic conditions .

### Example 19 Downregulation of LRP5/6 on the membrane of diabetic mice specifically activates cardiac β-arrestin1/2 signaling

Since hyperglycemia affects LRP5/6 expression on the membrane of diabetic hearts, we also wondered whether hyperglycemia also affected GPCR-β-arrestin1/2 signaling. First, as shown in Figures 19A and 19B, we have found the translocation of β-arrestin1/2 in STZ-induced type 1 diabetic hearts (including downregulation of membrane β-arrestin1/2, upregulation of β-arrestin1/2 in plasma and β-arrestin1 in nucleus) (Fig. 19A), and insulin treatment is able to reverse this translocation (Fig. 19B), suggesting that hyperglycemia can affect β-arrestin1/2 signaling. Second, as shown in Figures 19C and 19D, hyperglycemia-induced changes in β-arrestin1/2 translocation is also prevented by MDC intervention or LRP6N overexpression in LRP6N/Tg mice (Figures 19C and 19D), suggesting that hyperglycemia induces the translocation of cardiac β-arrestin1/2 by affecting the Dkk1-LRP5/6 axis (up-regulation of Dkkl and down-regulation of LRP5/6 on the membrane). Supporting this notion, direct injection of Dkkl into the heart induces down-regulation of membrane β-arrestin1/2 and plasma β-arrestin1/2 and nuclear β-arrestin1 are up-regulated, which can be inhibited by MDC (Fig. 19E) or BIM-46187 (Fig. 19F), suggesting that Dkkl directly affects GPCR-β-arrestin1/2 signaling by downregulating LRP5/6 on the cardiac membrane (Dkk1-LRP5/6 axis). As shown in Figure 19G, since neither MDC nor in vivo overexpression of LRP6N induced cardiac β-arrestin1/2 translocation with insulin alone, these results further support that hyperglycemia specifically affects GPCR-β-arrestin1/2 signaling by up-regulating Dkkl and down-regulating LRP5/6 on the membrane.

As previously described, LRP5/6-/- but not β-catenin-/- mice also induced altered cardiac β-arrestin1/2 translocation (Fig. 12A), this provides direct evidence for LRP5/6 regulation of β-arrestin1/2. Since the general inhibitor of G protein BIM-46187 can significantly prevent Dkkl direct endocytosis of LRP5/6-induced cardiac β-arrestin1/2 translocation changes (Fig. 19F) and DNA damage (Fig. 151) by inhibiting GPCR signaling disturbances, Loss of LRP5/6 can disrupt the homeostasis of numerous GPCRs and cause downstream β-arrestin1/2 signaling, which promotes DNA damage responses, and severe DNA damage can even lead to apoptosis, ultimately leading to impaired organ function.

It is worth noting that both LRP5/6 and insulin receptor (IR) are single-pass transmembrane proteins, and insulin receptor is thought to play a key role in the development of diabetes, which belongs to the receptor tyrosine kinase (RTKs) family, consists of two α subunits (IR-α) and two β subunits (IR-β) connected by disulfide bonds. The two alpha subunits are located outside the cell membrane and have binding sites for insulin; the two beta subunits are the transmembrane part of the receptor and play a role in signal transduction. To explore whether the protective effect of LRP5/6 in diabetic heart injury is related to insulin receptors, we investigated the expression of insulin receptors in diabetes and various intervention conditions. As shown in Figure 19A-E, insulin receptors on the membrane and in the cytoplasm of cardiac cells by direct injection of Dkkl protein or diabetic mice do not undergo translocation changes similar to LRP5/6 and β-arrestin1/2. In addition, insulin alone, MDC, or in vivo overexpression of LRP6N does not induce translocation of insulin receptors on the cardiac membrane or in the plasma (Fig. 19G). Therefore, these results strongly suggest that downregulation of LRP5/6 at the membrane is involved in diabetic heart injury by specifically regulating GPCR-β-arrestin1/2 signaling, independent of insulin receptors.

All documents mentioned herein are incorporated by reference in this application as if each document were individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. Use of an inhibitor of DKK1 gene or an encoded protein thereof for preparing a composition or preparation for the prevention and/or treatment of tumor cachexia and diseases accompanied with diabetes.

2. The use of claim 1, wherein the composition or preparation is further used for one or more purposes selected from the group consisting of:
(a) reducing the content of β-arrestin2 in a tumor cell;
(b) inhibiting the activation of IκB and NFκB in a mammal;
(c) Inhibiting the up-regulation of p53 and Bax/Bcl-2;
(d) inhibiting down-regulation of a muscle protein marker;
(e) inhibiting cytokine upregulation in a mammalian muscle tissue.

3. A pharmaceutical composition, comprising:
(a1) a first active ingredient for preventing and/or treating tumor cachexia and diseases accompanied with diabetes, the first active ingredient comprises: an inhibitor of *DKK1* gene or an encoded protein thereof;
(a2) a second active ingredient for preventing and/or treating tumor cachexia and diseases accompanied with diabetes, the second active ingredient comprises: other drugs for preventing and/or treating tumor cachexia and diseases accompanied with diabetes; and
(b) a pharmaceutically acceptable carrier.

4. A kit, comprising:
(i) a first container, and an active ingredient (a1) an inhibitor of *DKK1* gene or an encoded protein thereof, or a drug containing the active ingredient (a), located in the first container; and
(ii) a second container, and an active ingredient (a2) other drugs for preventing and/or treating tumor cachexia and diseases accompanied with diabetes, or a medicament containing the active ingredient (a2), located in the second container.

5. A method for reducing the content of β-arrestin2 in a tumor cell in vitro, comprising the steps of:
In the presence of the inhibitor of *DKK1* gene or an encoded protein thereof, culturing a tumor cell, thereby reducing the content of β-arrestin2 in a tumor cell.

6. Use of the pharmaceutical composition of claim 3 or the kit of claim 4 for the preparation of a medicament for preventing and/or treating tumor cachexia and diseases accompanied with diabetes.

7. A method for screening a potential therapeutic agent for tumor cachexia and diseases accompanied with diabetes, comprising:
(a) in a test group, in a culture system, in the presence of a test compound, culturing a cell expressing *DKK1* gene or a protein thereof for a period of time T1, and detecting the expression level E1 and/or activity A1 of *DKK1* gene or a protein thereof in the culture system of the test group;
and in the control group without the test compound and other conditions are the same, detecting the expression level E2 and/or activity A2 of the *DKK1* gene or a protein thereof in the culture system of the control group; and
(b) comparing E1 and E2, if E1 is significantly lower than E2, indicating that the test compound is a potential therapeutic agent for tumor cachexia and diseases accompanied with diabetes; or
comparing A1 and A2, and if A1 is significantly lower than A2, indicating that the test compound is a potential therapeutic agent for tumor cachexia and diseases accompanied with diabetes.

8. the method of claim 7, wherein the method includes step (c): administering the potential therapeutic agent identified in step (a) to a mammal, thereby determining its effects on tumor cachexia and diseases accompanied with diabetes in the mammal.

9. A method for screening a potential therapeutic agent for tumor cachexia and diseases accompanied with diabetes, comprising:
(a) in a test group, in a culture system, in the presence of a test compound, culturing a tumor cell for a period of time T1, detecting the formation of the complex of DKK1 and LRP6 in the culture system of the test group;
and in the control group without the test compound and other conditions being the same, detecting the formation of the complex of DKK1 and LRP6 in the culture system of the control group;
(b) if the formation quantity Q1 of the complex of DKK1 and LRP6 in the test group is significantly lower than the formation quantity Q2 of the complex of DKK1 and LRP6 in the control group, indicating that the test compound is a candidate compound.

10. The method of claim 9, wherein the method includes step (c): administering the candidate compound determined in step (b) to a mammal, thereby determining its effects on tumor cachexia and diseases accompanied with diabetes in a mammal.

11. A method for determining a therapeutic regimen for tumor cachexia and diseases accompanied with diabetes, comprising:
a) providing a test sample from a subject;
b) detecting the level of DKK1 protein in the test sample; and
c) determining a therapeutic regimen for tumor cachexia and diseases accompanied with diabetes based on the DKK1 protein level in the sample.


sequence listing

<110>  SHANGHAI DONGTSU BIOSCIENCES CO., LTD.

<120>  USE OF DKK1 INHIBITOR IN PREVENTION AND/OR TREATMENT OF TUMOR
CACHEXIA AND DISEASES
        ASSOCIATED WITH DIABETES

<130>  P2020-0577

<150>  CN201910600862.0
<151>  2019-07-04

<160>  38

<170>  PatentIn version 3.5

<210>  1
<211>  266
<212>  PRT
<213>  Homo sapiens

<400>  1

Met Met Ala Leu Gly Ala Ala Gly Ala Thr Arg Val Phe Val Ala Met
1               5                   10                  15

Val Ala Ala Ala Leu Gly Gly His Pro Leu Leu Gly Val Ser Ala Thr
            20                  25                  30

Leu Asn Ser Val Leu Asn Ser Asn Ala Ile Lys Asn Leu Pro Pro Pro
        35                  40                  45

Leu Gly Gly Ala Ala Gly His Pro Gly Ser Ala Val Ser Ala Ala Pro
    50                  55                  60

Gly Ile Leu Tyr Pro Gly Gly Asn Lys Tyr Gln Thr Ile Asp Asn Tyr
65                  70                  75                  80

Gln Pro Tyr Pro Cys Ala Glu Asp Glu Glu Cys Gly Thr Asp Glu Tyr
                85                  90                  95

Cys Ala Ser Pro Thr Arg Gly Gly Asp Ala Gly Val Gln Ile Cys Leu
            100                 105                 110

Ala Cys Arg Lys Arg Arg Lys Arg Cys Met Arg His Ala Met Cys Cys
        115                 120                 125

Pro Gly Asn Tyr Cys Lys Asn Gly Ile Cys Val Ser Ser Asp Gln Asn
    130                 135                 140

His Phe Arg Gly Glu Ile Glu Glu Thr Ile Thr Glu Ser Phe Gly Asn
145                 150                 155                 160

Asp His Ser Thr Leu Asp Gly Tyr Ser Arg Arg Thr Thr Leu Ser Ser
                165                 170                 175

Lys Met Tyr His Thr Lys Gly Gln Glu Gly Ser Val Cys Leu Arg Ser
            180                 185                 190

Ser Asp Cys Ala Ser Gly Leu Cys Cys Ala Arg His Phe Trp Ser Lys
        195                 200                 205

Ile Cys Lys Pro Val Leu Lys Glu Gly Gln Val Cys Thr Lys His Arg
    210                 215                 220

Arg Lys Gly Ser His Gly Leu Glu Ile Phe Gln Arg Cys Tyr Cys Gly
225                 230                 235                 240

Glu Gly Leu Ser Cys Arg Ile Gln Lys Asp His His Gln Ala Ser Asn
                245                 250                 255

Ser Ser Arg Leu His Thr Cys Gln Arg His
            260                 265

<210>  2
<211>  801
<212>  DNA
<213>  ÖÇÈË(Homo sapiens)

<400>  2
atgatggctc tgggcgcagc gggagctacc cgggtctttg tcgcgatggt agcggcggct      60

ctcggcggcc accctctgct gggagtgagc gccaccttga actcggttct caattccaac     120

gctatcaaga acctgccccc accgctgggc ggcgctgcgg ggcacccagg ctctgcagtc     180

agcgccgcgc cgggaatcct gtacccgggc gggaataagt accagaccat tgacaactac     240

cagccgtacc cgtgcgcaga ggacgaggag tgcggcactg atgagtactg cgctagtccc     300

acccgcggag gggacgcagg cgtgcaaatc tgtctcgcct gcaggaagcg ccgaaaacgc     360

tgcatgcgtc acgctatgtg ctgccccggg aattactgca aaaatggaat atgtgtgtct     420

tctgatcaaa atcatttccg aggagaaatt gaggaaacca tcactgaaag ctttggtaat     480

gatcatagca ccttggatgg gtattccaga agaaccacct tgtcttcaaa aatgtatcac     540

accaaaggac aagaaggttc tgtttgtctc cggtcatcag actgtgcctc aggattgtgt     600

tgtgctagac acttctggtc caagatctgt aaacctgtcc tgaaagaagg tcaagtgtgt     660

accaagcata ggagaaaagg ctctcatgga ctagaaatat tccagcgttg ttactgtgga     720

gaaggtctgt cttgccggat acagaaagat caccatcaag ccagtaattc ttctaggctt     780

cacacttgtc agagacacta a                                               801

<210>  3
<211>  272
<212>  PRT
<213>  Mus musculus

<400>  3

Met Met Val Val Cys Ala Ala Ala Ala Val Arg Phe Leu Ala Val Phe
1               5                   10                  15

Thr Met Met Ala Leu Cys Ser Leu Pro Leu Leu Gly Ala Ser Ala Thr
            20                  25                  30

Leu Asn Ser Val Leu Ile Asn Ser Asn Ala Ile Lys Asn Leu Pro Pro
        35                  40                  45

Pro Leu Gly Gly Ala Gly Gly Gln Pro Gly Ser Ala Val Ser Val Ala
    50                  55                  60

Pro Gly Val Leu Tyr Glu Gly Gly Asn Lys Tyr Gln Thr Leu Asp Asn
65                  70                  75                  80

Tyr Gln Pro Tyr Pro Cys Ala Glu Asp Glu Glu Cys Gly Ser Asp Glu
                85                  90                  95

Tyr Cys Ser Ser Pro Ser Arg Gly Ala Ala Gly Val Gly Gly Val Gln
            100                 105                 110

Ile Cys Leu Ala Cys Arg Lys Arg Arg Lys Arg Cys Met Arg His Ala
        115                 120                 125

Met Cys Cys Pro Gly Asn Tyr Cys Lys Asn Gly Ile Cys Met Pro Ser
    130                 135                 140

Asp His Ser His Phe Pro Arg Gly Glu Ile Glu Glu Ser Ile Ile Glu
145                 150                 155                 160

Asn Leu Gly Asn Asp His Asn Ala Ala Ala Gly Asp Gly Tyr Pro Arg
                165                 170                 175

Arg Thr Thr Leu Thr Ser Lys Ile Tyr His Thr Lys Gly Gln Glu Gly
            180                 185                 190

Ser Val Cys Leu Arg Ser Ser Asp Cys Ala Ala Gly Leu Cys Cys Ala
        195                 200                 205

Arg His Phe Trp Ser Lys Ile Cys Lys Pro Val Leu Lys Glu Gly Gln
    210                 215                 220

Val Cys Thr Lys His Lys Arg Lys Gly Ser His Gly Leu Glu Ile Phe
225                 230                 235                 240

Gln Arg Cys Tyr Cys Gly Glu Gly Leu Ala Cys Arg Ile Gln Lys Asp
                245                 250                 255

His His Gln Ala Ser Asn Ser Ser Arg Leu His Thr Cys Gln Arg His
            260                 265                 270

<210>  4
<211>  819
<212>  DNA
<213>  Mus musculus

<400>  4
atgatggttg tgtgtgcagc ggcagctgtc cggttcttgg ccgtgtttac aatgatggct      60

ctctgcagcc tccctctgct aggagccagt gccaccttga actcagttct catcaattcc     120

aacgcgatca agaacctgcc cccaccgctg ggtggtgctg gggggcagcc gggctctgct     180

gtcagtgtgg cgccgggagt tctctatgag ggcgggaaca agtaccagac tcttgacaac     240

taccagccct acccttgcgc tgaagatgag gagtgcggct ctgacgagta ctgctccagc     300

cccagccgcg gggcagccgg cgtcggaggt gtacagatct gtctggcttg ccgaaagcgc     360

aggaagcgct gcatgaggca cgctatgtgc tgccccggga actactgcaa aaatggaata     420

tgcatgccct ctgaccacag ccattttcct cgaggggaaa ttgaggaaag catcattgaa     480

aaccttggta atgaccacaa cgccgccgcg ggggatggat atcccagaag aaccacactg     540

acttcaaaaa tatatcacac caaaggacaa gaaggctccg tctgcctccg atcatcagac     600

tgtgccgcag ggctgtgttg tgcaagacac ttctggtcca agatctgtaa acctgtcctt     660

aaagaaggtc aggtgtgcac caagcacaaa cggaaaggct cccacgggct ggagatattc     720

cagcgctgtt actgcgggga aggcctggct tgcaggatac agaaagatca ccatcaagcc     780

agcaattctt ctaggctcca cacctgccag agacactaa                            819

<210>  5
<211>  19
<212>  DNA
<213> artificial sequence

<220>
<223>  primer

<400>  5
tggccttccg tgttcctac                                                   19

<210>  6
<211>  20
<212>  DNA

<213> artificial sequence

<220>
<223>  primer

<400>  6
gagttgctgt tgaagtcgca                                                   20


<210>  7
<211>  19
<212>  DNA
<213> artificial sequence

<220>
<223>  primer

<400>  7
ttgccgaaag cgcaggaac                                                    19


<210>  8
<211>  20
<212>  DNA
<213> artificial sequence

<220>
<223>  primer

<400>  8
cctcgaggta aatggctgtg                                                   20


<210>  9
<211>  23
<212>  DNA
<213> artificial sequence

<220>
<223>  primer

<400>  9
acgactaggc atctatgaag tgt                                               23


<210>  10
<211>  21
<212>  DNA
<213> artificial sequence

<220>
<223>  primer

<400>  10
ccggtctggt ccatactcat c                                                 21


<210>  11
<211>  21
<212>  DNA
<213> artificial sequence

<220>

<223>  primer

<400>  11
gaccccaaaa gatgaagggc t                                                21


<210>  12
<211>  20
<212>  DNA
<213> artificial sequence

<220>
<223>  primer

<400>  12
tgatgtgctg ctgcgagatt                                                  20


<210>  13
<211>  22
<212>  DNA
<213> artificial sequence

<220>
<223>  primer

<400>  13
tgatggatgc taccaaactg ga                                               22


<210>  14
<211>  22
<212>  DNA
<213> artificial sequence

<220>
<223>  primer

<400>  14
ctgaaggact ctggctttgt ct                                               22


<210>  15
<211>  20
<212>  DNA
<213> artificial sequence

<220>
<223>  primer

<400>  15
gcacagaaag catgatccgc                                                  20


<210>  16
<211>  20
<212>  DNA
<213> artificial sequence

<220>
<223>  primer

<400>  16

tcatcccttt ggggaccgat                                                    20

<210>  17
<211>  21
<212>  DNA
<213> artificial sequence

<220>
<223>  primer

<400>  17
cacacagtta tgggcatctg g                                                  21

<210>  18
<211>  20
<212>  DNA
<213> artificial sequence

<220>
<223>  primer

<400>  18
gggtgtagaa acgctcgctc                                                    20

<210>  19
<211>  21
<212>  DNA
<213> artificial sequence

<220>
<223>  primer

<400>  19
atctcagcag agtcggaaga a                                                  21

<210>  20
<211>  22
<212>  DNA
<213> artificial sequence

<220>
<223>  primer

<400>  20
caggtcctat tgcagtaaag gc                                                 22

<210>  21
<211>  22
<212>  DNA
<213> artificial sequence

<220>
<223>  primer

<400>  21
ccttacctcc tctcttcgga tg                                                 22

<210>  22
<211>  22
<212>  DNA
<213> artificial sequence

<220>
<223>  primer

<400>  22
tggagtagat cgtgtagcca aa                                                22


<210>  23
<211>  21
<212>  DNA
<213> artificial sequence

<220>
<223>  primer

<400>  23
actctccggg aacattacga t                                                 21


<210>  24
<211>  22
<212>  DNA
<213> artificial sequence

<220>
<223>  primer

<400>  24
caagacgatg aagctacagg tg                                                22


<210>  25
<211>  19
<212>  DNA
<213> artificial sequence

<220>
<223>  primer

<400>  25
ggccctctct agttcccag                                                    19


<210>  26
<211>  21
<212>  DNA
<213> artificial sequence

<220>
<223>  primer

<400>  26
tagccatcaa acctgttgag c                                                 21


<210>  27
<211>  21
<212>  DNA

<213> artificial sequence

<220>
<223>  primer

<400>  27
acaacctgta acagcaccct c                                                 21


<210>  28
<211>  22
<212>  DNA
<213> artificial sequence

<220>
<223>  primer

<400>  28
gcgatagcag aatacccaga gt                                                22


<210>  29
<211>  19
<212>  DNA
<213> artificial sequence

<220>
<223>  primer

<400>  29
tcgtgctgtc tcatcgagg                                                    19


<210>  30
<211>  22
<212>  DNA
<213> artificial sequence

<220>
<223>  primer

<400>  30
tcttcatgtg aaagcagaag cc                                                22


<210>  31
<211>  21
<212>  DNA
<213> artificial sequence

<220>
<223>  primer

<400>  31
agcaggtgac cttcgatgag t                                                 21


<210>  32
<211>  22
<212>  DNA
<213> artificial sequence

<220>

<223>  primer

<400>  32
acttccttga acacaatgga gc                                              22


<210>  33
<211>  21
<212>  DNA
<213> artificial sequence

<220>
<223>  primer

<400>  33
ttgtacgtgc aactcaggac t                                               21


<210>  34
<211>  21
<212>  DNA
<213> artificial sequence

<220>
<223>  primer

<400>  34
gatcccagag tgttgatgca a                                               21


<210>  35
<211>  21
<212>  DNA
<213> artificial sequence

<220>
<223>  primer

<400>  35
tcgatgctga caccaattca a                                               21


<210>  36
<211>  23
<212>  DNA
<213> artificial sequence

<220>
<223>  primer

<400>  36
tcccaataga caagtgagac caa                                             23


<210>  37
<211>  23
<212>  DNA
<213> artificial sequence

<220>
<223>  primer

<400>  37

atccacggca tactatcaac atc                                                23

<210>  38
<211>  21
<212>  DNA
<213> artificial sequence

<220>
<223>  primer

<400>  38
tcgtagtcat acggtgtggt g                                                  21